# EUROPEAN PATENT SPECIFICATION

(11) **EP 2 183 274 B1**
(45) Date of publication and mention of the grant of the patent: **18.07.2012**
(21) Application number: 08828032.6
(22) Date of filing: 14.08.2008
(51) Int. Cl.: C07K 14/195

(54) **METHODS AND COMPOSITIONS FOR TARGETING PROTEINS OF INTEREST TO THE HOST CELL ENVELOPE**
VERFAHREN UND ZUSAMMENSETZUNGEN ZUR RICHTUNG VON ZIELPROTEINEN GEGEN DIE WIRTSZELLHÜLLE
PROCÉDÉS ET COMPOSITIONS UTILISÉS POUR DIRIGER DES PROTÉINES D'INTÉRÊT VERS L'ENVELOPPE DE CELLULES HÔTES

(30) Priority: 21.08.2007 US 965649 P; 22.08.2007 US 965729 P
(43) Date of publication of application: 12.05.2010
(73) Proprietor: NEW ENGLAND BIOLABS, INC., Ipswich, MA 01938 (US)
(72) Inventor: SAMUELSON, James, C., Newburyport, MA 01950 (US); LUO, Jianying, Ipswich, MA (US)
(74) Representative: Froud, Clive
(86) International application number: PCT/US2008/073126
(87) International publication number: WO 2009/026089

(56) References cited:
- SHOKRI A ET AL: "CELL AND PROCESS DESIGN FOR TARGETING OF RECOMBINANT PROTEIN INTO THE CULTURE MEDIUM OF ESCHERICHIA COLI" APPLIED MICROBIOLOGY AND BIOTECHNOLOGY, SPRINGER VERLAG, BERLIN, DE, vol. 60, no. 6, 1 February 2003 (2003-02-01), pages 654-664, XP009078758 ISSN: 0175-7598
- SORENSEN H P ET AL: "Advanced genetic strategies for recombinant protein expression in Escherichia coli" JOURNAL OF BIOTECHNOLOGY, ELSEVIER SCIENCE PUBLISHERS, AMSTERDAM, NL, vol. 115, no. 2, 26 January 2005 (2005-01-26), pages 113-128, XP004682307 ISSN: 0168-1656
- LO ET AL: "Excretory over-expression of Bacillus sp. G1 cyclodextrin glucanotransferase (CGTase) in Escherichia coli: Optimization of the cultivation conditions by response surface methodology" ENZYME AND MICROBIAL TECHNOLOGY, STONEHAM, MA, US, vol. 40, no. 5, 8 March 2007 (2007-03-08), pages 1256-1263, XP005918048 ISSN: 0141-0229
- ZHANG G. ET AL.: "Extracellular accumulation of recombinant proteins fused to the carrier protein YebF in Escherichia coli" NATURE BIOTECHNOLOGY, vol. 24, no. 1, January 2006 (2006-01), pages 100-104, XP002504649
- BRAMBILLASCA S. ET AL.: "Unassisted translocation of large polypeptide domains across phospholipid bilayers" THE JOURNAL OF CELL BIOLOGY, vol. 175, no. 5, 27 November 2006 (2006-11-27), pages 767-777, XP002504650

### SEQUENCE LISTING

<110> New England Biolabs, Inc.
   samuelson, James
   Luo, Jianying
<120> Methods and Compositions for Targeting Proteins of interest to the Host Cell Envelope
<130> NEB-292-PEP
<150> PCT/US2008/073126
   <151> 2008-08-14
<150> 60/965,649 <151> 2007-08-21
<150> 60/965,729 <151> 2007-08-22
<160> 67
<170> PatentIn version 3.4
<210> 1
   <211> 5606
   <212> DNA
   <213> artificial
<220>
   <223> completely synthesized plasmid pVIII-phoA1
<400> 1
   aagcttgcat gcctgcaggt cgacggatcc ccggtgcctt cgtagtggca ttacgtattt     60
   tacccgttta atggaaactt cctcatgaaa aagtctttag tcctcaaagc ctctgtagcc    120
   gttgctaccc tcgttccgat gctgtctttc gctgctgagg gtgacgatcc cgcaaaagcg    180
   gcctttaact ccctgcaagc ctcagcgacc gaatatatcg gttatgcgtg ggcgatggtt    240
   gttgtcattg tcggcgcaac tatcggtatc aagctgttta agaaattcac ctcgaaagca    300
   aggggaattg gtcctgttct ggaaaaccgg gctgctcagg gcgatattac tgcacccggc    360
   ggtgctcgcc gtttaacggg tgatcagact gccgctctgc gtgattctct tagcgataaa    420
   cctgcaaaaa atattatttt gctgattggc gatgggatgg gggactcgga aattactgcc    480
   gcacgtaatt atgccgaagg tgcgggcggc ttttttaaag gtatagatgc cttaccgctt    540
   accgggcaat acactcacta tgcgctgaat aaaaaaaccg gcaaaccgga ctacgtcacc    600
   gactcggctg catcagcaac cgcctggtca accggtgtca aaacctataa cggcgcgctg    660
   ggcgtcgata ttcacgaaaa agatcaccca acgattctgg aaatggcaaa agccgcaggt    720
   ctggcgaccg gtaacgtttc taccgcagag ttgcaggatg ccacgcccgc tgcgctggtg    780
   gcacatgtga cctcgcgcaa atgctacggt ccgagcgcga ccagtgaaaa atgtccgggt    840
   aacgctctgg aaaaaggcgg aaaaggatcg attaccgaac agctgcttaa cgctcgtgcc    900
   gacgttacgc ttggcggcgg cgcaaaaacc tttgctgaaa cggcaaccgc tggtgaatgg    960
   cagggaaaaa cgctgcgtga acaggcacag gcgcgtggtt atcagttggt gagcgatgct   1020
   gcctcactga attcggtgac ggaagcgaat cagcaaaaac ccctgcttgg cctgtttgct   1080
   gacggcaata tgccagtgcg ctggctagga ccgaaagcaa cgtaccatgg caatatcgat 1140
   aagcccgcag tcacctgtac gccaaatccg caacgtaatg acagtgtacc aaccctggcg 1200
   cagatgaccg acaaagccat tgaattgttg agtaaaaatg agaaaggctt tttcctgcaa 1260
   gttgaaggtg cgtcaatcga taaacaggat catgctgcga atccttgtgg gcaaattggc 1320
   gagacggtcg atctcgatga agccgtacaa cgggcgctgg aattcgctaa aaaggagggt 1380
   aacacgctgg tcatagtcac cgctgatcac gcccacgcca gccagattgt tgcgccggat 1440
   accaaagctc cgggcctcac ccaggcgcta aataccaaag atggcgcagt gatggtgatg 1500
   agttacggga actccgaaga ggattcacaa gaacataccg gcagtcagtt gcgtattgcg 1560
   gcgtatggcc cgcatgccgc caatgttgtt ggactgaccg accagaccga tctcttctac 1620
   accatgaaag ccgctctggg gctgaaataa aaccgcgccc ggcagtgaat tttcgctgcc 1680
   ggcaattcag cttggctgtt ttggcggatg agagaagatt ttcagcctga tacagattaa 1740
   atcagaacgc agaagcggtc tgataaaaca gaatttgcct ggcggcagta gcgcggtggt 1800
   cccacctgac cccatgccga actcagaagt gaaacgccgt agcgccgatg gtagtgtggg 1860
   gtctccccat gcgagagtag ggaactgcca ggcatcaaat aaaacgaaag gctcagtcga 1920
   aagactgggc ctttcgtttt atctgttgtt tgtcggtgaa cgctctcctg agtaggacaa 1980
   atccgccggg agcggatttg aacgttgcga agcaacggcc cggagggtgg cgggcaggac 2040
   gcccgccata aactgccagg catcaaatta agcagaaggc catcctgacg gatggccttt 2100
   ttgcgtttct acaaactctt ttgtttattt ttctaaatac attcaaatat gtatccgctc 2160
   atgagacaat aaccctgata aatgcttcaa taatattgaa aaaggaagag tatgagtatt 2220
   caacatttcc gtgtcgccct tattcccttt tttgcggcat tttgccttcc tgtttttgct 2280
   cacccagaaa cgctggtgaa agtaaaagat gctgaagatc agttgggtgc acgagtgggt 2340
   tacatcgaac tggatctcaa cagcggtaag atccttgaga gttttcgccc cgaagaacgt 2400
   tttccaatga tgagcacttt taaagttctg ctatgtggcg cggtattatc ccgtgttgac 2460
   gccgggcaag agcaactcgg tcgccgcata cactattctc agaatgactt ggttgagtac 2520
   tcaccagtca cagaaaagca tcttacggat ggcatgacag taagagaatt atgcagtgct 2580
   gccataacca tgagtgataa cactgcggcc aacttacttc tgacaacgat cggaggaccg 2640
   aaggagctaa ccgctttttt gcacaacatg ggggatcatg taactcgcct tgatcgttgg 2700
   gaaccggagc tgaatgaagc cataccaaac gacgagcgtg acaccacgat gcctgcagca 2760
   atggcaacaa cgttgcgcaa actattaact ggcgaactac ttactctagc ttcccggcaa 2820
   caattaatag actggatgga ggcggataaa gttgcaggac cacttctgcg ctcggccctt 2880
   ccggctggct ggtttattgc tgataaatct ggagccggtg agcgtgggtc tcgcggtatc 2940
   attgcagcac tggggccaga tggtaagccc tcccgtatcg tagttatcta cacgacgggg 3000
   agtcaggcaa ctatggatga acgaaataga cagatcgctg agataggtgc ctcactgatt 3060
   aagcattggt aactgtcaga ccaagtttac tcatatatac tttagattga tttaaaactt 3120
   catttttaat ttaaaaggat ctaggtgaag atcctttttg ataatctcat gaccaaaatc 3180
   ccttaacgtg agttttcgtt ccactgagcg tcagaccccg tagaaaagat caaaggatct 3240
   tcttgagatc ctttttttct gcgcgtaatc tgctgcttgc aaacaaaaaa accaccgcta 3300
   ccagcggtgg tttgtttgcc ggatcaagag ctaccaactc tttttccgaa ggtaactggc 3360
   ttcagcagag cgcagatacc aaatactgtc cttctagtgt agccgtagtt aggccaccac 3420
   ttcaagaact ctgtagcacc gcctacatac ctcgctctgc taatcctgtt accagtggct 3480
   gctgccagtg gcgataagtc gtgtcttacc gggttggact caagacgata gttaccggat 3540
   aaggcgcagc ggtcgggctg aacggggggt tcgtgcacac agcccagctt ggagcgaacg 3600
   acctacaccg aactgagata cctacagcgt gagcattgag aaagcgccac gcttcccgaa 3660
   gggagaaagg cggacaggta tccggtaagc ggcagggtcg gaacaggaga gcgcacgagg 3720
   gagcttccag ggggaaacgc ctggtatctt tatagtcctg cgggtttcgc cacctctgac 3780
   ttgagcgtcg atttttgtga tgctcgtcac gggggcggag cctatggaaa aacgccagca 3840
   acgcggcctt tttacggttc ctggcctttt gctggccttt tgctcacatg ttctttcctg 3900
   cgttatcccc tgattctgtg gataaccgta ttaccgcctt tgagtgagct gataccgctc 3960
   gccgcagccg aacgaccgag cgcagcgagt cagtgagcga ggaagcggaa gagcgcctga 4020
   tgcggtattt tctccttacg catctgtgcg gtatttcaca ccgcatacga acgccagcaa 4080
   gacgtagccc agcgcgtcgg ccagcttgca attcgcgcta acttacatta attgcgttgc 4140
   gctcactgcc cgctttccag tcgggaaacc tgtcgtgcca gctgcattaa tgaatcggcc 4200
   aacgcgcggg gagaggcggt ttgcgtattg ggcgccaggg tggtttttct tttcaccagt 4260
   gagacgggca acagctgatt gcccttcacc gcctggccct gagagagttg cagcaagcgg 4320
   tccacgctgg tttgccccag caggcgaaaa tcctgtttgc tggtggttaa cggcgggata 4380
   taacatgagc tgtcttcggt atcgtcgtat cccactaccg agatatccgc accaacgcgc 4440
   agcccggact cggtaatggc gcgcattgcg cccagcgcca tctgatcgtt ggcaaccagc 4500
   atcgcagtgg gaacgatgcc ctcattcagc atttgcatgg tttgttgaaa accggacatg 4560
   gcactccagt cgccttcccg ttccgctatc ggctgaattt gattgcgagt gagatattta 4620
   tgccagccag ccagacgcag acgcgccgag acagaactta atgggcccgc taacagcgcg 4680
   atttgctggt gacccaatgc gaccagatgc tccacgccca gtcgcgtacc gtcttcatgg 4740
   gagaaaataa tactgttgat gggtgtctgg tcagagacat caagaaataa cgccggaaca 4800
   ttagtgcagg cagcttccac agcaatggca tcctggtcat ccagcgcata gttaatgatc 4860
   agcccactga cgcgttgcgc gagaagattg tgcaccgccg ctttacaggc ttcgacgccg 4920
   cttcgttcta ccatcgacac caccacgctg gcacccagtt gatcggcgcg agatttaatc 4980
   gccgcgacaa tttgcgacgg cgcgtgcagg gccagactgg aggtggcaac gccaatcagc 5040
   aacgactgtt tgcccgccag ttgttgtgcc acgcggttgg gaatgtaatt cagctccgcc 5100
   atcgccgctt ccactttttc ccgcgttttc gcagaaacgt ggctggcctg gttcaccacg 5160
   cgggaaacgg tctgataaga gacaccggca tactctgcga catcgtataa cgttactggt 5220
   ttcacattca ccaccctgaa ttgactctct tccgggcgct atcatgccat accgcgaaag 5280
   gttttgcacc attcgatggt gtcaacgtaa atgccgcttc gccttcgcgc gcgaattgca 5340
   agctgatccg ggcttatcga ctgcacggtg caccaatgct tctggcgtca ggcagccatc 5400
   ggaagctgtg gtatggctgt gcaggtcgta aatcactgca taattcgtgt cgctcaaggc 5460
   gcactcccgt tctggataat gttttttgcg ccgacatcat aacggttctg gcaaatattc 5520
   tgaaatgagc tgttgacaat taatcatcgg ctcgtataat gtgtggaatt gtgagcggat 5580
   aacaatttca cacaggaaac agaatt 5606
<210> 2
   <211> 4229
   <212> DNA
   <213> artificial
<220>
   <223> completely synthesized plasmid pVIII-EcoRI
<400> 2
   aagcttgcat gcctgcaggt cgacggatcc ccggtgcctt cgtagtggca ttacgtattt      60
   tacccgttta atggaaactt cctcatgaaa aagtctttag tcctcaaagc ctctgtagcc     120
   gttgctaccc tcgttccgat gctgtctttc gctgctgagg gtgacgatcc cgcaaaagcg     180
   gcctttaact ccctgcaagc ctcagcgacc gaatatatcg gttatgcgtg ggcgatggtt     240
   gttgtcattg tcggcgcaac tatcggtatc aagctgttta agaaattcac ctcgaaagca     300
   aggggaattc agcttggctg ttttggcgga tgagagaaga ttttcagcct gatacagatt     360
   aaatcagaac gcagaagcgg tctgataaaa cagaatttgc ctggcggcag tagcgcggtg     420
   gtcccacctg accccatgcc gaactcagaa gtgaaacgcc gtagcgctga tggtagtgtg     480
   gggtctcccc atgcgagagt agggaactgc caggcatcaa ataaaacgaa aggctcagtc     540
   gaaagactgg gcctttcgtt ttatctgttg tttgtcggtg aacgctctcc tgagtaggac     600
   aaatccgccg ggagcggatt tgaacgttgc gaagcaacgg cccggagggt ggcgggcagg     660
   acgcccgcca taaactgcca ggcatcaaat taagcagaag gccatcctga cggatggcct     720
   ttttgcgttt ctacaaactc tttttgttta tttttctaaa tacattcaaa tatgtatccg     780
   ctcatgagac aataaccctg ataaatgctt caataatatt gaaaaaggaa gagtatgagt     840
   attcaacatt tccgtgtcgc ccttattccc ttttttgcgg cattttgcct tcctgttttt     900
   gctcacccag aaacgctggt gaaagtaaaa gatgctgaag atcagttggg tgcacgagtg     960
   ggttacatcg aactggatct caacagcggt aagatccttg agagttttcg ccccgaagaa    1020
   cgttttccaa tgatgagcac ttttaaagtt ctgctatgtg gcgcggtatt atcccgtgtt    1080
   gacgccgggc aagagcaact cggtcgccgc atacactatt ctcagaatga cttggttgag    1140
   tactcaccag tcacagaaaa gcatcttacg gatggcatga cagtaagaga attatgcagt    1200
   gctgccataa ccatgagtga taacactgcg gccaacttac ttctgacaac gatcggagga    1260
   ccgaaggagc taaccgcttt tttgcacaac atgggggatc atgtaactcg ccttgatcgt 1320
   tgggaaccgg agctgaatga agccatacca aacgacgagc gtgacaccac gatgcctgca 1380
   gcaatggcaa caacgttgcg caaactatta actggcgaac tacttactct agcttcccgg 1440
   caacaattaa tagactggat ggaggcggat aaagttgcag gaccacttct gcgctcggcc 1500
   cttccggctg gctggtttat tgctgataaa tctggagccg gtgagcgtgg gtctcgcggt 1560
   atcattgcag cactggggcc agatggtaag ccctcccgta tcgtagttat ctacacgacg 1620
   gggagtcagg caactatgga tgaacgaaat agacagatcg ctgagatagg tgcctcactg 1680
   attaagcatt ggtaactgtc agaccaagtt tactcatata tactttagat tgatttaaaa 1740
   cttcattttt aatttaaaag gatctaggtg aagatccttt ttgataatct catgaccaaa 1800
   atcccttaac gtgagttttc gttccactga gcgtcagacc ccgtagaaaa gatcaaagga 1860
   tcttcttgag atcctttttt tctgcgcgta atctgctgct tgcaaacaaa aaaaccaccg 1920
   ctaccagcgg tggtttgttt gccggatcaa gagctaccaa ctctttttcc gaaggtaact 1980
   ggcttcagca gagcgcagat accaaatact gtccttctag tgtagccgta gttaggccac 2040
   cacttcaaga actctgtagc accgcctaca tacctcgctc tgctaatcct gttaccagtg 2100
   gctgctgcca gtggcgataa gtcgtgtctt accgggttgg actcaagacg atagttaccg 2160
   gataaggcgc agcggtcggg ctgaacgggg ggttcgtgca cacagcccag cttggagcga 2220
   acgacctaca ccgaactgag atacctacag cgtgagcatt gagaaagcgc cacgcttccc 2280
   gaagggagaa aggcggacag gtatccggta agcggcaggg tcggaacagg agagcgcacg 2340
   agggagcttc cagggggaaa cgcctggtat ctttatagtc ctgcgggttt cgccacctct 2400
   gacttgagcg tcgatttttg tgatgctcgt caggggggcg gagcctatgg aaaaacgcca 2460
   gcaacgcggc ctttttacgg ttcctggcct tttgctggcc ttttgctcac atgttctttc 2520
   ctgcgttatc ccctgattct gtggataacc gtattaccgc ctttgagtga gctgataccg 2580
   ctcgccgcag ccgaacgacc gagcgcagcg agtcagtgag cgaggaagcg gaagagcgcc 2640
   tgatgcggta ttttctcctt acgcatctgt gcggtatttc acaccgcata cgaacgccag 2700
   caagacgtag cccagcgcgt cggccagctt gcaattcgcg ctaacttaca ttaattgcgt 2760
   tgcgctcact gcccgctttc cagtcgggaa acctgtcgtg ccagctgcat taatgaatcg 2820
   gccaacgcgc ggggagaggc ggtttgcgta ttgggcgcca gggtggtttt tcttttcacc 2880
   agtgagacgg gcaacagctg attgcccttc accgcctggc cctgagagag ttgcagcaag 2940
   cggtccacgc tggtttgccc cagcaggcga aaatcctgtt tgctggtggt taacggcggg 3000
   atataacatg agctgtcttc ggtatcgtcg tatcccacta ccgagatatc cgcaccaacg 3060
   cgcagcccgg actcggtaat ggcgcgcatt gcgcccagcg ccatctgatc gttggcaacc 3120
   agcatcgcag tgggaacgat gccctcattc agcatttgca tggtttgttg aaaaccggac 3180
   atggcactcc agtcgccttc ccgttccgct atcggctgaa tttgattgcg agtgagatat 3240
   ttatgccagc cagccagacg cagacgcgcc gagacagaac ttaatgggcc cgctaacagc 3300
   gcgatttgct ggtgacccaa tgcgaccaga tgctccacgc ccagtcgcgt accgtcttca 3360
   tgggagaaaa taatactgtt gatgggtgtc tggtcagaga catcaagaaa taacgccgga 3420
   acattagtgc aggcagcttc cacagcaatg gcatcctggt catccagcgc atagttaatg 3480
   atcagcccac tgacgcgttg cgcgagaaga ttgtgcaccg ccgctttaca ggcttcgacg 3540
   ccgcttcgtt ctaccatcga caccaccacg ctggcaccca gttgatcggc gcgagattta 3600
   atcgccgcga caatttgcga cggcgcgtgc agggccagac tggaggtggc aacgccaatc 3660
   agcaacgact gtttgcccgc cagttgttgt gccacgcggt tgggaatgta attcagctcc 3720
   gccatcgccg cttccacttt ttcccgcgtt ttcgcagaaa cgtggctggc ctggttcacc 3780
   acgcgggaaa cggtctgata agagacaccg gcatactctg cgacatcgta taacgttact 3840
   ggtttcacat tcaccaccct gaattgactc tcttccgggc gctatcatgc cataccgcga 3900
   aaggttttgc accattcgat ggtgtcaacg taaatgccgc ttcgccttcg cgcgcgaatt 3960
   gcaagctgat ccgggcttat cgactgcacg gtgcaccaat gcttctggcg tcaggcagcc 4020
   atcggaagct gtggtatggc tgtgcaggtc gtaaatcact gcataattcg tgtcgctcaa 4080
   ggcgcactcc cgttctggat aatgtttttt gcgccgacat cataacggtt ctggcaaata 4140
   ttctgaaatg agctgttgac aattaatcat cggctcgtat aatgtgtgga attgtgagcg 4200
   gataacaatt tcacacagga aacagaatt 4229
<210> 3
   <211> 4415
   <212> DNA
   <213> artificial
<220>
   <223> completely synthesized plasmid pVIII-TM2
<400> 3
   aagcttgcat gcctgcaggt cgacggatcg atccccgtgc cttcgtagtg gcattacgta 60
   ttttacccgt ttaatggaaa cttcctcatg aaaaagtctt tagtcctcaa agcctctgta 120
   gccgttgcta ccctcgttcc gatgctgtct ttcgctgctg agggtgacga tcccgcaaaa 180
   gcggccttta actccctgca agcctcagcg accgaatata tcggttatgc gtgggcgatg 240
   gttgttgtca ttgtcggcgc aactatcggt atcaagctgt ttaagaaatt cacctcgaaa 300
   gcaaggggaa tttcgcgtca ggcagcggcg caggcggctg ccggggactc actggataaa 360
   gcaacgttga aaaaggttgc gccgaagcct ggctggctgg aaaccggtgc ttctgttttt 420
   ccggtactgg ctatcgtatt gattgtgcgt tcgtttattt atgaaccgtt ccagatcccg 480
   tcaggatcct cgaattcagc ttggctgttt tggcggatga gagaagattt tcagcctgat 540
   acagattaaa tcagaacgca gaagcggtct gataaaacag aatttgcctg gcggcagtag 600
   cgcggtggtc ccacctgacc ccatgccgaa ctcagaagtg aaacgccgta gcgccgatgg 660
   tagtgtgggg tctccccatg cgagagtagg gaactgccag gcatcaaata aaacgaaagg 720
   ctcagtcgaa agactgggcc tttcgtttta tctgttgttt gtcggtgaac gctctcctga 780
   gtaggacaaa tccgccggga gcggatttga acgttgcgaa gcaacggccc ggagggtggc 840
   gggcaggacg cccgccataa actgccaggc atcaaattaa gcagaaggcc atcctgacgg 900
   atggcctttt tgcgtttcta caaactcttt tgtttatttt tctaaataca ttcaaatatg 960
   tatccgctca tgagacaata accctgataa atgcttcaat aatattgaaa aaggaagagt 1020
   atgagtattc aacatttccg tgtcgccctt attccctttt ttgcggcatt ttgccttcct 1080
   gtttttgctc acccagaaac gctggtgaaa gtaaaagatg ctgaagatca gttgggtgca 1140
   cgagtgggtt acatcgaact ggatctcaac agcggtaaga tccttgagag ttttcgcccc 1200
   gaagaacgtt ttccaatgat gagcactttt aaagttctgc tatgtggcgc ggtattatcc 1260
   cgtgttgacg ccgggcaaga gcaactcggt cgccgcatac actattctca gaatgacttg 1320
   gttgagtact caccagtcac agaaaagcat cttacggatg gcatgacagt aagagaatta 1380
   tgcagtgctg ccataaccat gagtgataac actgcggcca acttacttct gacaacgatc 1440
   ggaggaccga aggagctaac cgcttttttg cacaacatgg gggatcatgt aactcgcctt 1500
   gatcgttggg aaccggagct gaatgaagcc ataccaaacg acgagcgtga caccacgatg 1560
   cctgcagcaa tggcaacaac gttgcgcaaa ctattaactg gcgaactact tactctagct 1620
   tcccggcaac aattaataga ctggatggag gcggataaag ttgcaggacc acttctgcgc 1680
   tcggcccttc cggctggctg gtttattgct gataaatctg gagccggtga gcgtgggtct 1740
   cgcggtatca ttgcagcact ggggccagat ggtaagccct cccgtatcgt agttatctac 1800
   acgacgggga gtcaggcaac tatggatgaa cgaaatagac agatcgctga gataggtgcc 1860
   tcactgatta agcattggta actgtcagac caagtttact catatatact ttagattgat 1920
   ttaaaacttc atttttaatt taaaaggatc taggtgaaga tcctttttga taatctcatg 1980
   accaaaatcc cttaacgtga gttttcgttc cactgagcgt cagaccccgt agaaaagatc 2040
   aaaggatctt cttgagatcc tttttttctg cgcgtaatct gctgcttgca aacaaaaaaa 2100
   ccaccgctac cagcggtggt ttgtttgccg gatcaagagc taccaactct ttttccgaag 2160
   gtaactggct tcagcagagc gcagatacca aatactgtcc ttctagtgta gccgtagtta 2220
   ggccaccact tcaagaactc tgtagcaccg cctacatacc tcgctctgct aatcctgtta 2280
   ccagtggctg ctgccagtgg cgataagtcg tgtcttaccg ggttggactc aagacgatag 2340
   ttaccggata aggcgcagcg gtcgggctga acggggggtt cgtgcacaca gcccagcttg 2400
   gagcgaacga cctacaccga actgagatac ctacagcgtg agcattgaga aagcgccacg 2460
   cttcccgaag ggagaaaggc ggacaggtat ccggtaagcg gcagggtcgg aacaggagag 2520
   cgcacgaggg agcttccagg gggaaacgcc tggtatcttt atagtcctgc gggtttcgcc 2580
   acctctgact tgagcgtcga tttttgtgat gctcgtcacg ggggcggagc ctatggaaaa 2640
   acgccagcaa cgcggccttt ttacggttcc tggccttttg ctggcctttt gctcacatgt 2700
   tctttcctgc gttatcccct gattctgtgg ataaccgtat taccgccttt gagtgagctg 2760
   ataccgctcg ccgcagccga acgaccgagc gcagcgagtc agtgagcgag gaagcggaag 2820
   agcgcctgat gcggtatttt ctccttacgc atctgtgcgg tatttcacac cgcatacgaa 2880
   cgccagcaag acgtagccca gcgcgtcggc cagcttgcaa ttcgcgctaa cttacattaa 2940
   ttgcgttgcg ctcactgccc gctttccagt cgggaaacct gtcgtgccag ctgcattaat 3000
   gaatcggcca acgcgcgggg agaggcggtt tgcgtattgg gcgccagggt ggtttttctt 3060
   ttcaccagtg agacgggcaa cagctgattg cccttcaccg cctggccctg agagagttgc 3120
   agcaagcggt ccacgctggt ttgccccagc aggcgaaaat cctgtttgct ggtggttaac 3180
   ggcgggatat aacatgagct gtcttcggta tcgtcgtatc ccactaccga gatatccgca 3240
   ccaacgcgca gcccggactc ggtaatggcg cgcattgcgc ccagcgccat ctgatcgttg 3300
   gcaaccagca tcgcagtggg aacgatgccc tcattcagca tttgcatggt ttgttgaaaa 3360
   ccggacatgg cactccagtc gccttcccgt tccgctatcg gctgaatttg attgcgagtg 3420
   agatatttat gccagccagc cagacgcaga cgcgccgaga cagaacttaa tgggcccgct 3480
   aacagcgcga tttgctggtg acccaatgcg accagatgct ccacgcccag tcgcgtaccg 3540
   tcttcatggg agaaaataat actgttgatg ggtgtctggt cagagacatc aagaaataac 3600
   gccggaacat tagtgcaggc agcttccaca gcaatggcat cctggtcatc cagcgcatag 3660
   ttaatgatca gcccactgac gcgttgcgcg agaagattgt gcaccgccgc tttacaggct 3720
   tcgacgccgc ttcgttctac catcgacacc accacgctgg cacccagttg atcggcgcga 3780
   gatttaatcg ccgcgacaat ttgcgacggc gcgtgcaggg ccagactgga ggtggcaacg 3840
   ccaatcagca acgactgttt gcccgccagt tgttgtgcca cgcggttggg aatgtaattc 3900
   agctccgcca tcgccgcttc cactttttcc cgcgttttcg cagaaacgtg gctggcctgg 3960
   ttcaccacgc gggaaacggt ctgataagag acaccggcat actctgcgac atcgtataac 4020
   gttactggtt tcacattcac caccctgaat tgactctctt ccgggcgcta tcatgccata 4080
   ccgcgaaagg ttttgcacca ttcgatggtg tcaacgtaaa tgccgcttcg ccttcgcgcg 4140
   cgaattgcaa gctgatccgg gcttatcgac tgcacggtgc accaatgctt ctggcgtcag 4200
   gcagccatcg gaagctgtgg tatggctgtg caggtcgtaa atcactgcat aattcgtgtc 4260
   gctcaaggcg cactcccgtt ctggataatg ttttttgcgc cgacatcata acggttctgg 4320
   caaatattct gaaatgagct gttgacaatt aatcatcggc tcgtataatg tgtggaattg 4380
   tgagcggata acaatttcac acaggaaaca gaatt 4415
<210> 4
   <211> 4415
   <212> DNA
   <213> artificial
<220>
   <223> completely synthesized plasmid pVIII-8His
<400> 4
   aagcttgcat gcctgcaggt cgacggatcg atccccgtgc cttcgtagtg gcattacgta 60
   ttttacccgt ttaatggaaa cttcctcatg aaaaagtctt tagtcctcaa agcctctgta 120
   gccgttgcta ccctcgttcc gatgctgtct ttcgctgctg agggtgacga tcccgcaaaa 180
   gcggccttta actccctgca agcctcagcg accgaatata tcggttatgc gtgggcgatg 240
   gttgttgtca ttgtcggcgc aactatcggt atcaagctgt ttaagaaatt cacctcgaaa 300
   gcaaggggaa tttcgcgtca tcaccatcac catcaccatc acggggactc actggataaa 360
   gcaacgttga aaaaggttgc gccgaagcct ggctggctgg aaaccggtgc ttctgttttt 420
   ccggtactgg ctatcgtatt gattgtgcgt tcgtttattt atgaaccgtt ccagatcccg 480
   tcaggatcct cgaattcagc ttggctgttt tggcggatga gagaagattt tcagcctgat 540
   acagattaaa tcagaacgca gaagcggtct gataaaacag aatttgcctg gcggcagtag 600
   cgcggtggtc ccacctgacc ccatgccgaa ctcagaagtg aaacgccgta gcgccgatgg 660
   tagtgtgggg tctccccatg cgagagtagg gaactgccag gcatcaaata aaacgaaagg 720
   ctcagtcgaa agactgggcc tttcgtttta tctgttgttt gtcggtgaac gctctcctga 780
   gtaggacaaa tccgccggga gcggatttga acgttgcgaa gcaacggccc ggagggtggc 840
   gggcaggacg cccgccataa actgccaggc atcaaattaa gcagaaggcc atcctgacgg 900
   atggcctttt tgcgtttcta caaactcttt tgtttatttt tctaaataca ttcaaatatg 960
   tatccgctca tgagacaata accctgataa atgcttcaat aatattgaaa aaggaagagt 1020
   atgagtattc aacatttccg tgtcgccctt attccctttt ttgcggcatt ttgccttcct 1080
   gtttttgctc acccagaaac gctggtgaaa gtaaaagatg ctgaagatca gttgggtgca 1140
   cgagtgggtt acatcgaact ggatctcaac agcggtaaga tccttgagag ttttcgcccc 1200
   gaagaacgtt ttccaatgat gagcactttt aaagttctgc tatgtggcgc ggtattatcc 1260
   cgtgttgacg ccgggcaaga gcaactcggt cgccgcatac actattctca gaatgacttg 1320
   gttgagtact caccagtcac agaaaagcat cttacggatg gcatgacagt aagagaatta 1380
   tgcagtgctg ccataaccat gagtgataac actgcggcca acttacttct gacaacgatc 1440
   ggaggaccga aggagctaac cgcttttttg cacaacatgg gggatcatgt aactcgcctt 1500
   gatcgttggg aaccggagct gaatgaagcc ataccaaacg acgagcgtga caccacgatg 1560
   cctgcagcaa tggcaacaac gttgcgcaaa ctattaactg gcgaactact tactctagct 1620
   tcccggcaac aattaataga ctggatggag gcggataaag ttgcaggacc acttctgcgc 1680
   tcggcccttc cggctggctg gtttattgct gataaatctg gagccggtga gcgtgggtct 1740
   cgcggtatca ttgcagcact ggggccagat ggtaagccct cccgtatcgt agttatctac 1800
   acgacgggga gtcaggcaac tatggatgaa cgaaatagac agatcgctga gataggtgcc 1860
   tcactgatta agcattggta actgtcagac caagtttact catatatact ttagattgat 1920
   ttaaaacttc atttttaatt taaaaggatc taggtgaaga tcctttttga taatctcatg 1980
   accaaaatcc cttaacgtga gttttcgttc cactgagcgt cagaccccgt agaaaagatc 2040
   aaaggatctt cttgagatcc tttttttctg cgcgtaatct gctgcttgca aacaaaaaaa 2100
   ccaccgctac cagcggtggt ttgtttgccg gatcaagagc taccaactct ttttccgaag 2160
   gtaactggct tcagcagagc gcagatacca aatactgtcc ttctagtgta gccgtagtta 2220
   ggccaccact tcaagaactc tgtagcaccg cctacatacc tcgctctgct aatcctgtta 2280
   ccagtggctg ctgccagtgg cgataagtcg tgtcttaccg ggttggactc aagacgatag 2340
   ttaccggata aggcgcagcg gtcgggctga acggggggtt cgtgcacaca gcccagcttg 2400
   gagcgaacga cctacaccga actgagatac ctacagcgtg agcattgaga aagcgccacg 2460
   cttcccgaag ggagaaaggc ggacaggtat ccggtaagcg gcagggtcgg aacaggagag 2520
   cgcacgaggg agcttccagg gggaaacgcc tggtatcttt atagtcctgc gggtttcgcc 2580
   acctctgact tgagcgtcga tttttgtgat gctcgtcacg ggggcggagc ctatggaaaa 2640
   acgccagcaa cgcggccttt ttacggttcc tggccttttg ctggcctttt gctcacatgt 2700
   tctttcctgc gttatcccct gattctgtgg ataaccgtat taccgccttt gagtgagctg 2760
   ataccgctcg ccgcagccga acgaccgagc gcagcgagtc agtgagcgag gaagcggaag 2820
   agcgcctgat gcggtatttt ctccttacgc atctgtgcgg tatttcacac cgcatacgaa 2880
   cgccagcaag acgtagccca gcgcgtcggc cagcttgcaa ttcgcgctaa cttacattaa 2940
   ttgcgttgcg ctcactgccc gctttccagt cgggaaacct gtcgtgccag ctgcattaat 3000
   gaatcggcca acgcgcgggg agaggcggtt tgcgtattgg gcgccagggt ggtttttctt 3060
   ttcaccagtg agacgggcaa cagctgattg cccttcaccg cctggccctg agagagttgc 3120
   agcaagcggt ccacgctggt ttgccccagc aggcgaaaat cctgtttgct ggtggttaac 3180
   ggcgggatat aacatgagct gtcttcggta tcgtcgtatc ccactaccga gatatccgca 3240
   ccaacgcgca gcccggactc ggtaatggcg cgcattgcgc ccagcgccat ctgatcgttg 3300
   gcaaccagca tcgcagtggg aacgatgccc tcattcagca tttgcatggt ttgttgaaaa 3360
   ccggacatgg cactccagtc gccttcccgt tccgctatcg gctgaatttg attgcgagtg 3420
   agatatttat gccagccagc cagacgcaga cgcgccgaga cagaacttaa tgggcccgct 3480
   aacagcgcga tttgctggtg acccaatgcg accagatgct ccacgcccag tcgcgtaccg 3540
   tcttcatggg agaaaataat actgttgatg ggtgtctggt cagagacatc aagaaataac 3600
   gccggaacat tagtgcaggc agcttccaca gcaatggcat cctggtcatc cagcgcatag 3660
   ttaatgatca gcccactgac gcgttgcgcg agaagattgt gcaccgccgc tttacaggct 3720
   tcgacgccgc ttcgttctac catcgacacc accacgctgg cacccagttg atcggcgcga 3780
   gatttaatcg ccgcgacaat ttgcgacggc gcgtgcaggg ccagactgga ggtggcaacg 3840
   ccaatcagca acgactgttt gcccgccagt tgttgtgcca cgcggttggg aatgtaattc 3900
   agctccgcca tcgccgcttc cactttttcc cgcgttttcg cagaaacgtg gctggcctgg 3960
   ttcaccacgc gggaaacggt ctgataagag acaccggcat actctgcgac atcgtataac 4020
   gttactggtt tcacattcac caccctgaat tgactctctt ccgggcgcta tcatgccata 4080
   ccgcgaaagg ttttgcacca ttcgatggtg tcaacgtaaa tgccgcttcg ccttcgcgcg 4140
   cgaattgcaa gctgatccgg gcttatcgac tgcacggtgc accaatgctt ctggcgtcag 4200
   gcagccatcg gaagctgtgg tatggctgtg caggtcgtaa atcactgcat aattcgtgtc 4260
   gctcaaggcg cactcccgtt ctggataatg ttttttgcgc cgacatcata acggttctgg 4320
   caaatattct gaaatgagct gttgacaatt aatcatcggc tcgtataatg tgtggaattg 4380
   tgagcggata acaatttcac acaggaaaca gaatt 4415
<210> 5
   <211> 4580
   <212> DNA
   <213> artificial
<220>
   <223> completely synthesized plasmid pVIII-CBD
<400> 5
   aagcttgcat gcctgcaggt cgacggatcg atccccgtgc cttcgtagtg gcattacgta 60
   ttttacccgt ttaatggaaa cttcctcatg aaaaagtctt tagtcctcaa agcctctgta 120
   gccgttgcta ccctcgttcc gatgctgtct ttcgctgctg agggtgacga tcccgcaaaa 180
   gcggccttta actccctgca agcctcagcg accgaatata tcggttatgc gtgggcgatg 240
   gttgttgtca ttgtcggcgc aactatcggt atcaagctgt ttaagaaatt cacctcgaaa 300
   gcaaggggaa tttcgcgtca ggcagcggcg caggcggctg ccacgacaaa tcctggtgta 360
   tccgcttggc aggtcaacac agcttatact gcgggacaat tggtcacata taacggcaag 420
   acgtataaat gtttgcagcc ccacacctcc ttggcaggat gggaaccatc caacgttcct 480
   gccttgtggc agcttcaagg tggccatggg gactcactgg ataaagcaac gttgaaaaag 540
   gttgcgccga agcctggctg gctggaaacc ggtgcttctg tttttccggt actggctatc 600
   gtattgattg tgcgttcgtt tatttatgaa ccgttccaga tcccgtcagg atcctcgaat 660
   tcagcttggc tgttttggcg gatgagagaa gattttcagc ctgatacaga ttaaatcaga 720
   acgcagaagc ggtctgataa aacagaattt gcctggcggc agtagcgcgg tggtcccacc 780
   tgaccccatg ccgaactcag aagtgaaacg ccgtagcgcc gatggtagtg tggggtctcc 840
   ccatgcgaga gtagggaact gccaggcatc aaataaaacg aaaggctcag tcgaaagact 900
   gggcctttcg ttttatctgt tgtttgtcgg tgaacgctct cctgagtagg acaaatccgc 960
   cgggagcgga tttgaacgtt gcgaagcaac ggcccggagg gtggcgggca ggacgcccgc 1020
   cataaactgc caggcatcaa attaagcaga aggccatcct gacggatggc ctttttgcgt 1080
   ttctacaaac tcttttgttt atttttctaa atacattcaa atatgtatcc gctcatgaga 1140
   caataaccct gataaatgct tcaataatat tgaaaaagga agagtatgag tattcaacat 1200
   ttccgtgtcg cccttattcc cttttttgcg gcattttgcc ttcctgtttt tgctcaccca 1260
   gaaacgctgg tgaaagtaaa agatgctgaa gatcagttgg gtgcacgagt gggttacatc 1320
   gaactggatc tcaacagcgg taagatcctt gagagttttc gccccgaaga acgttttcca 1380
   atgatgagca cttttaaagt tctgctatgt ggcgcggtat tatcccgtgt tgacgccggg 1440
   caagagcaac tcggtcgccg catacactat tctcagaatg acttggttga gtactcacca 1500
   gtcacagaaa agcatcttac ggatggcatg acagtaagag aattatgcag tgctgccata 1560
   accatgagtg ataacactgc ggccaactta cttctgacaa cgatcggagg accgaaggag 1620
   ctaaccgctt ttttgcacaa catgggggat catgtaactc gccttgatcg ttgggaaccg 1680
   gagctgaatg aagccatacc aaacgacgag cgtgacacca cgatgcctgc agcaatggca 1740
   acaacgttgc gcaaactatt aactggcgaa ctacttactc tagcttcccg gcaacaatta 1800
   atagactgga tggaggcgga taaagttgca ggaccacttc tgcgctcggc ccttccggct 1860
   ggctggttta ttgctgataa atctggagcc ggtgagcgtg ggtctcgcgg tatcattgca 1920
   gcactggggc cagatggtaa gccctcccgt atcgtagtta tctacacgac ggggagtcag 1980
   gcaactatgg atgaacgaaa tagacagatc gctgagatag gtgcctcact gattaagcat 2040
   tggtaactgt cagaccaagt ttactcatat atactttaga ttgatttaaa acttcatttt 2100
   taatttaaaa ggatctaggt gaagatcctt tttgataatc tcatgaccaa aatcccttaa 2160
   cgtgagtttt cgttccactg agcgtcagac cccgtagaaa agatcaaagg atcttcttga 2220
   gatccttttt ttctgcgcgt aatctgctgc ttgcaaacaa aaaaaccacc gctaccagcg 2280
   gtggtttgtt tgccggatca agagctacca actctttttc cgaaggtaac tggcttcagc 2340
   agagcgcaga taccaaatac tgtccttcta gtgtagccgt agttaggcca ccacttcaag 2400
   aactctgtag caccgcctac atacctcgct ctgctaatcc tgttaccagt ggctgctgcc 2460
   agtggcgata agtcgtgtct taccgggttg gactcaagac gatagttacc ggataaggcg 2520
   cagcggtcgg gctgaacggg gggttcgtgc acacagccca gcttggagcg aacgacctac 2580
   accgaactga gatacctaca gcgtgagcat tgagaaagcg ccacgcttcc cgaagggaga 2640
   aaggcggaca ggtatccggt aagcggcagg gtcggaacag gagagcgcac gagggagctt 2700
   ccagggggaa acgcctggta tctttatagt cctgcgggtt tcgccacctc tgacttgagc 2760
   gtcgattttt gtgatgctcg tcaggggggc ggagcctatg gaaaaacgcc agcaacgcgg 2820
   cctttttacg gttcctggcc ttttgctggc cttttgctca catgttcttt cctgcgttat 2880
   cccctgattc tgtggataac cgtattaccg cctttgagtg agctgatacc gctcgccgca 2940
   gccgaacgac cgagcgcagc gagtcagtga gcgaggaagc ggaagagcgc ctgatgcggt 3000
   attttctcct tacgcatctg tgcggtattt cacaccgcat acgaacgcca gcaagacgta 3060
   gcccagcgcg tcggccagct tgcaattcgc gctaacttac attaattgcg ttgcgctcac 3120
   tgcccgcttt ccagtcggga aacctgtcgt gccagctgca ttaatgaatc ggccaacgcg 3180
   cggggagagg cggtttgcgt attgggcgcc agggtggttt ttcttttcac cagtgagacg 3240
   ggcaacagct gattgccctt caccgcctgg ccctgagaga gttgcagcaa gcggtccacg 3300
   ctggtttgcc ccagcaggcg aaaatcctgt ttgctggtgg ttaacggcgg gatataacat 3360
   gagctgtctt cggtatcgtc gtatcccact accgagatat ccgcaccaac gcgcagcccg 3420
   gactcggtaa tggcgcgcat tgcgcccagc gccatctgat cgttggcaac cagcatcgca 3480
   gtgggaacga tgccctcatt cagcatttgc atggtttgtt gaaaaccgga catggcactc 3540
   cagtcgcctt cccgttccgc tatcggctga atttgattgc gagtgagata tttatgccag   3600
   ccagccagac gcagacgcgc cgagacagaa cttaatgggc ccgctaacag cgcgatttgc   3660
   tggtgaccca atgcgaccag atgctccacg cccagtcgcg taccgtcttc atgggagaaa   3720
   ataatactgt tgatgggtgt ctggtcagag acatcaagaa ataacgccgg aacattagtg   3780
   caggcagctt ccacagcaat ggcatcctgg tcatccagcg catagttaat gatcagccca   3840
   ctgacgcgtt gcgcgagaag attgtgcacc gccgctttac aggcttcgac gccgcttcgt   3900
   tctaccatcg acaccaccac gctggcaccc agttgatcgg cgcgagattt aatcgccgcg   3960
   acaatttgcg acggcgcgtg cagggccaga ctggaggtgg caacgccaat cagcaacgac   4020
   tgtttgcccg ccagttgttg tgccacgcgg ttgggaatgt aattcagctc cgccatcgcc   4080
   gcttccactt tttcccgcgt tttcgcagaa acgtggctgg cctggttcac cacgcgggaa   4140
   acggtctgat aagagacacc ggcatactct gcgacatcgt ataacgttac tggtttcaca   4200
   ttcaccaccc tgaattgact ctcttccggg cgctatcatg ccataccgcg aaaggttttg   4260
   caccattcga tggtgtcaac gtaaatgccg cttcgccttc gcgcgcgaat tgcaagctga   4320
   tccgggctta tcgactgcac ggtgcaccaa tgcttctggc gtcaggcagc catcggaagc   4380
   tgtggtatgg ctgtgcaggt cgtaaatcac tgcataattc gtgtcgctca aggcgcactc   4440
   ccgttctgga taatgttttt tgcgccgaca tcataacggt tctggcaaat attctgaaat   4500
   gagctgttga caattaatca tcggctcgta taatgtgtgg aattgtgagc ggataacaat   4560
   ttcacacagg aaacagaatt                                               4580
<210> 6
   <211> 5957
   <212> DNA
   <213> artificial
<220>
   <223> completely synthesized plasmit pVIII-CBD-phoA9
<400> 6
   aagcttgcat gcctgcaggt cgacggatcg atccccgtgc cttcgtagtg gcattacgta      60
   ttttacccgt ttaatggaaa cttcctcatg aaaaagtctt tagtcctcaa agcctctgta     120
   gccgttgcta ccctcgttcc gatgctgtct ttcgctgctg agggtgacga tcccgcaaaa     180
   gcggccttta actccctgca agcctcagcg accgaatata tcggttatgc gtgggcgatg     240
   gttgttgtca ttgtcggcgc aactatcggt atcaagctgt ttaagaaatt cacctcgaaa     300
   gcaaggggaa tttcgcgtca ggcagcggcg caggcggctg ccacgacaaa tcctggtgta     360
   tccgcttggc aggtcaacac agcttatact gcgggacaat tggtcacata taacggcaag     420
   acgtataaat gtttgcagcc ccacacctcc ttggcaggat gggaaccatc caacgttcct     480
   gccttgtggc agcttcaagg tggccatggg gactcactgg ataaagcaac gttgaaaaag     540
   gttgcgccga agcctggctg gctggaaacc ggtgcttctg tttttccggt actggctatc     600
   gtattgattg tgcgttcgtt tatttatgaa ccgttccaga tcccgtcagg atcctcgaat     660
   tgccctgttc tggaaaaccg ggctgctcag ggcgatatta ctgcacccgg cggtgctcgc 720
   cgtttaacgg gtgatcagac tgccgctctg cgtgattctc ttagcgataa acctgcaaaa 780
   aatattattt tgctgattgg cgatgggatg ggggactcgg aaattactgc cgcacgtaat 840
   tatgccgaag gtgcgggcgg cttttttaaa ggtatagatg ccttaccgct taccgggcaa 900
   tacactcact atgcgctgaa taaaaaaacc ggcaaaccgg actacgtcac cgactcggct 960
   gcatcagcaa ccgcctggtc aaccggtgtc aaaacctata acggcgcgct gggcgtcgat 1020
   attcacgaaa aagatcaccc aacgattctg gaaatggcaa aagccgcagg tctggcgacc 1080
   ggtaacgttt ctaccgcaga gttgcaggat gccacgcccg ctgcgctggt ggcacatgtg 1140
   acctcgcgca aatgctacgg tccgagcgcg accagtgaaa aatgtccggg taacgctctg 1200
   gaaaaaggcg gaaaaggatc gattaccgaa cagctgctta acgctcgtgc cgacgttacg 1260
   cttggcggcg gcgcaaaaac ctttgctgaa acggcaaccg ctggtgaatg gcagggaaaa 1320
   acgctgcgtg aacaggcaca ggcgcgtggt tatcagttgg tgagcgatgc tgcctcactg 1380
   aattcggtga cggaagcgaa tcagcaaaaa cccctgcttg gcctgtttgc tgacggcaat 1440
   atgccagtgc gctggctagg accgaaagca acgtaccatg gcaatatcga taagcccgca 1500
   gtcacctgta cgccaaatcc gcaacgtaat gacagtgtac caaccctggc gcagatgacc 1560
   gacaaagcca ttgaattgtt gagtaaaaat gagaaaggct ttttcctgca agttgaaggt 1620
   gcgtcaatcg ataaacagga tcatgctgcg aatccttgtg ggcaaattgg cgagacggtc 1680
   gatctcgatg aagccgtaca acgggcgctg gaattcgcta aaaaggaggg taacacgctg 1740
   gtcatagtca ccgctgatca cgcccacgcc agccagattg ttgcgccgga taccaaagct 1800
   ccgggcctca cccaggcgct aaataccaaa gatggcgcag tgatggtgat gagttacggg 1860
   aactccgaag aggattcaca agaacatacc ggcagtcagt tgcgtattgc ggcgtatggc 1920
   ccgcatgccg ccaatgttgt tggactgacc gaccagaccg atctcttcta caccatgaaa 1980
   gccgctctgg ggctgaaata aaaccgcgcc cggcagtgaa ttttcgctgc cggcaattca 2040
   gcttggctgt tttggcggat gagagaagat tttcagcctg atacagatta aatcagaacg 2100
   cagaagcggt ctgataaaac agaatttgcc tggcggcagt agcgcggtgg tcccacctga 2160
   ccccatgccg aactcagaag tgaaacgccg tagcgccgat ggtagtgtgg ggtctcccca 2220
   tgcgagagta gggaactgcc aggcatcaaa taaaacgaaa ggctcagtcg aaagactggg 2280
   cctttcgttt tatctgttgt ttgtcggtga acgctctcct gagtaggaca aatccgccgg 2340
   gagcggattt gaacgttgcg aagcaacggc ccggagggtg gcgggcagga cgcccgccat 2400
   aaactgccag gcatcaaatt aagcagaagg ccatcctgac ggatggcctt tttgcgtttc 2460
   tacaaactct tttgtttatt tttctaaata cattcaaata tgtatccgct catgagacaa 2520
   taaccctgat aaatgcttca ataatattga aaaaggaaga gtatgagtat tcaacatttc 2580
   cgtgtcgccc ttattccctt ttttgcggca ttttgccttc ctgtttttgc tcacccagaa 2640
   acgctggtga aagtaaaaga tgctgaagat cagttgggtg cacgagtggg ttacatcgaa 2700
   ctggatctca acagcggtaa gatccttgag agttttcgcc ccgaagaacg ttttccaatg 2760
   atgagcactt ttaaagttct gctatgtggc gcggtattat cccgtgttga cgccgggcaa 2820
   gagcaactcg gtcgccgcat acactattct cagaatgact tggttgagta ctcaccagtc 2880
   acagaaaagc atcttacgga tggcatgaca gtaagagaat tatgcagtgc tgccataacc 2940
   atgagtgata acactgcggc caacttactt ctgacaacga tcggaggacc gaaggagcta 3000
   accgcttttt tgcacaacat gggggatcat gtaactcgcc ttgatcgttg ggaaccggag 3060
   ctgaatgaag ccataccaaa cgacgagcgt gacaccacga tgcctgcagc aatggcaaca 3120
   acgttgcgca aactattaac tggcgaacta cttactctag cttcccggca acaattaata 3180
   gactggatgg aggcggataa agttgcagga ccacttctgc gctcggccct tccggctggc 3240
   tggtttattg ctgataaatc tggagccggt gagcgtgggt ctcgcggtat cattgcagca 3300
   ctggggccag atggtaagcc ctcccgtatc gtagttatct acacgacggg gagtcaggca 3360
   actatggatg aacgaaatag acagatcgct gagataggtg cctcactgat taagcattgg 3420
   taactgtcag accaagttta ctcatatata ctttagattg atttaaaact tcatttttaa 3480
   tttaaaagga tctaggtgaa gatccttttt gataatctca tgaccaaaat cccttaacgt 3540
   gagttttcgt tccactgagc gtcagacccc gtagaaaaga tcaaaggatc ttcttgagat 3600
   cctttttttc tgcgcgtaat ctgctgcttg caaacaaaaa aaccaccgct accagcggtg 3660
   gtttgtttgc cggatcaaga gctaccaact ctttttccga aggtaactgg cttcagcaga 3720
   gcgcagatac caaatactgt ccttctagtg tagccgtagt taggccacca cttcaagaac 3780
   tctgtagcac cgcctacata cctcgctctg ctaatcctgt taccagtggc tgctgccagt 3840
   ggcgataagt cgtgtcttac cgggttggac tcaagacgat agttaccgga taaggcgcag 3900
   cggtcgggct gaacgggggg ttcgtgcaca cagcccagct tggagcgaac gacctacacc 3960
   gaactgagat acctacagcg tgagcattga gaaagcgcca cgcttcccga agggagaaag 4020
   gcggacaggt atccggtaag cggcagggtc ggaacaggag agcgcacgag ggagcttcca 4080
   gggggaaacg cctggtatct ttatagtcct gcgggtttcg ccacctctga cttgagcgtc 4140
   gatttttgtg atgctcgtca ggggggcgga gcctatggaa aaacgccagc aacgcggcct 4200
   ttttacggtt cctggccttt tgctggcctt ttgctcacat gttctttcct gcgttatccc 4260
   ctgattctgt ggataaccgt attaccgcct ttgagtgagc tgataccgct cgccgcagcc 4320
   gaacgaccga gcgcagcgag tcagtgagcg aggaagcgga agagcgcctg atgcggtatt 4380
   ttctccttac gcatctgtgc ggtatttcac accgcatacg aacgccagca agacgtagcc 4440
   cagcgcgtcg gccagcttgc aattcgcgct aacttacatt aattgcgttg cgctcactgc 4500
   ccgctttcca gtcgggaaac ctgtcgtgcc agctgcatta atgaatcggc caacgcgcgg 4560
   ggagaggcgg tttgcgtatt gggcgccagg gtggtttttc ttttcaccag tgagacgggc 4620
   aacagctgat tgcccttcac cgcctggccc tgagagagtt gcagcaagcg gtccacgctg 4680
   gtttgcccca gcaggcgaaa atcctgtttg ctggtggtta acggcgggat ataacatgag 4740
   ctgtcttcgg tatcgtcgta tcccactacc gagatatccg caccaacgcg cagcccggac    4800
   tcggtaatgg cgcgcattgc gcccagcgcc atctgatcgt tggcaaccag catcgcagtg    4860
   ggaacgatgc cctcattcag catttgcatg gtttgttgaa aaccggacat ggcactccag    4920
   tcgccttccc gttccgctat cggctgaatt tgattgcgag tgagatattt atgccagcca    4980
   gccagacgca gacgcgccga gacagaactt aatgggcccg ctaacagcgc gatttgctgg    5040
   tgacccaatg cgaccagatg ctccacgccc agtcgcgtac cgtcttcatg ggagaaaata    5100
   atactgttga tgggtgtctg gtcagagaca tcaagaaata acgccggaac attagtgcag    5160
   gcagcttcca cagcaatggc atcctggtca tccagcgcat agttaatgat cagcccactg    5220
   acgcgttgcg cgagaagatt gtgcaccgcc gctttacagg cttcgacgcc gcttcgttct    5280
   accatcgaca ccaccacgct ggcacccagt tgatcggcgc gagatttaat cgccgcgaca    5340
   atttgcgacg gcgcgtgcag ggccagactg gaggtggcaa cgccaatcag caacgactgt    5400
   ttgcccgcca gttgttgtgc cacgcggttg ggaatgtaat tcagctccgc catcgccgct    5460
   tccacttttt cccgcgtttt cgcagaaacg tggctggcct ggttcaccac gcgggaaacg    5520
   gtctgataag agacaccggc atactctgcg acatcgtata acgttactgg tttcacattc    5580
   accaccctga attgactctc ttccgggcgc tatcatgcca taccgcgaaa ggttttgcac    5640
   cattcgatgg tgtcaacgta aatgccgctt cgccttcgcg cgcgaattgc aagctgatcc    5700
   gggcttatcg actgcacggt gcaccaatgc ttctggcgtc aggcagccat cggaagctgt    5760
   ggtatggctg tgcaggtcgt aaatcactgc ataattcgtg tcgctcaagg cgcactcccg    5820
   ttctggataa tgttttttgc gccgacatca taacggttct ggcaaatatt ctgaaatgag    5880
   ctgttgacaa ttaatcatcg gctcgtataa tgtgtggaat tgtgagcgga taacaatttc    5940
   acacaggaaa cagaatt                                                   5957
<210> 7
   <211> 5662
   <212> DNA
   <213> artificial
<220>
   <223> completely synthesized plasmid pVIII-CBD-Oxa1p
<400> 7
   aagcttgcat gcctgcaggt cgacggatcg atccccgtgc cttcgtagtg gcattacgta     60
   ttttacccgt ttaatggaaa cttcctcatg aaaaagtctt tagtcctcaa agcctctgta    120
   gccgttgcta ccctcgttcc gatgctgtct ttcgctgctg agggtgacga tcccgcaaaa    180
   gcggccttta actccctgca agcctcagcg accgaatata tcggttatgc gtgggcgatg    240
   gttgttgtca ttgtcggcgc aactatcggt atcaagctgt ttaagaaatt cacctcgaaa    300
   gcaaggggaa tttcgcgtca ggcagcggcg caggcggctg ccacgacaaa tcctggtgta    360
   tccgcttggc aggtcaacac agcttatact gcgggacaat tggtcacata taacggcaag    420
   acgtataaat gtttgcagcc ccacacctcc ttggcaggat gggaaccatc caacgttcct    480
   gccttgtggc agcttcaagg tggccatggg gactcactgg ataaagcaac gttgaaaaag     540
   gttgcgccga agcctggctg gctggaaacc ggtgcttctg tttttccggt actggctatc     600
   gtattgattg tgcgttcgtt tatttatgaa ccgttccaga tcccgtcagg atcctcgaat     660
   tcgacgggcc caaatgccaa cgatgtctcg gaaatccaaa cccagttgcc ttccatcgat     720
   gaattaacct cttcagctcc ttctctttcc gcttctactt cggaccttat cgctaacacg     780
   acccaaacag tgggcgagtt gtcctcccat atagggtact taaatagcat tggcctggcc     840
   caaacctggt actggccctc ggacattatc caacacgtct tggaggccgt tcatgtttac     900
   tctgggttgc cttggtgggg aactatcgcg gccaccacca tcctcattcg atgcctgatg     960
   tttcccctct atgtcaagtc ctctgatact gttgctagaa attcccatat caagcccgag    1020
   ctggacgcct tgaataataa gctaatgtcc actacagatt tgcaacaagg tcagctagtc    1080
   gccatgcaaa ggaaaaaact gctctcctcg cacggcatta agaacagatg gctggccgca    1140
   cccatgctac aaattccaat cgcccttggg tttttcaacg cattgagaca catggctaac    1200
   tacccagtag atgggttcgc taatcaaggt gtcgcttggt ttacagactt gactcaagca    1260
   gacccttact taggtttgca agtaatcact gccgctgtgt tcatctcatt tacaaggctg    1320
   gggggtgaga ctggtgctca acaattcagt tctcccatga agcgtctttt cactattcta    1380
   ccgatcattt ctataccggc cacaatgaac ttatcgtccg ctgtggtcct ctactttgcc    1440
   tttaatggtg ccttctccgt cctacagaca atgattttga gaaacaaatg ggttcgttcg    1500
   aaactgaaga taacagaagt agctaaacca aggactccta tcgctggcgc ttcccccaca    1560
   gagaacatgg gcatcttcca atcattaaaa cataacattc aaaaggcaag agatcaggcg    1620
   gaaagaaggc aattgatgca agataatgag aagaagttac aagaaagctt caaggagaag    1680
   aggcagaact ccaaaatcaa aattgttcac aaatcaaact tcattaataa caaaaaatga    1740
   attcagcttg gctgttttgg cggatgagag aagattttca gcctgataca gattaaatca    1800
   gaacgcagaa gcggtctgat aaaacagaat ttgcctggcg gcagtagcgc ggtggtccca    1860
   cctgacccca tgccgaactc agaagtgaaa cgccgtagcg ccgatggtag tgtggggtct    1920
   ccccatgcga gagtagggaa ctgccaggca tcaaataaaa cgaaaggctc agtcgaaaga    1980
   ctgggccttt cgttttatct gttgtttgtc ggtgaacgct ctcctgagta ggacaaatcc    2040
   gccgggagcg gatttgaacg ttgcgaagca acggcccgga gggtggcggg caggacgccc    2100
   gccataaact gccaggcatc aaattaagca gaaggccatc ctgacggatg gcctttttgc    2160
   gtttctacaa actcttttgt ttatttttct aaatacattc aaatatgtat ccgctcatga    2220
   gacaataacc ctgataaatg cttcaataat attgaaaaag gaagagtatg agtattcaac    2280
   atttccgtgt cgcccttatt cccttttttg cggcattttg ccttcctgtt tttgctcacc    2340
   cagaaacgct ggtgaaagta aaagatgctg aagatcagtt gggtgcacga gtgggttaca    2400
   tcgaactgga tctcaacagc ggtaagatcc ttgagagttt tcgccccgaa gaacgttttc    2460
   caatgatgag cacttttaaa gttctgctat gtggcgcggt attatcccgt gttgacgccg    2520
   ggcaagagca actcggtcgc cgcatacact attctcagaa tgacttggtt gagtactcac 2580
   cagtcacaga aaagcatctt acggatggca tgacagtaag agaattatgc agtgctgcca 2640
   taaccatgag tgataacact gcggccaact tacttctgac aacgatcgga ggaccgaagg 2700
   agctaaccgc ttttttgcac aacatggggg atcatgtaac tcgccttgat cgttgggaac 2760
   cggagctgaa tgaagccata ccaaacgacg agcgtgacac cacgatgcct gcagcaatgg 2820
   caacaacgtt gcgcaaacta ttaactggcg aactacttac tctagcttcc cggcaacaat 2880
   taatagactg gatggaggcg gataaagttg caggaccact tctgcgctcg gcccttccgg 2940
   ctggctggtt tattgctgat aaatctggag ccggtgagcg tgggtctcgc ggtatcattg 3000
   cagcactggg gccagatggt aagccctccc gtatcgtagt tatctacacg acggggagtc 3060
   aggcaactat ggatgaacga aatagacaga tcgctgagat aggtgcctca ctgattaagc 3120
   attggtaact gtcagaccaa gtttactcat atatacttta gattgattta aaacttcatt 3180
   tttaatttaa aaggatctag gtgaagatcc tttttgataa tctcatgacc aaaatccctt 3240
   aacgtgagtt ttcgttccac tgagcgtcag accccgtaga aaagatcaaa ggatcttctt 3300
   gagatccttt ttttctgcgc gtaatctgct gcttgcaaac aaaaaaacca ccgctaccag 3360
   cggtggtttg tttgccggat caagagctac caactctttt tccgaaggta actggcttca 3420
   gcagagcgca gataccaaat actgtccttc tagtgtagcc gtagttaggc caccacttca 3480
   agaactctgt agcaccgcct acatacctcg ctctgctaat cctgttacca gtggctgctg 3540
   ccagtggcga taagtcgtgt cttaccgggt tggactcaag acgatagtta ccggataagg 3600
   cgcagcggtc gggctgaacg gggggttcgt gcacacagcc cagcttggag cgaacgacct 3660
   acaccgaact gagataccta cagcgtgagc attgagaaag cgccacgctt cccgaaggga 3720
   gaaaggcgga caggtatccg gtaagcggca gggtcggaac aggagagcgc acgagggagc 3780
   ttccaggggg aaacgcctgg tatctttata gtcctgcggg tttcgccacc tctgacttga 3840
   gcgtcgattt ttgtgatgct cgtcaggggg gcggagccta tggaaaaacg ccagcaacgc 3900
   ggccttttta cggttcctgg ccttttgctg gccttttgct cacatgttct ttcctgcgtt 3960
   atcccctgat tctgtggata accgtattac cgcctttgag tgagctgata ccgctcgccg 4020
   cagccgaacg accgagcgca gcgagtcagt gagcgaggaa gcggaagagc gcctgatgcg 4080
   gtattttctc cttacgcatc tgtgcggtat ttcacaccgc atacgaacgc cagcaagacg 4140
   tagcccagcg cgtcggccag cttgcaattc gcgctaactt acattaattg cgttgcgctc 4200
   actgcccgct ttccagtcgg gaaacctgtc gtgccagctg cattaatgaa tcggccaacg 4260
   cgcggggaga ggcggtttgc gtattgggcg ccagggtggt ttttcttttc accagtgaga 4320
   cgggcaacag ctgattgccc ttcaccgcct ggccctgaga gagttgcagc aagcggtcca 4380
   cgctggtttg ccccagcagg cgaaaatcct gtttgctggt ggttaacggc gggatataac 4440
   atgagctgtc ttcggtatcg tcgtatccca ctaccgagat atccgcacca acgcgcagcc 4500
   cggactcggt aatggcgcgc attgcgccca gcgccatctg atcgttggca accagcatcg 4560
   cagtgggaac gatgccctca ttcagcattt gcatggtttg ttgaaaaccg gacatggcac    4620
   tccagtcgcc ttcccgttcc gctatcggct gaatttgatt gcgagtgaga tatttatgcc    4680
   agccagccag acgcagacgc gccgagacag aacttaatgg gcccgctaac agcgcgattt    4740
   gctggtgacc caatgcgacc agatgctcca cgcccagtcg cgtaccgtct tcatgggaga    4800
   aaataatact gttgatgggt gtctggtcag agacatcaag aaataacgcc ggaacattag    4860
   tgcaggcagc ttccacagca atggcatcct ggtcatccag cgcatagtta atgatcagcc    4920
   cactgacgcg ttgcgcgaga agattgtgca ccgccgcttt acaggcttcg acgccgcttc    4980
   gttctaccat cgacaccacc acgctggcac ccagttgatc ggcgcgagat ttaatcgccg    5040
   cgacaatttg cgacggcgcg tgcagggcca gactggaggt ggcaacgcca atcagcaacg    5100
   actgtttgcc cgccagttgt tgtgccacgc ggttgggaat gtaattcagc tccgccatcg    5160
   ccgcttccac tttttcccgc gttttcgcag aaacgtggct ggcctggttc accacgcggg    5220
   aaacggtctg ataagagaca ccggcatact ctgcgacatc gtataacgtt actggtttca    5280
   cattcaccac cctgaattga ctctcttccg ggcgctatca tgccataccg cgaaaggttt    5340
   tgcaccattc gatggtgtca acgtaaatgc cgcttcgcct tcgcgcgcga attgcaagct    5400
   gatccgggct tatcgactgc acggtgcacc aatgcttctg gcgtcaggca gccatcggaa    5460
   gctgtggtat ggctgtgcag gtcgtaaatc actgcataat tcgtgtcgct caaggcgcac    5520
   tcccgttctg gataatgttt tttgcgccga catcataacg gttctggcaa atattctgaa    5580
   atgagctgtt gacaattaat catcggctcg tataatgtgt ggaattgtga gcggataaca    5640
   atttcacaca ggaaacagaa tt                                             5662
<210> 8
   <211> 5686
   <212> DNA
   <213> artificial
<220>
   <223> completely synthesized plasmid pVIII-CBD-oxa1p-8His
<400> 8
   aagcttgcat gcctgcaggt cgacggatcg atccccgtgc cttcgtagtg gcattacgta      60
   ttttacccgt ttaatggaaa cttcctcatg aaaaagtctt tagtcctcaa agcctctgta     120
   gccgttgcta ccctcgttcc gatgctgtct ttcgctgctg agggtgacga tcccgcaaaa     180
   gcggccttta actccctgca agcctcagcg accgaatata tcggttatgc gtgggcgatg     240
   gttgttgtca ttgtcggcgc aactatcggt atcaagctgt ttaagaaatt cacctcgaaa     300
   gcaaggggaa tttcgcgtca ggcagcggcg caggcggctg ccacgacaaa tcctggtgta     360
   tccgcttggc aggtcaacac agcttatact gcgggacaat tggtcacata taacggcaag     420
   acgtataaat gtttgcagcc ccacacctcc ttggcaggat gggaaccatc caacgttcct     480
   gccttgtggc agcttcaagg tggccatggg gactcactgg ataaagcaac gttgaaaaag     540
   gttgcgccga agcctggctg gctggaaacc ggtgcttctg tttttccggt actggctatc     600
   gtattgattg tgcgttcgtt tatttatgaa ccgttccaga tcccgtcagg atcctcgaat 660
   tcgacgggcc caaatgccaa cgatgtctcg gaaatccaaa cccagttgcc ttccatcgat 720
   gaattaacct cttcagctcc ttctctttcc gcttctactt cggaccttat cgctaacacg 780
   acccaaacag tgggcgagtt gtcctcccat atagggtact taaatagcat tggcctggcc 840
   caaacctggt actggccctc ggacattatc caacacgtct tggaggccgt tcatgtttac 900
   tctgggttgc cttggtgggg aactatcgcg gccaccacca tcctcattcg atgcctgatg 960
   tttcccctct atgtcaagtc ctctgatact gttgctagaa attcccatat caagcccgag 1020
   ctggacgcct tgaataataa gctaatgtcc actacagatt tgcaacaagg tcagctagtc 1080
   gccatgcaaa ggaaaaaact gctctcctcg cacggcatta agaacagatg gctggccgca 1140
   cccatgctac aaattccaat cgcccttggg tttttcaacg cattgagaca catggctaac 1200
   tacccagtag atgggttcgc taatcaaggt gtcgcttggt ttacagactt gactcaagca 1260
   gacccttact taggtttgca agtaatcact gccgctgtgt tcatctcatt tacaaggctg 1320
   gggggtgaga ctggtgctca acaattcagt tctcccatga agcgtctttt cactattcta 1380
   ccgatcattt ctataccggc cacaatgaac ttatcgtccg ctgtggtcct ctactttgcc 1440
   tttaatggtg ccttctccgt cctacagaca atgattttga gaaacaaatg ggttcgttcg 1500
   aaactgaaga taacagaagt agctaaacca aggactccta tcgctggcgc ttcccccaca 1560
   gagaacatgg gcatcttcca atcattaaaa cataacattc aaaaggcaag agatcaggcg 1620
   gaaagaaggc aattgatgca agataatgag aagaagttac aagaaagctt caaggagaag 1680
   aggcagaact ccaaaatcaa aattgttcac aaatcaaact tcattaataa caaaaaacac 1740
   catcaccatc accatcacca ttgaattcag cttggctgtt ttggcggatg agagaagatt 1800
   ttcagcctga tacagattaa atcagaacgc agaagcggtc tgataaaaca gaatttgcct 1860
   ggcggcagta gcgcggtggt cccacctgac cccatgccga actcagaagt gaaacgccgt 1920
   agcgccgatg gtagtgtggg gtctccccat gcgagagtag ggaactgcca ggcatcaaat 1980
   aaaacgaaag gctcagtcga aagactgggc ctttcgtttt atctgttgtt tgtcggtgaa 2040
   cgctctcctg agtaggacaa atccgccggg agcggatttg aacgttgcga agcaacggcc 2100
   cggagggtgg cgggcaggac gcccgccata aactgccagg catcaaatta agcagaaggc 2160
   catcctgacg gatggccttt ttgcgtttct acaaactctt ttgtttattt ttctaaatac 2220
   attcaaatat gtatccgctc atgagacaat aaccctgata aatgcttcaa taatattgaa 2280
   aaaggaagag tatgagtatt caacatttcc gtgtcgccct tattcccttt tttgcggcat 2340
   tttgccttcc tgtttttgct cacccagaaa cgctggtgaa agtaaaagat gctgaagatc 2400
   agttgggtgc acgagtgggt tacatcgaac tggatctcaa cagcggtaag atccttgaga 2460
   gttttcgccc cgaagaacgt tttccaatga tgagcacttt taaagttctg ctatgtggcg 2520
   cggtattatc ccgtgttgac gccgggcaag agcaactcgg tcgccgcata cactattctc 2580
   agaatgactt ggttgagtac tcaccagtca cagaaaagca tcttacggat ggcatgacag 2640
   taagagaatt atgcagtgct gccataacca tgagtgataa cactgcggcc aacttacttc 2700
   tgacaacgat cggaggaccg aaggagctaa ccgctttttt gcacaacatg ggggatcatg 2760
   taactcgcct tgatcgttgg gaaccggagc tgaatgaagc cataccaaac gacgagcgtg 2820
   acaccacgat gcctgcagca atggcaacaa cgttgcgcaa actattaact ggcgaactac 2880
   ttactctagc ttcccggcaa caattaatag actggatgga ggcggataaa gttgcaggac 2940
   cacttctgcg ctcggccctt ccggctggct ggtttattgc tgataaatct ggagccggtg 3000
   agcgtgggtc tcgcggtatc attgcagcac tggggccaga tggtaagccc tcccgtatcg 3060
   tagttatcta cacgacgggg agtcaggcaa ctatggatga acgaaataga cagatcgctg 3120
   agataggtgc ctcactgatt aagcattggt aactgtcaga ccaagtttac tcatatatac 3180
   tttagattga tttaaaactt catttttaat ttaaaaggat ctaggtgaag atcctttttg 3240
   ataatctcat gaccaaaatc ccttaacgtg agttttcgtt ccactgagcg tcagaccccg 3300
   tagaaaagat caaaggatct tcttgagatc ctttttttct gcgcgtaatc tgctgcttgc 3360
   aaacaaaaaa accaccgcta ccagcggtgg tttgtttgcc ggatcaagag ctaccaactc 3420
   tttttccgaa ggtaactggc ttcagcagag cgcagatacc aaatactgtc cttctagtgt 3480
   agccgtagtt aggccaccac ttcaagaact ctgtagcacc gcctacatac ctcgctctgc 3540
   taatcctgtt accagtggct gctgccagtg gcgataagtc gtgtcttacc gggttggact 3600
   caagacgata gttaccggat aaggcgcagc ggtcgggctg aacggggggt tcgtgcacac 3660
   agcccagctt ggagcgaacg acctacaccg aactgagata cctacagcgt gagcattgag 3720
   aaagcgccac gcttcccgaa gggagaaagg cggacaggta tccggtaagc ggcagggtcg 3780
   gaacaggaga gcgcacgagg gagcttccag ggggaaacgc ctggtatctt tatagtcctg 3840
   cgggtttcgc cacctctgac ttgagcgtcg atttttgtga tgctcgtcac gggggcggag 3900
   cctatggaaa aacgccagca acgcggcctt tttacggttc ctggcctttt gctggccttt 3960
   tgctcacatg ttctttcctg cgttatcccc tgattctgtg gataaccgta ttaccgcctt 4020
   tgagtgagct gataccgctc gccgcagccg aacgaccgag cgcagcgagt cagtgagcga 4080
   ggaagcggaa gagcgcctga tgcggtattt tctccttacg catctgtgcg gtatttcaca 4140
   ccgcatacga acgccagcaa gacgtagccc agcgcgtcgg ccagcttgca attcgcgcta 4200
   acttacatta attgcgttgc gctcactgcc cgctttccag tcgggaaacc tgtcgtgcca 4260
   gctgcattaa tgaatcggcc aacgcgcggg gagaggcggt ttgcgtattg ggcgccaggg 4320
   tggtttttct tttcaccagt gagacgggca acagctgatt gcccttcacc gcctggccct 4380
   gagagagttg cagcaagcgg tccacgctgg tttgccccag caggcgaaaa tcctgtttgc 4440
   tggtggttaa cggcgggata taacatgagc tgtcttcggt atcgtcgtat cccactaccg 4500
   agatatccgc accaacgcgc agcccggact cggtaatggc gcgcattgcg cccagcgcca 4560
   tctgatcgtt ggcaaccagc atcgcagtgg gaacgatgcc ctcattcagc atttgcatgg 4620
   tttgttgaaa accggacatg gcactccagt cgccttcccg ttccgctatc ggctgaattt 4680
   gattgcgagt gagatattta tgccagccag ccagacgcag acgcgccgag acagaactta 4740
   atgggcccgc taacagcgcg atttgctggt gacccaatgc gaccagatgc tccacgccca 4800
   gtcgcgtacc gtcttcatgg gagaaaataa tactgttgat gggtgtctgg tcagagacat 4860
   caagaaataa cgccggaaca ttagtgcagg cagcttccac agcaatggca tcctggtcat 4920
   ccagcgcata gttaatgatc agcccactga cgcgttgcgc gagaagattg tgcaccgccg 4980
   ctttacaggc ttcgacgccg cttcgttcta ccatcgacac caccacgctg gcacccagtt 5040
   gatcggcgcg agatttaatc gccgcgacaa tttgcgacgg cgcgtgcagg gccagactgg 5100
   aggtggcaac gccaatcagc aacgactgtt tgcccgccag ttgttgtgcc acgcggttgg 5160
   gaatgtaatt cagctccgcc atcgccgctt ccactttttc ccgcgttttc gcagaaacgt 5220
   ggctggcctg gttcaccacg cgggaaacgg tctgataaga gacaccggca tactctgcga 5280
   catcgtataa cgttactggt ttcacattca ccaccctgaa ttgactctct tccgggcgct 5340
   atcatgccat accgcgaaag gttttgcacc attcgatggt gtcaacgtaa atgccgcttc 5400
   gccttcgcgc gcgaattgca agctgatccg ggcttatcga ctgcacggtg caccaatgct 5460
   tctggcgtca ggcagccatc ggaagctgtg gtatggctgt gcaggtcgta aatcactgca 5520
   taattcgtgt cgctcaaggc gcactcccgt tctggataat gttttttgcg ccgacatcat 5580
   aacggttctg gcaaatattc tgaaatgagc tgttgacaat taatcatcgg ctcgtataat 5640
   gtgtggaatt gtgagcggat aacaatttca cacaggaaac agaatt 5686
<210> 9
   <211> 5716
   <212> DNA
   <213> artificial
<220>
   <223> completely synthesized plasmid pVIII-CBD-ent-oxa1p-8His
<400> 9
   aagcttgcat gcctgcaggt cgacggatcg atccccgtgc cttcgtagtg gcattacgta     60
   ttttacccgt ttaatggaaa cttcctcatg aaaaagtctt tagtcctcaa agcctctgta    120
   gccgttgcta ccctcgttcc gatgctgtct ttcgctgctg agggtgacga tcccgcaaaa    180
   gcggccttta actccctgca agcctcagcg accgaatata tcggttatgc gtgggcgatg    240
   gttgttgtca ttgtcggcgc aactatcggt atcaagctgt ttaagaaatt cacctcgaaa    300
   gcaaggggaa tttcgcgtca ggcagcggcg caggcggctg ccacgacaaa tcctggtgta    360
   tccgcttggc aggtcaacac agcttatact gcgggacaat tggtcacata taacggcaag    420
   acgtataaat gtttgcagcc ccacacctcc ttggcaggat gggaaccatc caacgttcct    480
   gccttgtggc agcttcaagg tggccatggg gactcactgg ataaagcaac gttgaaaaag    540
   gttgcgccga agcctggctg gctggaaacc ggtgcttctg tttttccggt actggctatc    600
   gtattgattg tgcgttcgtt tatttatgaa ccgttccaga tcccgtcagg atcggactac    660
   aaagatgacg atgacaaagg atcctcgaat tcgacgggcc caaatgccaa cgatgtctcg    720
   gaaatccaaa cccagttgcc ttccatcgat gaattaacct cttcagctcc ttctctttcc 780
   gcttctactt cggaccttat cgctaacacg acccaaacag tgggcgagtt gtcctcccat 840
   atagggtact taaatagcat tggcctggcc caaacctggt actggccctc ggacattatc 900
   caacacgtct tggaggccgt tcatgtttac tctgggttgc cttggtgggg aactatcgcg 960
   gccaccacca tcctcattcg atgcctgatg tttcccctct atgtcaagtc ctctgatact 1020
   gttgctagaa attcccatat caagcccgag ctggacgcct tgaataataa gctaatgtcc 1080
   actacagatt tgcaacaagg tcagctagtc gccatgcaaa ggaaaaaact gctctcctcg 1140
   cacggcatta agaacagatg gctggccgca cccatgctac aaattccaat cgcccttggg 1200
   tttttcaacg cattgagaca catggctaac tacccagtag atgggttcgc taatcaaggt 1260
   gtcgcttggt ttacagactt gactcaagca gacccttact taggtttgca agtaatcact 1320
   gccgctgtgt tcatctcatt tacaaggctg gggggtgaga ctggtgctca acaattcagt 1380
   tctcccatga agcgtctttt cactattcta ccgatcattt ctataccggc cacaatgaac 1440
   ttatcgtccg ctgtggtcct ctactttgcc tttaatggtg ccttctccgt cctacagaca 1500
   atgattttga gaaacaaatg ggttcgttcg aaactgaaga taacagaagt agctaaacca 1560
   aggactccta tcgctggcgc ttcccccaca gagaacatgg gcatcttcca atcattaaaa 1620
   cataacattc aaaaggcaag agatcaggcg gaaagaaggc aattgatgca agataatgag 1680
   aagaagttac aagaaagctt caaggagaag aggcagaact ccaaaatcaa aattgttcac 1740
   aaatcaaact tcattaataa caaaaaacac catcaccatc accatcacca ttgaattcag 1800
   cttggctgtt ttggcggatg agagaagatt ttcagcctga tacagattaa atcagaacgc 1860
   agaagcggtc tgataaaaca gaatttgcct ggcggcagta gcgcggtggt cccacctgac 1920
   cccatgccga actcagaagt gaaacgccgt agcgccgatg gtagtgtggg gtctccccat 1980
   gcgagagtag ggaactgcca ggcatcaaat aaaacgaaag gctcagtcga aagactgggc 2040
   ctttcgtttt atctgttgtt tgtcggtgaa cgctctcctg agtaggacaa atccgccggg 2100
   agcggatttg aacgttgcga agcaacggcc cggagggtgg cgggcaggac gcccgccata 2160
   aactgccagg catcaaatta agcagaaggc catcctgacg gatggccttt ttgcgtttct 2220
   acaaactctt ttgtttattt ttctaaatac attcaaatat gtatccgctc atgagacaat 2280
   aaccctgata aatgcttcaa taatattgaa aaaggaagag tatgagtatt caacatttcc 2340
   gtgtcgccct tattcccttt tttgcggcat tttgccttcc tgtttttgct cacccagaaa 2400
   cgctggtgaa agtaaaagat gctgaagatc agttgggtgc acgagtgggt tacatcgaac 2460
   tggatctcaa cagcggtaag atccttgaga gttttcgccc cgaagaacgt tttccaatga 2520
   tgagcacttt taaagttctg ctatgtggcg cggtattatc ccgtgttgac gccgggcaag 2580
   agcaactcgg tcgccgcata cactattctc agaatgactt ggttgagtac tcaccagtca 2640
   cagaaaagca tcttacggat ggcatgacag taagagaatt atgcagtgct gccataacca 2700
   tgagtgataa cactgcggcc aacttacttc tgacaacgat cggaggaccg aaggagctaa 2760
   ccgctttttt gcacaacatg ggggatcatg taactcgcct tgatcgttgg gaaccggagc 2820
   tgaatgaagc cataccaaac gacgagcgtg acaccacgat gcctgcagca atggcaacaa 2880
   cgttgcgcaa actattaact ggcgaactac ttactctagc ttcccggcaa caattaatag 2940
   actggatgga ggcggataaa gttgcaggac cacttctgcg ctcggccctt ccggctggct 3000
   ggtttattgc tgataaatct ggagccggtg agcgtgggtc tcgcggtatc attgcagcac 3060
   tggggccaga tggtaagccc tcccgtatcg tagttatcta cacgacgggg agtcaggcaa 3120
   ctatggatga acgaaataga cagatcgctg agataggtgc ctcactgatt aagcattggt 3180
   aactgtcaga ccaagtttac tcatatatac tttagattga tttaaaactt catttttaat 3240
   ttaaaaggat ctaggtgaag atcctttttg ataatctcat gaccaaaatc ccttaacgtg 3300
   agttttcgtt ccactgagcg tcagaccccg tagaaaagat caaaggatct tcttgagatc 3360
   ctttttttct gcgcgtaatc tgctgcttgc aaacaaaaaa accaccgcta ccagcggtgg 3420
   tttgtttgcc ggatcaagag ctaccaactc tttttccgaa ggtaactggc ttcagcagag 3480
   cgcagatacc aaatactgtc cttctagtgt agccgtagtt aggccaccac ttcaagaact 3540
   ctgtagcacc gcctacatac ctcgctctgc taatcctgtt accagtggct gctgccagtg 3600
   gcgataagtc gtgtcttacc gggttggact caagacgata gttaccggat aaggcgcagc 3660
   ggtcgggctg aacggggggt tcgtgcacac agcccagctt ggagcgaacg acctacaccg 3720
   aactgagata cctacagcgt gagcattgag aaagcgccac gcttcccgaa gggagaaagg 3780
   cggacaggta tccggtaagc ggcagggtcg gaacaggaga gcgcacgagg gagcttccag 3840
   ggggaaacgc ctggtatctt tatagtcctg cgggtttcgc cacctctgac ttgagcgtcg 3900
   atttttgtga tgctcgtcac gggggcggag cctatggaaa aacgccagca acgcggcctt 3960
   tttacggttc ctggcctttt gctggccttt tgctcacatg ttctttcctg cgttatcccc 4020
   tgattctgtg gataaccgta ttaccgcctt tgagtgagct gataccgctc gccgcagccg 4080
   aacgaccgag cgcagcgagt cagtgagcga ggaagcggaa gagcgcctga tgcggtattt 4140
   tctccttacg catctgtgcg gtatttcaca ccgcatacga acgccagcaa gacgtagccc 4200
   agcgcgtcgg ccagcttgca attcgcgcta acttacatta attgcgttgc gctcactgcc 4260
   cgctttccag tcgggaaacc tgtcgtgcca gctgcattaa tgaatcggcc aacgcgcggg 4320
   gagaggcggt ttgcgtattg ggcgccaggg tggtttttct tttcaccagt gagacgggca 4380
   acagctgatt gcccttcacc gcctggccct gagagagttg cagcaagcgg tccacgctgg 4440
   tttgccccag caggcgaaaa tcctgtttgc tggtggttaa cggcgggata taacatgagc 4500
   tgtcttcggt atcgtcgtat cccactaccg agatatccgc accaacgcgc agcccggact 4560
   cggtaatggc gcgcattgcg cccagcgcca tctgatcgtt ggcaaccagc atcgcagtgg 4620
   gaacgatgcc ctcattcagc atttgcatgg tttgttgaaa accggacatg gcactccagt 4680
   cgccttcccg ttccgctatc ggctgaattt gattgcgagt gagatattta tgccagccag 4740
   ccagacgcag acgcgccgag acagaactta atgggcccgc taacagcgcg atttgctggt 4800
   gacccaatgc gaccagatgc tccacgccca gtcgcgtacc gtcttcatgg gagaaaataa 4860
   tactgttgat gggtgtctgg tcagagacat caagaaataa cgccggaaca ttagtgcagg 4920
   cagcttccac agcaatggca tcctggtcat ccagcgcata gttaatgatc agcccactga 4980
   cgcgttgcgc gagaagattg tgcaccgccg ctttacaggc ttcgacgccg cttcgttcta 5040
   ccatcgacac caccacgctg gcacccagtt gatcggcgcg agatttaatc gccgcgacaa 5100
   tttgcgacgg cgcgtgcagg gccagactgg aggtggcaac gccaatcagc aacgactgtt 5160
   tgcccgccag ttgttgtgcc acgcggttgg gaatgtaatt cagctccgcc atcgccgctt 5220
   ccactttttc ccgcgttttc gcagaaacgt ggctggcctg gttcaccacg cgggaaacgg 5280
   tctgataaga gacaccggca tactctgcga catcgtataa cgttactggt ttcacattca 5340
   ccaccctgaa ttgactctct tccgggcgct atcatgccat accgcgaaag gttttgcacc 5400
   attcgatggt gtcaacgtaa atgccgcttc gccttcgcgc gcgaattgca agctgatccg 5460
   ggcttatcga ctgcacggtg caccaatgct tctggcgtca ggcagccatc ggaagctgtg 5520
   gtatggctgt gcaggtcgta aatcactgca taattcgtgt cgctcaaggc gcactcccgt 5580
   tctggataat gttttttgcg ccgacatcat aacggttctg gcaaatattc tgaaatgagc 5640
   tgttgacaat taatcatcgg ctcgtataat gtgtggaatt gtgagcggat aacaatttca 5700
   cacaggaaac agaatt 5716
<210> 10
   <211> 5710
   <212> DNA
   <213> artificial
<220>
   <223> completely synthesized plasmid pVIII-CBD-tev-oxa1p-8His
<400> 10
   aagcttgcat gcctgcaggt cgacggatcg atccccgtgc cttcgtagtg gcattacgta    60
   ttttacccgt ttaatggaaa cttcctcatg aaaaagtctt tagtcctcaa agcctctgta   120
   gccgttgcta ccctcgttcc gatgctgtct ttcgctgctg agggtgacga tcccgcaaaa   180
   gcggccttta actccctgca agcctcagcg accgaatata tcggttatgc gtgggcgatg   240
   gttgttgtca ttgtcggcgc aactatcggt atcaagctgt ttaagaaatt cacctcgaaa   300
   gcaaggggaa tttcgcgtca ggcagcggcg caggcggctg ccacgacaaa tcctggtgta   360
   tccgcttggc aggtcaacac agcttatact gcgggacaat tggtcacata taacggcaag   420
   acgtataaat gtttgcagcc ccacacctcc ttggcaggat gggaaccatc caacgttcct   480
   gccttgtggc agcttcaagg tggccatggg gactcactgg ataaagcaac gttgaaaaag   540
   gttgcgccga agcctggctg gctggaaacc ggtgcttctg tttttccggt actggctatc   600
   gtattgattg tgcgttcgtt tatttatgaa ccgttccaga tcccgtcagg atcggagaac   660
   ctgtacttcc agggatcctc gaattcgacg ggcccaaatg ccaacgatgt ctcggaaatc   720
   caaacccagt tgccttccat cgatgaatta acctcttcag ctccttctct ttccgcttct   780
   acttcggacc ttatcgctaa cacgacccaa acagtgggcg agttgtcctc ccatataggg 840
   tacttaaata gcattggcct ggcccaaacc tggtactggc cctcggacat tatccaacac 900
   gtcttggagg ccgttcatgt ttactctggg ttgccttggt ggggaactat cgcggccacc 960
   accatcctca ttcgatgcct gatgtttccc ctctatgtca agtcctctga tactgttgct 1020
   agaaattccc atatcaagcc cgagctggac gccttgaata ataagctaat gtccactaca 1080
   gatttgcaac aaggtcagct agtcgccatg caaaggaaaa aactgctctc ctcgcacggc 1140
   attaagaaca gatggctggc cgcacccatg ctacaaattc caatcgccct tgggtttttc 1200
   aacgcattga gacacatggc taactaccca gtagatgggt tcgctaatca aggtgtcgct 1260
   tggtttacag acttgactca agcagaccct tacttaggtt tgcaagtaat cactgccgct 1320
   gtgttcatct catttacaag gctggggggt gagactggtg ctcaacaatt cagttctccc 1380
   atgaagcgtc ttttcactat tctaccgatc atttctatac cggccacaat gaacttatcg 1440
   tccgctgtgg tcctctactt tgcctttaat ggtgccttct ccgtcctaca gacaatgatt 1500
   ttgagaaaca aatgggttcg ttcgaaactg aagataacag aagtagctaa accaaggact 1560
   cctatcgctg gcgcttcccc cacagagaac atgggcatct tccaatcatt aaaacataac 1620
   attcaaaagg caagagatca ggcggaaaga aggcaattga tgcaagataa tgagaagaag 1680
   ttacaagaaa gcttcaagga gaagaggcag aactccaaaa tcaaaattgt tcacaaatca 1740
   aacttcatta ataacaaaaa acaccatcac catcaccatc accattgaat tcagcttggc 1800
   tgttttggcg gatgagagaa gattttcagc ctgatacaga ttaaatcaga acgcagaagc 1860
   ggtctgataa aacagaattt gcctggcggc agtagcgcgg tggtcccacc tgaccccatg 1920
   ccgaactcag aagtgaaacg ccgtagcgcc gatggtagtg tggggtctcc ccatgcgaga 1980
   gtagggaact gccaggcatc aaataaaacg aaaggctcag tcgaaagact gggcctttcg 2040
   ttttatctgt tgtttgtcgg tgaacgctct cctgagtagg acaaatccgc cgggagcgga 2100
   tttgaacgtt gcgaagcaac ggcccggagg gtggcgggca ggacgcccgc cataaactgc 2160
   caggcatcaa attaagcaga aggccatcct gacggatggc ctttttgcgt ttctacaaac 2220
   tcttttgttt atttttctaa atacattcaa atatgtatcc gctcatgaga caataaccct 2280
   gataaatgct tcaataatat tgaaaaagga agagtatgag tattcaacat ttccgtgtcg 2340
   cccttattcc cttttttgcg gcattttgcc ttcctgtttt tgctcaccca gaaacgctgg 2400
   tgaaagtaaa agatgctgaa gatcagttgg gtgcacgagt gggttacatc gaactggatc 2460
   tcaacagcgg taagatcctt gagagttttc gccccgaaga acgttttcca atgatgagca 2520
   cttttaaagt tctgctatgt ggcgcggtat tatcccgtgt tgacgccggg caagagcaac 2580
   tcggtcgccg catacactat tctcagaatg acttggttga gtactcacca gtcacagaaa 2640
   agcatcttac ggatggcatg acagtaagag aattatgcag tgctgccata accatgagtg 2700
   ataacactgc ggccaactta cttctgacaa cgatcggagg accgaaggag ctaaccgctt 2760
   ttttgcacaa catgggggat catgtaactc gccttgatcg ttgggaaccg gagctgaatg 2820
   aagccatacc aaacgacgag cgtgacacca cgatgcctgc agcaatggca acaacgttgc 2880
   gcaaactatt aactggcgaa ctacttactc tagcttcccg gcaacaatta atagactgga 2940
   tggaggcgga taaagttgca ggaccacttc tgcgctcggc ccttccggct ggctggttta 3000
   ttgctgataa atctggagcc ggtgagcgtg ggtctcgcgg tatcattgca gcactggggc 3060
   cagatggtaa gccctcccgt atcgtagtta tctacacgac ggggagtcag gcaactatgg 3120
   atgaacgaaa tagacagatc gctgagatag gtgcctcact gattaagcat tggtaactgt 3180
   cagaccaagt ttactcatat atactttaga ttgatttaaa acttcatttt taatttaaaa 3240
   ggatctaggt gaagatcctt tttgataatc tcatgaccaa aatcccttaa cgtgagtttt 3300
   cgttccactg agcgtcagac cccgtagaaa agatcaaagg atcttcttga gatccttttt 3360
   ttctgcgcgt aatctgctgc ttgcaaacaa aaaaaccacc gctaccagcg gtggtttgtt 3420
   tgccggatca agagctacca actctttttc cgaaggtaac tggcttcagc agagcgcaga 3480
   taccaaatac tgtccttcta gtgtagccgt agttaggcca ccacttcaag aactctgtag 3540
   caccgcctac atacctcgct ctgctaatcc tgttaccagt ggctgctgcc agtggcgata 3600
   agtcgtgtct taccgggttg gactcaagac gatagttacc ggataaggcg cagcggtcgg 3660
   gctgaacggg gggttcgtgc acacagccca gcttggagcg aacgacctac accgaactga 3720
   gatacctaca gcgtgagcat tgagaaagcg ccacgcttcc cgaagggaga aaggcggaca 3780
   ggtatccggt aagcggcagg gtcggaacag gagagcgcac gagggagctt ccagggggaa 3840
   acgcctggta tctttatagt cctgcgggtt tcgccacctc tgacttgagc gtcgattttt 3900
   gtgatgctcg tcaggggggc ggagcctatg gaaaaacgcc agcaacgcgg cctttttacg 3960
   gttcctggcc ttttgctggc cttttgctca catgttcttt cctgcgttat cccctgattc 4020
   tgtggataac cgtattaccg cctttgagtg agctgatacc gctcgccgca gccgaacgac 4080
   cgagcgcagc gagtcagtga gcgaggaagc ggaagagcgc ctgatgcggt attttctcct 4140
   tacgcatctg tgcggtattt cacaccgcat acgaacgcca gcaagacgta gcccagcgcg 4200
   tcggccagct tgcaattcgc gctaacttac attaattgcg ttgcgctcac tgcccgcttt 4260
   ccagtcggga aacctgtcgt gccagctgca ttaatgaatc ggccaacgcg cggggagagg 4320
   cggtttgcgt attgggcgcc agggtggttt ttcttttcac cagtgagacg ggcaacagct 4380
   gattgccctt caccgcctgg ccctgagaga gttgcagcaa gcggtccacg ctggtttgcc 4440
   ccagcaggcg aaaatcctgt ttgctggtgg ttaacggcgg gatataacat gagctgtctt 4500
   cggtatcgtc gtatcccact accgagatat ccgcaccaac gcgcagcccg gactcggtaa 4560
   tggcgcgcat tgcgcccagc gccatctgat cgttggcaac cagcatcgca gtgggaacga 4620
   tgccctcatt cagcatttgc atggtttgtt gaaaaccgga catggcactc cagtcgcctt 4680
   cccgttccgc tatcggctga atttgattgc gagtgagata tttatgccag ccagccagac 4740
   gcagacgcgc cgagacagaa cttaatgggc ccgctaacag cgcgatttgc tggtgaccca 4800
   atgcgaccag atgctccacg cccagtcgcg taccgtcttc atgggagaaa ataatactgt 4860
   tgatgggtgt ctggtcagag acatcaagaa ataacgccgg aacattagtg caggcagctt   4920
   ccacagcaat ggcatcctgg tcatccagcg catagttaat gatcagccca ctgacgcgtt   4980
   gcgcgagaag attgtgcacc gccgctttac aggcttcgac gccgcttcgt tctaccatcg   5040
   acaccaccac gctggcaccc agttgatcgg cgcgagattt aatcgccgcg acaatttgcg   5100
   acggcgcgtg cagggccaga ctggaggtgg caacgccaat cagcaacgac tgtttgcccg   5160
   ccagttgttg tgccacgcgg ttgggaatgt aattcagctc cgccatcgcc gcttccactt   5220
   tttcccgcgt tttcgcagaa acgtggctgg cctggttcac cacgcgggaa acggtctgat   5280
   aagagacacc ggcatactct gcgacatcgt ataacgttac tggtttcaca ttcaccaccc   5340
   tgaattgact ctcttccggg cgctatcatg ccataccgcg aaaggttttg caccattcga   5400
   tggtgtcaac gtaaatgccg cttcgccttc gcgcgcgaat tgcaagctga tccgggctta   5460
   tcgactgcac ggtgcaccaa tgcttctggc gtcaggcagc catcggaagc tgtggtatgg   5520
   ctgtgcaggt cgtaaatcac tgcataattc gtgtcgctca aggcgcactc ccgttctgga   5580
   taatgttttt tgcgccgaca tcataacggt tctggcaaat attctgaaat gagctgttga   5640
   caattaatca tcggctcgta taatgtgtgg aattgtgagc ggataacaat ttcacacagg   5700
   aaacagaatt                                                          5710
<210> 11
   <211> 5657
   <212> DNA
   <213> artificial
<220>
   <223> completely synthesized plasmid Foc(L31F)-phoA9
<400> 11
   aagcttgcat gcctgcaggt cgacggatcg atccccgtgc cttcgtagtg gcattacgta 60
   ttttacccgt ttaatggaaa cttcctcatg gaaaacctga atatggatct gctgtacatg 120
   gctgccgctg tgatgatggg tctggcggca atcggtgctg cgatcggtat cggcatcttc 180
   gggggtaaat tcctggaagg cgcagcgcgt caacctgatc tgattcctct gctgcgtact 240
   cagttcttta tcgttatggg tctggtggat gctatcccga tgatcgctgt aggtctgggt 300
   ctgtacgtga tgttcgctgt cgcgtatgaa ccgttccaga tcccgtcagg atcctcgaat 360
   tgccctgttc tggaaaaccg ggctgctcag ggcgatatta ctgcacccgg cggtgctcgc 420
   cgtttaacgg gtgatcagac tgccgctctg cgtgattctc ttagcgataa acctgcaaaa 480
   aatattattt tgctgattgg cgatgggatg ggggactcgg aaattactgc cgcacgtaat 540
   tatgccgaag gtgcgggcgg cttttttaaa ggtatagatg ccttaccgct taccgggcaa 600
   tacactcact atgcgctgaa taaaaaaacc ggcaaaccgg actacgtcac cgactcggct 660
   gcatcagcaa ccgcctggtc aaccggtgtc aaaacctata acggcgcgct gggcgtcgat 720
   attcacgaaa aagatcaccc aacgattctg gaaatggcaa aagccgcagg tctggcgacc 780
   ggtaacgttt ctaccgcaga gttgcaggat gccacgcccg ctgcgctggt ggcacatgtg 840
   acctcgcgca aatgctacgg tccgagcgcg accagtgaaa aatgtccggg taacgctctg 900
   gaaaaaggcg gaaaaggatc gattaccgaa cagctgctta acgctcgtgc cgacgttacg 960
   cttggcggcg gcgcaaaaac ctttgctgaa acggcaaccg ctggtgaatg gcagggaaaa 1020
   acgctgcgtg aacaggcaca ggcgcgtggt tatcagttgg tgagcgatgc tgcctcactg 1080
   aattcggtga cggaagcgaa tcagcaaaaa cccctgcttg gcctgtttgc tgacggcaat 1140
   atgccagtgc gctggctagg accgaaagca acgtaccatg gcaatatcga taagcccgca 1200
   gtcacctgta cgccaaatcc gcaacgtaat gacagtgtac caaccctggc gcagatgacc 1260
   gacaaagcca ttgaattgtt gagtaaaaat gagaaaggct ttttcctgca agttgaaggt 1320
   gcgtcaatcg ataaacagga tcatgctgcg aatccttgtg ggcaaattgg cgagacggtc 1380
   gatctcgatg aagccgtaca acgggcgctg gaattcgcta aaaaggaggg taacacgctg 1440
   gtcatagtca ccgctgatca cgcccacgcc agccagattg ttgcgccgga taccaaagct 1500
   ccgggcctca cccaggcgct aaataccaaa gatggcgcag tgatggtgat gagttacggg 1560
   aactccgaag aggattcaca agaacatacc ggcagtcagt tgcgtattgc ggcgtatggc 1620
   ccgcatgccg ccaatgttgt tggactgacc gaccagaccg atctcttcta caccatgaaa 1680
   gccgctctgg ggctgaaata aaaccgcgcc cggcagtgaa ttttcgctgc cggcaattca 1740
   gcttggctgt tttggcggat gagagaagat tttcagcctg atacagatta aatcagaacg 1800
   cagaagcggt ctgataaaac agaatttgcc tggcggcagt agcgcggtgg tcccacctga 1860
   ccccatgccg aactcagaag tgaaacgccg tagcgccgat ggtagtgtgg ggtctcccca 1920
   tgcgagagta gggaactgcc aggcatcaaa taaaacgaaa ggctcagtcg aaagactggg 1980
   cctttcgttt tatctgttgt ttgtcggtga acgctctcct gagtaggaca aatccgccgg 2040
   gagcggattt gaacgttgcg aagcaacggc ccggagggtg gcgggcagga cgcccgccat 2100
   aaactgccag gcatcaaatt aagcagaagg ccatcctgac ggatggcctt tttgcgtttc 2160
   tacaaactct tttgtttatt tttctaaata cattcaaata tgtatccgct catgagacaa 2220
   taaccctgat aaatgcttca ataatattga aaaaggaaga gtatgagtat tcaacatttc 2280
   cgtgtcgccc ttattccctt ttttgcggca ttttgccttc ctgtttttgc tcacccagaa 2340
   acgctggtga aagtaaaaga tgctgaagat cagttgggtg cacgagtggg ttacatcgaa 2400
   ctggatctca acagcggtaa gatccttgag agttttcgcc ccgaagaacg ttttccaatg 2460
   atgagcactt ttaaagttct gctatgtggc gcggtattat cccgtgttga cgccgggcaa 2520
   gagcaactcg gtcgccgcat acactattct cagaatgact tggttgagta ctcaccagtc 2580
   acagaaaagc atcttacgga tggcatgaca gtaagagaat tatgcagtgc tgccataacc 2640
   atgagtgata acactgcggc caacttactt ctgacaacga tcggaggacc gaaggagcta 2700
   accgcttttt tgcacaacat gggggatcat gtaactcgcc ttgatcgttg ggaaccggag 2760
   ctgaatgaag ccataccaaa cgacgagcgt gacaccacga tgcctgcagc aatggcaaca 2820
   acgttgcgca aactattaac tggcgaacta cttactctag cttcccggca acaattaata 2880
   gactggatgg aggcggataa agttgcagga ccacttctgc gctcggccct tccggctggc 2940
   tggtttattg ctgataaatc tggagccggt gagcgtgggt ctcgcggtat cattgcagca 3000
   ctggggccag atggtaagcc ctcccgtatc gtagttatct acacgacggg gagtcaggca 3060
   actatggatg aacgaaatag acagatcgct gagataggtg cctcactgat taagcattgg 3120
   taactgtcag accaagttta ctcatatata ctttagattg atttaaaact tcatttttaa 3180
   tttaaaagga tctaggtgaa gatccttttt gataatctca tgaccaaaat cccttaacgt 3240
   gagttttcgt tccactgagc gtcagacccc gtagaaaaga tcaaaggatc ttcttgagat 3300
   cctttttttc tgcgcgtaat ctgctgcttg caaacaaaaa aaccaccgct accagcggtg 3360
   gtttgtttgc cggatcaaga gctaccaact ctttttccga aggtaactgg cttcagcaga 3420
   gcgcagatac caaatactgt ccttctagtg tagccgtagt taggccacca cttcaagaac 3480
   tctgtagcac cgcctacata cctcgctctg ctaatcctgt taccagtggc tgctgccagt 3540
   ggcgataagt cgtgtcttac cgggttggac tcaagacgat agttaccgga taaggcgcag 3600
   cggtcgggct gaacgggggg ttcgtgcaca cagcccagct tggagcgaac gacctacacc 3660
   gaactgagat acctacagcg tgagcattga gaaagcgcca cgcttcccga agggagaaag 3720
   gcggacaggt atccggtaag cggcagggtc ggaacaggag agcgcacgag ggagcttcca 3780
   gggggaaacg cctggtatct ttatagtcct gcgggtttcg ccacctctga cttgagcgtc 3840
   gatttttgtg atgctcgtca ggggggcgga gcctatggaa aaacgccagc aacgcggcct 3900
   ttttacggtt cctggccttt tgctggcctt ttgctcacat gttctttcct gcgttatccc 3960
   ctgattctgt ggataaccgt attaccgcct ttgagtgagc tgataccgct cgccgcagcc 4020
   gaacgaccga gcgcagcgag tcagtgagcg aggaagcgga agagcgcctg atgcggtatt 4080
   ttctccttac gcatctgtgc ggtatttcac accgcatacg aacgccagca agacgtagcc 4140
   cagcgcgtcg gccagcttgc aattcgcgct aacttacatt aattgcgttg cgctcactgc 4200
   ccgctttcca gtcgggaaac ctgtcgtgcc agctgcatta atgaatcggc caacgcgcgg 4260
   ggagaggcgg tttgcgtatt gggcgccagg gtggtttttc ttttcaccag tgagacgggc 4320
   aacagctgat tgcccttcac cgcctggccc tgagagagtt gcagcaagcg gtccacgctg 4380
   gtttgcccca gcaggcgaaa atcctgtttg ctggtggtta acggcgggat ataacatgag 4440
   ctgtcttcgg tatcgtcgta tcccactacc gagatatccg caccaacgcg cagcccggac 4500
   tcggtaatgg cgcgcattgc gcccagcgcc atctgatcgt tggcaaccag catcgcagtg 4560
   ggaacgatgc cctcattcag catttgcatg gtttgttgaa aaccggacat ggcactccag 4620
   tcgccttccc gttccgctat cggctgaatt tgattgcgag tgagatattt atgccagcca 4680
   gccagacgca gacgcgccga gacagaactt aatgggcccg ctaacagcgc gatttgctgg 4740
   tgacccaatg cgaccagatg ctccacgccc agtcgcgtac cgtcttcatg ggagaaaata 4800
   atactgttga tgggtgtctg gtcagagaca tcaagaaata acgccggaac attagtgcag 4860
   gcagcttcca cagcaatggc atcctggtca tccagcgcat agttaatgat cagcccactg 4920
   acgcgttgcg cgagaagatt gtgcaccgcc gctttacagg cttcgacgcc gcttcgttct 4980
   accatcgaca ccaccacgct ggcacccagt tgatcggcgc gagatttaat cgccgcgaca 5040
   atttgcgacg gcgcgtgcag ggccagactg gaggtggcaa cgccaatcag caacgactgt 5100
   ttgcccgcca gttgttgtgc cacgcggttg ggaatgtaat tcagctccgc catcgccgct 5160
   tccacttttt cccgcgtttt cgcagaaacg tggctggcct ggttcaccac gcgggaaacg 5220
   gtctgataag agacaccggc atactctgcg acatcgtata acgttactgg tttcacattc 5280
   accaccctga attgactctc ttccgggcgc tatcatgcca taccgcgaaa ggttttgcac 5340
   cattcgatgg tgtcaacgta aatgccgctt cgccttcgcg cgcgaattgc aagctgatcc 5400
   gggcttatcg actgcacggt gcaccaatgc ttctggcgtc aggcagccat cggaagctgt 5460
   ggtatggctg tgcaggtcgt aaatcactgc ataattcgtg tcgctcaagg cgcactcccg 5520
   ttctggataa tgttttttgc gccgacatca taacggttct ggcaaatatt ctgaaatgag 5580
   ctgttgacaa ttaatcatcg gctcgtataa tgtgtggaat tgtgagcgga taacaatttc 5640
   acacaggaaa cagaatt 5657
<210> 12
   <211> 5687
   <212> DNA
   <213> artificial
<220>
   <223> completely synthesized plasmid Foc(L31F-10His)-PhoA9
<400> 12
   aagcttgcat gcctgcaggt cgacggatcg atccccgtgc cttcgtagtg gcattacgta    60
   ttttacccgt ttaatggaaa cttcctcatg gaaaacctga atatggatct gctgtacatg   120
   gctgccgctg tgatgatggg tctggcggca atcggtgctg cgatcggtat cggcatcttc   180
   gggggtaaat tcctggaagg cgcagcgcgt catcaccatc accatcacca tcaccatcac   240
   caacctgatc tgattcctct gctgcgtact cagttcttta tcgttatggg tctggtggat   300
   gctatcccga tgatcgctgt aggtctgggt ctgtacgtga tgttcgctgt cgcgtatgaa   360
   ccgttccaga tcccgtcagg atcctcgaat tgccctgttc tggaaaaccg ggctgctcag   420
   ggcgatatta ctgcacccgg cggtgctcgc cgtttaacgg gtgatcagac tgccgctctg   480
   cgtgattctc ttagcgataa acctgcaaaa aatattattt tgctgattgg cgatgggatg   540
   ggggactcgg aaattactgc cgcacgtaat tatgccgaag gtgcgggcgg cttttttaaa   600
   ggtatagatg ccttaccgct taccgggcaa tacactcact atgcgctgaa taaaaaaacc   660
   ggcaaaccgg actacgtcac cgactcggct gcatcagcaa ccgcctggtc aaccggtgtc   720
   aaaacctata acggcgcgct gggcgtcgat attcacgaaa aagatcaccc aacgattctg   780
   gaaatggcaa aagccgcagg tctggcgacc ggtaacgttt ctaccgcaga gttgcaggat   840
   gccacgcccg ctgcgctggt ggcacatgtg acctcgcgca aatgctacgg tccgagcgcg   900
   accagtgaaa aatgtccggg taacgctctg gaaaaaggcg gaaaaggatc gattaccgaa   960
   cagctgctta acgctcgtgc cgacgttacg cttggcggcg gcgcaaaaac ctttgctgaa 1020
   acggcaaccg ctggtgaatg gcagggaaaa acgctgcgtg aacaggcaca ggcgcgtggt 1080
   tatcagttgg tgagcgatgc tgcctcactg aattcggtga cggaagcgaa tcagcaaaaa 1140
   cccctgcttg gcctgtttgc tgacggcaat atgccagtgc gctggctagg accgaaagca 1200
   acgtaccatg gcaatatcga taagcccgca gtcacctgta cgccaaatcc gcaacgtaat 1260
   gacagtgtac caaccctggc gcagatgacc gacaaagcca ttgaattgtt gagtaaaaat 1320
   gagaaaggct ttttcctgca agttgaaggt gcgtcaatcg ataaacagga tcatgctgcg 1380
   aatccttgtg ggcaaattgg cgagacggtc gatctcgatg aagccgtaca acgggcgctg 1440
   gaattcgcta aaaaggaggg taacacgctg gtcatagtca ccgctgatca cgcccacgcc 1500
   agccagattg ttgcgccgga taccaaagct ccgggcctca cccaggcgct aaataccaaa 1560
   gatggcgcag tgatggtgat gagttacggg aactccgaag aggattcaca agaacatacc 1620
   ggcagtcagt tgcgtattgc ggcgtatggc ccgcatgccg ccaatgttgt tggactgacc 1680
   gaccagaccg atctcttcta caccatgaaa gccgctctgg ggctgaaata aaaccgcgcc 1740
   cggcagtgaa ttttcgctgc cggcaattca gcttggctgt tttggcggat gagagaagat 1800
   tttcagcctg atacagatta aatcagaacg cagaagcggt ctgataaaac agaatttgcc 1860
   tggcggcagt agcgcggtgg tcccacctga ccccatgccg aactcagaag tgaaacgccg 1920
   tagcgccgat ggtagtgtgg ggtctcccca tgcgagagta gggaactgcc aggcatcaaa 1980
   taaaacgaaa ggctcagtcg aaagactggg cctttcgttt tatctgttgt ttgtcggtga 2040
   acgctctcct gagtaggaca aatccgccgg gagcggattt gaacgttgcg aagcaacggc 2100
   ccggagggtg gcgggcagga cgcccgccat aaactgccag gcatcaaatt aagcagaagg 2160
   ccatcctgac ggatggcctt tttgcgtttc tacaaactct tttgtttatt tttctaaata 2220
   cattcaaata tgtatccgct catgagacaa taaccctgat aaatgcttca ataatattga 2280
   aaaaggaaga gtatgagtat tcaacatttc cgtgtcgccc ttattccctt ttttgcggca 2340
   ttttgccttc ctgtttttgc tcacccagaa acgctggtga aagtaaaaga tgctgaagat 2400
   cagttgggtg cacgagtggg ttacatcgaa ctggatctca acagcggtaa gatccttgag 2460
   agttttcgcc ccgaagaacg ttttccaatg atgagcactt ttaaagttct gctatgtggc 2520
   gcggtattat cccgtgttga cgccgggcaa gagcaactcg gtcgccgcat acactattct 2580
   cagaatgact tggttgagta ctcaccagtc acagaaaagc atcttacgga tggcatgaca 2640
   gtaagagaat tatgcagtgc tgccataacc atgagtgata acactgcggc caacttactt 2700
   ctgacaacga tcggaggacc gaaggagcta accgcttttt tgcacaacat gggggatcat 2760
   gtaactcgcc ttgatcgttg ggaaccggag ctgaatgaag ccataccaaa cgacgagcgt 2820
   gacaccacga tgcctgcagc aatggcaaca acgttgcgca aactattaac tggcgaacta 2880
   cttactctag cttcccggca acaattaata gactggatgg aggcggataa agttgcagga 2940
   ccacttctgc gctcggccct tccggctggc tggtttattg ctgataaatc tggagccggt 3000
   gagcgtgggt ctcgcggtat cattgcagca ctggggccag atggtaagcc ctcccgtatc 3060
   gtagttatct acacgacggg gagtcaggca actatggatg aacgaaatag acagatcgct 3120
   gagataggtg cctcactgat taagcattgg taactgtcag accaagttta ctcatatata 3180
   ctttagattg atttaaaact tcatttttaa tttaaaagga tctaggtgaa gatccttttt 3240
   gataatctca tgaccaaaat cccttaacgt gagttttcgt tccactgagc gtcagacccc 3300
   gtagaaaaga tcaaaggatc ttcttgagat cctttttttc tgcgcgtaat ctgctgcttg 3360
   caaacaaaaa aaccaccgct accagcggtg gtttgtttgc cggatcaaga gctaccaact 3420
   ctttttccga aggtaactgg cttcagcaga gcgcagatac caaatactgt ccttctagtg 3480
   tagccgtagt taggccacca cttcaagaac tctgtagcac cgcctacata cctcgctctg 3540
   ctaatcctgt taccagtggc tgctgccagt ggcgataagt cgtgtcttac cgggttggac 3600
   tcaagacgat agttaccgga taaggcgcag cggtcgggct gaacgggggg ttcgtgcaca 3660
   cagcccagct tggagcgaac gacctacacc gaactgagat acctacagcg tgagcattga 3720
   gaaagcgcca cgcttcccga agggagaaag gcggacaggt atccggtaag cggcagggtc 3780
   ggaacaggag agcgcacgag ggagcttcca gggggaaacg cctggtatct ttatagtcct 3840
   gcgggtttcg ccacctctga cttgagcgtc gatttttgtg atgctcgtca ggggggcgga 3900
   gcctatggaa aaacgccagc aacgcggcct ttttacggtt cctggccttt tgctggcctt 3960
   ttgctcacat gttctttcct gcgttatccc ctgattctgt ggataaccgt attaccgcct 4020
   ttgagtgagc tgataccgct cgccgcagcc gaacgaccga gcgcagcgag tcagtgagcg 4080
   aggaagcgga agagcgcctg atgcggtatt ttctccttac gcatctgtgc ggtatttcac 4140
   accgcatacg aacgccagca agacgtagcc cagcgcgtcg gccagcttgc aattcgcgct 4200
   aacttacatt aattgcgttg cgctcactgc ccgctttcca gtcgggaaac ctgtcgtgcc 4260
   agctgcatta atgaatcggc caacgcgcgg ggagaggcgg tttgcgtatt gggcgccagg 4320
   gtggtttttc ttttcaccag tgagacgggc aacagctgat tgcccttcac cgcctggccc 4380
   tgagagagtt gcagcaagcg gtccacgctg gtttgcccca gcaggcgaaa atcctgtttg 4440
   ctggtggtta acggcgggat ataacatgag ctgtcttcgg tatcgtcgta tcccactacc 4500
   gagatatccg caccaacgcg cagcccggac tcggtaatgg cgcgcattgc gcccagcgcc 4560
   atctgatcgt tggcaaccag catcgcagtg ggaacgatgc cctcattcag catttgcatg 4620
   gtttgttgaa aaccggacat ggcactccag tcgccttccc gttccgctat cggctgaatt 4680
   tgattgcgag tgagatattt atgccagcca gccagacgca gacgcgccga gacagaactt 4740
   aatgggcccg ctaacagcgc gatttgctgg tgacccaatg cgaccagatg ctccacgccc 4800
   agtcgcgtac cgtcttcatg ggagaaaata atactgttga tgggtgtctg gtcagagaca 4860
   tcaagaaata acgccggaac attagtgcag gcagcttcca cagcaatggc atcctggtca 4920
   tccagcgcat agttaatgat cagcccactg acgcgttgcg cgagaagatt gtgcaccgcc 4980
   gctttacagg cttcgacgcc gcttcgttct accatcgaca ccaccacgct ggcacccagt 5040
   tgatcggcgc gagatttaat cgccgcgaca atttgcgacg gcgcgtgcag ggccagactg 5100
   gaggtggcaa cgccaatcag caacgactgt ttgcccgcca gttgttgtgc cacgcggttg 5160
   ggaatgtaat tcagctccgc catcgccgct tccacttttt cccgcgtttt cgcagaaacg 5220
   tggctggcct ggttcaccac gcgggaaacg gtctgataag agacaccggc atactctgcg 5280
   acatcgtata acgttactgg tttcacattc accaccctga attgactctc ttccgggcgc 5340
   tatcatgcca taccgcgaaa ggttttgcac cattcgatgg tgtcaacgta aatgccgctt 5400
   cgccttcgcg cgcgaattgc aagctgatcc gggcttatcg actgcacggt gcaccaatgc 5460
   ttctggcgtc aggcagccat cggaagctgt ggtatggctg tgcaggtcgt aaatcactgc 5520
   ataattcgtg tcgctcaagg cgcactcccg ttctggataa tgttttttgc gccgacatca 5580
   taacggttct ggcaaatatt ctgaaatgag ctgttgacaa ttaatcatcg gctcgtataa 5640
   tgtgtggaat tgtgagcgga taacaatttc acacaggaaa cagaatt 5687
<210> 13
   <211> 8
   <212> PRT
   <213> artificial
<220>
   <223> completely synthetic
<400> 13
   Asp Tyr Lys Asp Asp Asp Asp Lys
   1 5
<210> 14
   <211> 5
   <212> PRT
   <213> artificial
<220>
   <223> completely synthetic
<400> 14
   Asp Asp Asp Asp Lys
   1 5
<210> 15
   <211> 93
   <212> DNA
   <213> artificial
<220>
   <223> synthetic
<220>
   <221> misc_feature
   <222> (82)..(93)
   <223> nt 82-93 correspond to nt 4-15 of Staphylococcus aureus 3A
<400> 15
   tccccgtctg cacaggcggc atatgaaccg ttccagatcc cgtcaggatc ggactacaaa 60
   gatgacgatg acaaaggatc cgaaagttat ttg 93
<210> 16
   <211> 31
   <212> PRT
   <213> artificial
<220>
   <223> synthetic
<220>
   <221> MISC_FEATURE
   <222> (28)..(31)
   <223> residues 28-31 correspond to 2-6 of Staphylococcus aureus 3A
<400> 16
   Ser Pro Ser Ala Gln Ala Ala Tyr Glu Pro Phe Gln Ile Pro Ser Gly
   1 5 10 15
   Ser Asp Tyr Lys Asp Asp Asp Asp Lys Gly Ser Glu Ser Tyr Leu
   20 25 30
<210> 17
   <211> 25
   <212> PRT
   <213> artificial
<220>
   <223> synthetic
<220>
   <221> MISC_FEATURE
   <222> (1)..(25)
   <223> amino acid residues 1-25 correspond to residues 7-31 of seq id no. 16
<220>
   <221> MISC_FEATURE
   <222> (22)..(25)
   <223> amino acid residues 22-25 correspond to residues 2-6 of Staphylococcus aureus 3A
<400> 17
   Ala Tyr Glu Pro Phe Gln Ile Pro Ser Gly Ser Asp Tyr Lys Asp Asp
   1 5 10 15
   Asp Asp Lys Gly Ser Glu Ser Tyr Leu
   20 25
<210> 18
   <211> 5
   <212> PRT
   <213> artificial
<220>
   <223> synthetic
<220>
   <221> MISC_FEATURE
   <222> (2)..(6)
   <223> residues 2-6 correspond to residues 2-6 of Bacillus fusiformis 1226
<400> 18
   Ala Phe Glu Thr Glu
   1 5
<210> 19
   <211> 5
   <212> PRT
   <213> artificial
<220>
   <223> synthetic
<220>
   <221> MISC_FEATURE
   <222> (2)..(6)
   <223> residues 2-6 correspond to residues 2-6 of Bacillus fusiformis 1226
<400> 19
   Met Glu Ser Tyr Leu
   1 5
<210> 20
   <211> 5
   <212> PRT
   <213> artificial
<220>
   <223> synthetic
<220>
   <221> MISC_FEATURE
   <222> (2)..(6)
   <223> residues 2-6 correspond to residues 2-6 of Bacillus fusiformis 1226
<400> 20
   Val Phe Glu Thr Glu
   1 5
<210> 21
   <211> 5987
   <212> DNA
   <213> artificial
<220>
   <223> completely synthetic
<400> 21
   aagcttgcat gcctgcaggt cgacggatcg atccccgtgc cttcgtagtg gcattacgta     60
   ttttacccgt ttaatggaaa cttcctcatg aaaaagtctt tagtcctcaa agcctctgta    120
   gccgttgcta ccctcgttcc gatgctgtct ttcgctgctg agggtgacga tcccgcaaaa    180
   gcggccttta actccctgca agcctcagcg accgaatata tcggttatgc gtgggcgatg    240
   gttgttgtca ttgtcggcgc aactatcggt atcaagctgt ttaagaaatt cacctcgaaa    300
   gcaaggggaa tttcgcgtca ggcagcggcg caggcggctg ccacgacaaa tcctggtgta    360
   tccgcttggc aggtcaacac agcttatact gcgggacaat tggtcacata taacggcaag    420
   acgtataaat gtttgcagcc ccacacctcc ttggcaggat gggaaccatc caacgttcct    480
   gccttgtggc agcttcaagg tggccatggg gactcactgg ataaagcaac gttgaaaaag    540
   gttgcgccga agcctggctg gctggaaacc ggtgcttctg tttttccggt actggctatc    600
   gtattgattg tgcgttcgtt tatttatgaa ccgttccaga tcccgtcagg atcggactac    660
   aaagatgacg atgacaaagg atcctcgaat tgccctgttc tggaaaaccg ggctgctcag    720
   ggcgatatta ctgcacccgg cggtgctcgc cgtttaacgg gtgatcagac tgccgctctg    780
   cgtgattctc ttagcgataa acctgcaaaa aatattattt tgctgattgg cgatgggatg    840
   ggggactcgg aaattactgc cgcacgtaat tatgccgaag gtgcgggcgg cttttttaaa    900
   ggtatagatg ccttaccgct taccgggcaa tacactcact atgcgctgaa taaaaaaacc    960
   ggcaaaccgg actacgtcac cgactcggct gcatcagcaa ccgcctggtc aaccggtgtc   1020
   aaaacctata acggcgcgct gggcgtcgat attcacgaaa aagatcaccc aacgattctg   1080
   gaaatggcaa aagccgcagg tctggcgacc ggtaacgttt ctaccgcaga gttgcaggat   1140
   gccacgcccg ctgcgctggt ggcacatgtg acctcgcgca aatgctacgg tccgagcgcg   1200
   accagtgaaa aatgtccggg taacgctctg gaaaaaggcg gaaaaggatc gattaccgaa   1260
   cagctgctta acgctcgtgc cgacgttacg cttggcggcg gcgcaaaaac ctttgctgaa   1320
   acggcaaccg ctggtgaatg gcagggaaaa acgctgcgtg aacaggcaca ggcgcgtggt   1380
   tatcagttgg tgagcgatgc tgcctcactg aattcggtga cggaagcgaa tcagcaaaaa   1440
   cccctgcttg gcctgtttgc tgacggcaat atgccagtgc gctggctagg accgaaagca   1500
   acgtaccatg gcaatatcga taagcccgca gtcacctgta cgccaaatcc gcaacgtaat   1560
   gacagtgtac caaccctggc gcagatgacc gacaaagcca ttgaattgtt gagtaaaaat   1620
   gagaaaggct ttttcctgca agttgaaggt gcgtcaatcg ataaacagga tcatgctgcg   1680
   aatccttgtg ggcaaattgg cgagacggtc gatctcgatg aagccgtaca acgggcgctg   1740
   gaattcgcta aaaaggaggg taacacgctg gtcatagtca ccgctgatca cgcccacgcc   1800
   agccagattg ttgcgccgga taccaaagct ccgggcctca cccaggcgct aaataccaaa   1860
   gatggcgcag tgatggtgat gagttacggg aactccgaag aggattcaca agaacatacc   1920
   ggcagtcagt tgcgtattgc ggcgtatggc ccgcatgccg ccaatgttgt tggactgacc   1980
   gaccagaccg atctcttcta caccatgaaa gccgctctgg ggctgaaata aaaccgcgcc   2040
   cggcagtgaa ttttcgctgc cggcaattca gcttggctgt tttggcggat gagagaagat   2100
   tttcagcctg atacagatta aatcagaacg cagaagcggt ctgataaaac agaatttgcc   2160
   tggcggcagt agcgcggtgg tcccacctga ccccatgccg aactcagaag tgaaacgccg   2220
   tagcgccgat ggtagtgtgg ggtctcccca tgcgagagta gggaactgcc aggcatcaaa 2280
   taaaacgaaa ggctcagtcg aaagactggg cctttcgttt tatctgttgt ttgtcggtga 2340
   acgctctcct gagtaggaca aatccgccgg gagcggattt gaacgttgcg aagcaacggc 2400
   ccggagggtg gcgggcagga cgcccgccat aaactgccag gcatcaaatt aagcagaagg 2460
   ccatcctgac ggatggcctt tttgcgtttc tacaaactct tttgtttatt tttctaaata 2520
   cattcaaata tgtatccgct catgagacaa taaccctgat aaatgcttca ataatattga 2580
   aaaaggaaga gtatgagtat tcaacatttc cgtgtcgccc ttattccctt ttttgcggca 2640
   ttttgccttc ctgtttttgc tcacccagaa acgctggtga aagtaaaaga tgctgaagat 2700
   cagttgggtg cacgagtggg ttacatcgaa ctggatctca acagcggtaa gatccttgag 2760
   agttttcgcc ccgaagaacg ttttccaatg atgagcactt ttaaagttct gctatgtggc 2820
   gcggtattat cccgtgttga cgccgggcaa gagcaactcg gtcgccgcat acactattct 2880
   cagaatgact tggttgagta ctcaccagtc acagaaaagc atcttacgga tggcatgaca 2940
   gtaagagaat tatgcagtgc tgccataacc atgagtgata acactgcggc caacttactt 3000
   ctgacaacga tcggaggacc gaaggagcta accgcttttt tgcacaacat gggggatcat 3060
   gtaactcgcc ttgatcgttg ggaaccggag ctgaatgaag ccataccaaa cgacgagcgt 3120
   gacaccacga tgcctgcagc aatggcaaca acgttgcgca aactattaac tggcgaacta 3180
   cttactctag cttcccggca acaattaata gactggatgg aggcggataa agttgcagga 3240
   ccacttctgc gctcggccct tccggctggc tggtttattg ctgataaatc tggagccggt 3300
   gagcgtgggt ctcgcggtat cattgcagca ctggggccag atggtaagcc ctcccgtatc 3360
   gtagttatct acacgacggg gagtcaggca actatggatg aacgaaatag acagatcgct 3420
   gagataggtg cctcactgat taagcattgg taactgtcag accaagttta ctcatatata 3480
   ctttagattg atttaaaact tcatttttaa tttaaaagga tctaggtgaa gatccttttt 3540
   gataatctca tgaccaaaat cccttaacgt gagttttcgt tccactgagc gtcagacccc 3600
   gtagaaaaga tcaaaggatc ttcttgagat cctttttttc tgcgcgtaat ctgctgcttg 3660
   caaacaaaaa aaccaccgct accagcggtg gtttgtttgc cggatcaaga gctaccaact 3720
   ctttttccga aggtaactgg cttcagcaga gcgcagatac caaatactgt ccttctagtg 3780
   tagccgtagt taggccacca cttcaagaac tctgtagcac cgcctacata cctcgctctg 3840
   ctaatcctgt taccagtggc tgctgccagt ggcgataagt cgtgtcttac cgggttggac 3900
   tcaagacgat agttaccgga taaggcgcag cggtcgggct gaacgggggg ttcgtgcaca 3960
   cagcccagct tggagcgaac gacctacacc gaactgagat acctacagcg tgagcattga 4020
   gaaagcgcca cgcttcccga agggagaaag gcggacaggt atccggtaag cggcagggtc 4080
   ggaacaggag agcgcacgag ggagcttcca gggggaaacg cctggtatct ttatagtcct 4140
   gcgggtttcg ccacctctga cttgagcgtc gatttttgtg atgctcgtca cgggggcgga 4200
   gcctatggaa aaacgccagc aacgcggcct ttttacggtt cctggccttt tgctggcctt 4260
   ttgctcacat gttctttcct gcgttatccc ctgattctgt ggataaccgt attaccgcct 4320
   ttgagtgagc tgataccgct cgccgcagcc gaacgaccga gcgcagcgag tcagtgagcg 4380
   aggaagcgga agagcgcctg atgcggtatt ttctccttac gcatctgtgc ggtatttcac 4440
   accgcatacg aacgccagca agacgtagcc cagcgcgtcg gccagcttgc aattcgcgct 4500
   aacttacatt aattgcgttg cgctcactgc ccgctttcca gtcgggaaac ctgtcgtgcc 4560
   agctgcatta atgaatcggc caacgcgcgg ggagaggcgg tttgcgtatt gggcgccagg 4620
   gtggtttttc ttttcaccag tgagacgggc aacagctgat tgcccttcac cgcctggccc 4680
   tgagagagtt gcagcaagcg gtccacgctg gtttgcccca gcaggcgaaa atcctgtttg 4740
   ctggtggtta acggcgggat ataacatgag ctgtcttcgg tatcgtcgta tcccactacc 4800
   gagatatccg caccaacgcg cagcccggac tcggtaatgg cgcgcattgc gcccagcgcc 4860
   atctgatcgt tggcaaccag catcgcagtg ggaacgatgc cctcattcag catttgcatg 4920
   gtttgttgaa aaccggacat ggcactccag tcgccttccc gttccgctat cggctgaatt 4980
   tgattgcgag tgagatattt atgccagcca gccagacgca gacgcgccga gacagaactt 5040
   aatgggcccg ctaacagcgc gatttgctgg tgacccaatg cgaccagatg ctccacgccc 5100
   agtcgcgtac cgtcttcatg ggagaaaata atactgttga tgggtgtctg gtcagagaca 5160
   tcaagaaata acgccggaac attagtgcag gcagcttcca cagcaatggc atcctggtca 5220
   tccagcgcat agttaatgat cagcccactg acgcgttgcg cgagaagatt gtgcaccgcc 5280
   gctttacagg cttcgacgcc gcttcgttct accatcgaca ccaccacgct ggcacccagt 5340
   tgatcggcgc gagatttaat cgccgcgaca atttgcgacg gcgcgtgcag ggccagactg 5400
   gaggtggcaa cgccaatcag caacgactgt ttgcccgcca gttgttgtgc cacgcggttg 5460
   ggaatgtaat tcagctccgc catcgccgct tccacttttt cccgcgtttt cgcagaaacg 5520
   tggctggcct ggttcaccac gcgggaaacg gtctgataag agacaccggc atactctgcg 5580
   acatcgtata acgttactgg tttcacattc accaccctga attgactctc ttccgggcgc 5640
   tatcatgcca taccgcgaaa ggttttgcac cattcgatgg tgtcaacgta aatgccgctt 5700
   cgccttcgcg cgcgaattgc aagctgatcc gggcttatcg actgcacggt gcaccaatgc 5760
   ttctggcgtc aggcagccat cggaagctgt ggtatggctg tgcaggtcgt aaatcactgc 5820
   ataattcgtg tcgctcaagg cgcactcccg ttctggataa tgttttttgc gccgacatca 5880
   taacggttct ggcaaatatt ctgaaatgag ctgttgacaa ttaatcatcg gctcgtataa 5940
   tgtgtggaat tgtgagcgga taacaatttc acacaggaaa cagaatt 5987
<210> 22
   <211> 5987
   <212> DNA
   <213> artificial
<220>
   <223> completely synthetic
<400> 22
   aagcttgcat gcctgcaggt cgacggatcg atccccgtgc cttcgtagtg gcattacgta 60
   ttttacccgt ttaatggaaa cttcctcatg aaaaagtctt tagtcctcaa agcctctgta 120
   gccgttgcta ccctcgttcc gatgctgtct ttcgctgctg agggtgacga tcccgcaaaa 180
   gcggccttta actccctgca agcctcagcg accgaatata tcggttatgc gtgggcgatg 240
   gttgttgtca ttgtcggcgc aactatcggt atcaagctgt ttaagaaatt cacctcgaaa 300
   gcaaggggaa tttcgcgtca ggcagcggcg caggcggctg ccacgacaaa tcctggtgta 360
   tccgcttggc aggtcaacac agcttatact gcgggacaat tggtcacata taacggcaag 420
   acgtataaat gtttgcagcc ccacacctcc ttggcaggat gggaaccatc caacgttcct 480
   gccttgtggc agcttcaagg tggccatggg gactcactgg ataaagcaac gttgaaaaag 540
   gttgcgccga agcctggctg gctggaaacc ggtgcttctg tttttccggt actggctatc 600
   gtattgattg tgcgttcgtt tatttatgaa ccgttccaga tcccgtcagg atcggactac 660
   aaagatgacg atgacaaagg atcctcgaat tgccctgttc tggaaaaccg ggctgctcag 720
   ggcgatatta ctgcacccgg cggtgctcgc cgtttaacgg gtgatcagac tgccgctctg 780
   cgtgattctc ttagcgataa acctgcaaaa aatattattt tgctgattgg cgatgggatg 840
   ggggactcgg aaattactgc cgcacgtaat tatgccgaag gtgcgggcgg cttttttaaa 900
   ggtatagatg ccttaccgct taccgggcaa tacactcact atgcgctgaa taaaaaaacc 960
   ggcaaaccgg actacgtcac cgactcggct gcatcagcaa ccgcctggtc aaccggtgtc 1020
   aaaacctata acggcgcgct gggcgtcgat attcacgaaa aagatcaccc aacgattctg 1080
   gaaatggcaa aagccgcagg tctggcgacc ggtaacgttt ctaccgcaga gttgcaggat 1140
   gccacgcccg ctgcgctggt ggcacatgtg acctcgcgca aatgctacgg tccgagcgcg 1200
   accagtgaaa aatgtccggg taacgctctg gaaaaaggcg gaaaaggatc gattaccgaa 1260
   cagctgctta acgctcgtgc cgacgttacg cttggcggcg gcgcaaaaac ctttgctgaa 1320
   acggcaaccg ctggtgaatg gcagggaaaa acgctgcgtg aacaggcaca ggcgcgtggt 1380
   tatcagttgg tgagcgatgc tgcctcactg aattcggtga cggaagcgaa tcagcaaaaa 1440
   cccctgcttg gcctgtttgc tgacggcaat atgccagtgc gctggctagg accgaaagca 1500
   acgtaccatg gcaatatcga taagcccgca gtcacctgta cgccaaatcc gcaacgtaat 1560
   gacagtgtac caaccctggc gcagatgacc gacaaagcca ttgaattgtt gagtaaaaat 1620
   gagaaaggct ttttcctgca agttgaaggt gcgtcaatcg ataaacagga tcatgctgcg 1680
   aatccttgtg ggcaaattgg cgagacggtc gatctcgatg aagccgtaca acgggcgctg 1740
   gaattcgcta aaaaggaggg taacacgctg gtcatagtca ccgctgatca cgcccacgcc 1800
   agccagattg ttgcgccgga taccaaagct ccgggcctca cccaggcgct aaataccaaa 1860
   gatggcgcag tgatggtgat gagttacggg aactccgaag aggattcaca agaacatacc 1920
   ggcagtcagt tgcgtattgc ggcgtatggc ccgcatgccg ccaatgttgt tggactgacc 1980
   gaccagaccg atctcttcta caccatgaaa gccgctctgg ggctgaaata aaaccgcgcc 2040
   cggcagtgaa ttttcgctgc cggcaattca gcttggctgt tttggcggat gagagaagat 2100
   tttcagcctg atacagatta aatcagaacg cagaagcggt ctgataaaac agaatttgcc 2160
   tggcggcagt agcgcggtgg tcccacctga ccccatgccg aactcagaag tgaaacgccg 2220
   tagcgccgat ggtagtgtgg ggtctcccca tgcgagagta gggaactgcc aggcatcaaa 2280
   taaaacgaaa ggctcagtcg aaagactggg cctttcgttt tatctgttgt ttgtcggtga 2340
   acgctctcct gagtaggaca aatccgccgg gagcggattt gaacgttgcg aagcaacggc 2400
   ccggagggtg gcgggcagga cgcccgccat aaactgccag gcatcaaatt aagcagaagg 2460
   ccatcctgac ggatggcctt tttgcgtttc tacaaactct tttgtttatt tttctaaata 2520
   cattcaaata tgtatccgct catgagacaa taaccctgat aaatgcttca ataatattga 2580
   aaaaggaaga gtatgagtat tcaacatttc cgtgtcgccc ttattccctt ttttgcggca 2640
   ttttgccttc ctgtttttgc tcacccagaa acgctggtga aagtaaaaga tgctgaagat 2700
   cagttgggtg cacgagtggg ttacatcgaa ctggatctca acagcggtaa gatccttgag 2760
   agttttcgcc ccgaagaacg ttttccaatg atgagcactt ttaaagttct gctatgtggc 2820
   gcggtattat cccgtgttga cgccgggcaa gagcaactcg gtcgccgcat acactattct 2880
   cagaatgact tggttgagta ctcaccagtc acagaaaagc atcttacgga tggcatgaca 2940
   gtaagagaat tatgcagtgc tgccataacc atgagtgata acactgcggc caacttactt 3000
   ctgacaacga tcggaggacc gaaggagcta accgcttttt tgcacaacat gggggatcat 3060
   gtaactcgcc ttgatcgttg ggaaccggag ctgaatgaag ccataccaaa cgacgagcgt 3120
   gacaccacga tgcctgcagc aatggcaaca acgttgcgca aactattaac tggcgaacta 3180
   cttactctag cttcccggca acaattaata gactggatgg aggcggataa agttgcagga 3240
   ccacttctgc gctcggccct tccggctggc tggtttattg ctgataaatc tggagccggt 3300
   gagcgtgggt ctcgcggtat cattgcagca ctggggccag atggtaagcc ctcccgtatc 3360
   gtagttatct acacgacggg gagtcaggca actatggatg aacgaaatag acagatcgct 3420
   gagataggtg cctcactgat taagcattgg taactgtcag accaagttta ctcatatata 3480
   ctttagattg atttaaaact tcatttttaa tttaaaagga tctaggtgaa gatccttttt 3540
   gataatctca tgaccaaaat cccttaacgt gagttttcgt tccactgagc gtcagacccc 3600
   gtagaaaaga tcaaaggatc ttcttgagat cctttttttc tgcgcgtaat ctgctgcttg 3660
   caaacaaaaa aaccaccgct accagcggtg gtttgtttgc cggatcaaga gctaccaact 3720
   ctttttccga aggtaactgg cttcagcaga gcgcagatac caaatactgt ccttctagtg 3780
   tagccgtagt taggccacca cttcaagaac tctgtagcac cgcctacata cctcgctctg 3840
   ctaatcctgt taccagtggc tgctgccagt ggcgataagt cgtgtcttac cgggttggac 3900
   tcaagacgat agttaccgga taaggcgcag cggtcgggct gaacgggggg ttcgtgcaca 3960
   cagcccagct tggagcgaac gacctacacc gaactgagat acctacagcg tgagcattga 4020
   gaaagcgcca cgcttcccga agggagaaag gcggacaggt atccggtaag cggcagggtc 4080
   ggaacaggag agcgcacgag ggagcttcca gggggaaacg cctggtatct ttatagtcct 4140
   gcgggtttcg ccacctctga cttgagcgtc gatttttgtg atgctcgtca ggggggcgga 4200
   gcctatggaa aaacgccagc aacgcggcct ttttacggtt cctggccttt tgctggcctt 4260
   ttgctcacat gttctttcct gcgttatccc ctgattctgt ggataaccgt attaccgcct 4320
   ttgagtgagc tgataccgct cgccgcagcc gaacgaccga gcgcagcgag tcagtgagcg 4380
   aggaagcgga agagcgcctg atgcggtatt ttctccttac gcatctgtgc ggtatttcac 4440
   accgcatacg aacgccagca agacgtagcc cagcgcgtcg gccagcttgc aattcgcgct 4500
   aacttacatt aattgcgttg cgctcactgc ccgctttcca gtcgggaaac ctgtcgtgcc 4560
   agctgcatta atgaatcggc caacgcgcgg ggagaggcgg tttgcgtatt gggcgccagg 4620
   gtggtttttc ttttcaccag tgagacgggc aacagctgat tgcccttcac cgcctggccc 4680
   tgagagagtt gcagcaagcg gtccacgctg gtttgcccca gcaggcgaaa atcctgtttg 4740
   ctggtggtta acggcgggat ataacatgag ctgtcttcgg tatcgtcgta tcccactacc 4800
   gagatatccg caccaacgcg cagcccggac tcggtaatgg cgcgcattgc gcccagcgcc 4860
   atctgatcgt tggcaaccag catcgcagtg ggaacgatgc cctcattcag catttgcatg 4920
   gtttgttgaa aaccggacat ggcactccag tcgccttccc gttccgctat cggctgaatt 4980
   tgattgcgag tgagatattt atgccagcca gccagacgca gacgcgccga gacagaactt 5040
   aatgggcccg ctaacagcgc gatttgctgg tgacccaatg cgaccagatg ctccacgccc 5100
   agtcgcgtac cgtcttcatg ggagaaaata atactgttga tgggtgtctg gtcagagaca 5160
   tcaagaaata acgccggaac attagtgcag gcagcttcca cagcaatggc atcctggtca 5220
   tccagcgcat agttaatgat cagcccactg acgcgttgcg cgagaagatt gtgcaccgcc 5280
   gctttacagg cttcgacgcc gcttcgttct accatcgaca ccaccacgct ggcacccagt 5340
   tgatcggcgc gagatttaat cgccgcgaca atttgcgacg gcgcgtgcag ggccagactg 5400
   gaggtggcaa cgccaatcag caacgactgt ttgcccgcca gttgttgtgc cacgcggttg 5460
   ggaatgtaat tcagctccgc catcgccgct tccacttttt cccgcgtttt cgcagaaacg 5520
   tggctggcct ggttcaccac gcgggaaacg gtctgataag agacaccggc atactctgcg 5580
   acatcgtata acgttactgg tttcacattc accaccctga attgactctc ttccgggcgc 5640
   tatcatgcca taccgcgaaa ggttttgcac cattcgatgg tgtcaacgta aatgccgctt 5700
   cgccttcgcg cgcgaattgc aagctgatcc gggcttatcg actgcacggt gcaccaatgc 5760
   ttctggcgtc aggcagccat cggaagctgt ggtatggctg tgcaggtcgt aaatcactgc 5820
   ataattcgtg tcgctcaagg cgcactcccg ttctggataa tgttttttgc gccgacatca 5880
   taacggttct ggcaaatatt ctgaaatgag ctgttgacaa ttaatcatcg gctcgtataa 5940
   tgtgtggaat tgtgagcgga taacaatttc acacaggaaa cagaatt 5987
<210> 23
   <211> 5917
   <212> DNA
   <213> artificial
<220>
   <223> synthetic
<220>
   <221> misc_feature
   <222> (631)..(1986)
   <223> BfuCI (Bacillus fusiformis 1226) open reading frame lacking Met # 1 codon
<400> 23
   aagcttgcat gcctgcaggt cgacggatcg atccccgtgc cttcgtagtg gcattacgta     60
   ttttacccgt ttaatggaaa cttcctcatg aaaaagtctt tagtcctcaa agcctctgta    120
   gccgttgcta ccctcgttcc gatgctgtct ttcgctgctg agggtgacga tcccgcaaaa    180
   gcggccttta actccctgca agcctcagcg accgaatata tcggttatgc gtgggcgatg    240
   gttgttgtca ttgtcggcgc aactatcggt atcaagctgt ttaagaaatt cacctcgaaa    300
   gcaaggggaa tttcgcgtca ggcagcggcg caggcggctg ccacgacaaa tcctggtgta    360
   tccgcttggc aggtcaacac agcttatact gcgggacaat tggtcacata taacggcaag    420
   acgtataaat gtttgcagcc ccacacctcc ttggcaggat gggaaccatc caacgttcct    480
   gccttgtggc agcttcaagg tggccatggg gactcactgg ataaagcaac gttgaaaaag    540
   gttgcgccga agcctggctg gctggaaacc ggtgcttctg tttttccggt actggctatc    600
   gtattgattg tgtccccgtc agcacaggct gtttttgaaa ctgaagaggc acttttatat    660
   aaggcacaag aggcgcgtgg taaaacattc agtgagattg atcagcatgg tagattaaat    720
   agtaaacgat ctacaggtgc gctaggtcag attgttgaag aaagtttttt tggatatgaa    780
   gtgaatagca atgctgaggc tgatttttcg aacttaggta tagagctaaa ggtgacgcct    840
   tttaagcaga ataaggataa aagtttgtct gctaaggaac gactagtact taacattata    900
   aactacatga cagaagcgga taaaaatttt tatgattcga gcttttggaa gaaaaatcag    960
   aagttacttt tgatgtttta tgagtggaaa aaagaattaa atcgaggaga ttataaaatt   1020
   attgaaacac ttcttttcga atatcctgag aaggatttag ctgttataaa atctgactgg   1080
   gcattgattc aagggaaaat tcgagctggg ttggcgcatg agttatcaga gggggatacg   1140
   caatatttag gggcctgtac  gaagggagca aacaagaatt cattacgaga acaacctttt   1200
   tctgatgtac ctgcaatgca acgagctttc tctttgaagc aatcttatat gaccgcatta   1260
   gtacggcagt atatatcaaa agaaaagtta gtatatttct caagtcttga ggaactacag   1320
   gaaaaaacga tagaacaatt attaaatgat cgtttcgagc cttttatggg aatgacaatg   1380
   gaagaaatgg cagaaaagct aaatattcaa attaatcctg ggaatcgttc tgctgtacca   1440
   aatttaatta gtgcgttatt aggggttaaa ggaacaaaac tagataaaat agctgaattt   1500
   gcaaaagcaa atatacaatt taaaacagtg cgtttacaac aaagcggaag acctaaagaa   1560
   agtatgtcct ttaaaaatat tgattttaat gaaattatta atgaggaatg ggaagatagt   1620
   tatattagaa actatttttt agaaacacaa atattatttg ttgtctttca atttgataat 1680
   aacgatttat tacgttttaa aggtataaaa ctttggcata tggctatgaa aactattaat 1740
   aatgagctat ttaatttttg gaatgaaata cgcagagtat taactgaggg ggtaatttta 1800
   acccaatcta aaaagggaat agaaaataac ttcccaaaat caaattttaa tggtgtatta 1860
   catgttcgac caaaaggggc tgatggtagt gataaaatca aactacctga tggtcaatgg 1920
   attacaaaac agtgttattg gttgaatgca agttatgttg cagaaatagt aaaggaagtc 1980
   aaataaggta cctgaattca gcttggctgt tttggcggat gagagaagat tttcagcctg 2040
   atacagatta aatcagaacg cagaagcggt ctgataaaac agaatttgcc tggcggcagt 2100
   agcgcggtgg tcccacctga ccccatgccg aactcagaag tgaaacgccg tagcgccgat 2160
   ggtagtgtgg ggtctcccca tgcgagagta gggaactgcc aggcatcaaa taaaacgaaa 2220
   ggctcagtcg aaagactggg cctttcgttt tatctgttgt ttgtcggtga acgctctcct 2280
   gagtaggaca aatccgccgg gagcggattt gaacgttgcg aagcaacggc ccggagggtg 2340
   gcgggcagga cgcccgccat aaactgccag gcatcaaatt aagcagaagg ccatcctgac 2400
   ggatggcctt tttgcgtttc tacaaactct tttgtttatt tttctaaata cattcaaata 2460
   tgtatccgct catgagacaa taaccctgat aaatgcttca ataatattga aaaaggaaga 2520
   gtatgagtat tcaacatttc cgtgtcgccc ttattccctt ttttgcggca ttttgccttc 2580
   ctgtttttgc tcacccagaa acgctggtga aagtaaaaga tgctgaagat cagttgggtg 2640
   cacgagtggg ttacatcgaa ctggatctca acagcggtaa gatccttgag agttttcgcc 2700
   ccgaagaacg ttttccaatg atgagcactt ttaaagttct gctatgtggc gcggtattat 2760
   cccgtgttga cgccgggcaa gagcaactcg gtcgccgcat acactattct cagaatgact 2820
   tggttgagta ctcaccagtc acagaaaagc atcttacgga tggcatgaca gtaagagaat 2880
   tatgcagtgc tgccataacc atgagtgata acactgcggc caacttactt ctgacaacga 2940
   tcggaggacc gaaggagcta accgcttttt tgcacaacat gggggatcat gtaactcgcc 3000
   ttgatcgttg ggaaccggag ctgaatgaag ccataccaaa cgacgagcgt gacaccacga 3060
   tgcctgcagc aatggcaaca acgttgcgca aactattaac tggcgaacta cttactctag 3120
   cttcccggca acaattaata gactggatgg aggcggataa agttgcagga ccacttctgc 3180
   gctcggccct tccggctggc tggtttattg ctgataaatc tggagccggt gagcgtgggt 3240
   ctcgcggtat cattgcagca ctggggccag atggtaagcc ctcccgtatc gtagttatct 3300
   acacgacggg gagtcaggca actatggatg aacgaaatag acagatcgct gagataggtg 3360
   cctcactgat taagcattgg taactgtcag accaagttta ctcatatata ctttagattg 3420
   atttaaaact tcatttttaa tttaaaagga tctaggtgaa gatccttttt gataatctca 3480
   tgaccaaaat cccttaacgt gagttttcgt tccactgagc gtcagacccc gtagaaaaga 3540
   tcaaaggatc ttcttgagat cctttttttc tgcgcgtaat ctgctgcttg caaacaaaaa 3600
   aaccaccgct accagcggtg gtttgtttgc cggatcaaga gctaccaact ctttttccga 3660
   aggtaactgg cttcagcaga gcgcagatac caaatactgt ccttctagtg tagccgtagt 3720
   taggccacca cttcaagaac tctgtagcac cgcctacata cctcgctctg ctaatcctgt 3780
   taccagtggc tgctgccagt ggcgataagt cgtgtcttac cgggttggac tcaagacgat 3840
   agttaccgga taaggcgcag cggtcgggct gaacgggggg ttcgtgcaca cagcccagct 3900
   tggagcgaac gacctacacc gaactgagat acctacagcg tgagcattga gaaagcgcca 3960
   cgcttcccga agggagaaag gcggacaggt atccggtaag cggcagggtc ggaacaggag 4020
   agcgcacgag ggagcttcca gggggaaacg cctggtatct ttatagtcct gcgggtttcg 4080
   ccacctctga cttgagcgtc gatttttgtg atgctcgtca ggggggcgga gcctatggaa 4140
   aaacgccagc aacgcggcct ttttacggtt cctggccttt tgctggcctt ttgctcacat 4200
   gttctttcct gcgttatccc ctgattctgt ggataaccgt attaccgcct ttgagtgagc 4260
   tgataccgct cgccgcagcc gaacgaccga gcgcagcgag tcagtgagcg aggaagcgga 4320
   agagcgcctg atgcggtatt ttctccttac gcatctgtgc ggtatttcac accgcatacg 4380
   aacgccagca agacgtagcc cagcgcgtcg gccagcttgc aattcgcgct aacttacatt 4440
   aattgcgttg cgctcactgc ccgctttcca gtcgggaaac ctgtcgtgcc agctgcatta 4500
   atgaatcggc caacgcgcgg ggagaggcgg tttgcgtatt gggcgccagg gtggtttttc 4560
   ttttcaccag tgagacgggc aacagctgat tgcccttcac cgcctggccc tgagagagtt 4620
   gcagcaagcg gtccacgctg gtttgcccca gcaggcgaaa atcctgtttg ctggtggtta 4680
   acggcgggat ataacatgag ctgtcttcgg tatcgtcgta tcccactacc gagatatccg 4740
   caccaacgcg cagcccggac tcggtaatgg cgcgcattgc gcccagcgcc atctgatcgt 4800
   tggcaaccag catcgcagtg ggaacgatgc cctcattcag catttgcatg gtttgttgaa 4860
   aaccggacat ggcactccag tcgccttccc gttccgctat cggctgaatt tgattgcgag 4920
   tgagatattt atgccagcca gccagacgca gacgcgccga gacagaactt aatgggcccg 4980
   ctaacagcgc gatttgctgg tgacccaatg cgaccagatg ctccacgccc agtcgcgtac 5040
   cgtcttcatg ggagaaaata atactgttga tgggtgtctg gtcagagaca tcaagaaata 5100
   acgccggaac attagtgcag gcagcttcca cagcaatggc atcctggtca tccagcgcat 5160
   agttaatgat cagcccactg acgcgttgcg cgagaagatt gtgcaccgcc gctttacagg 5220
   cttcgacgcc gcttcgttct accatcgaca ccaccacgct ggcacccagt tgatcggcgc 5280
   gagatttaat cgccgcgaca atttgcgacg gcgcgtgcag ggccagactg gaggtggcaa 5340
   cgccaatcag caacgactgt ttgcccgcca gttgttgtgc cacgcggttg ggaatgtaat 5400
   tcagctccgc catcgccgct tccacttttt cccgcgtttt cgcagaaacg tggctggcct 5460
   ggttcaccac gcgggaaacg gtctgataag agacaccggc atactctgcg acatcgtata 5520
   acgttactgg tttcacattc accaccctga attgactctc ttccgggcgc tatcatgcca 5580
   taccgcgaaa ggttttgcac cattcgatgg tgtcaacgta aatgccgctt cgccttcgcg 5640
   cgcgaattgc aagctgatcc gggcttatcg actgcacggt gcaccaatgc ttctggcgtc 5700
   aggcagccat cggaagctgt ggtatggctg tgcaggtcgt aaatcactgc ataattcgtg 5760
   tcgctcaagg cgcactcccg ttctggataa tgttttttgc gccgacatca taacggttct 5820
   ggcaaatatt ctgaaatgag ctgttgacaa ttaatcatcg gctcgtataa tgtgtggaat 5880
   tgtgagcgga taacaatttc acacaggaaa cagaatt 5917
<210> 24
   <211> 51
   <212> DNA
   <213> M13 phage
<220>
   <221> misc_feature
   <222> (1)..(51)
   <223> nt 1-51 correspond to 1250-1300 of M13 phage
<400> 24
   gtgccttcgt agtggcatta cgtattttac ccgtttaatg gaaacttcct c 51
<210> 25
   <211> 36
   <212> DNA
   <213> artificial
<220>
   <223> primer
<400> 25
   ccaccacaat tggtcctgtt ctggaaaacc gggctg 36
<210> 26
   <211> 36
   <212> DNA
   <213> artificial
<220>
   <223> primer
<400> 26
   ccaccacaat tggtcctgtt ctggaaaacc gggctg 36
<210> 27
   <211> 4
   <212> PRT
   <213> artificial
<220>
   <223> completely synthetic
<400> 27
   Arg Gly Ile Arg
   1
<210> 28
   <211> 4
   <212> PRT
   <213> artificial
<220>
   <223> completely synthetic
<220>
   <221> MISC_FEATURE
   <222> (2) .. (3)
   <223> x=any amino acid
<400> 28
   Arg Xaa Xaa Arg
   1
<210> 29
   <211> 38
   <212> DNA
   <213> artificial
<220>
   <223> primer
<400> 29
   ccaccagaat ttcgcgtcag gcagcggcgc aggcggct 38
<210> 30
   <211> 38
   <212> DNA
   <213> artificial
<220>
   <223> primer
<400> 30
   ccagaattcg aggatcctga cgggatctgg aacggttc 38
<210> 31
   <211> 6
   <212> PRT
   <213> artificial
<220>
   <223> completely synthetic
<400> 31
   Ser Gly Ser Ser Asn Ser
   1 5
<210> 32
   <211> 8
   <212> PRT
   <213> Escherichia coli
<400> 32
   Gln Ala Ala Ala Gln Ala Ala Ala
   1               5
<210> 33
   <211> 20
   <212> DNA
   <213> artificial
<220>
   <223> primer
<400> 33
   ggcagccgcc tgcgccgctg 20
<210> 34
   <211> 24
   <212> DNA
   <213> artificial
<220>
   <223> primer
<400> 34
   ggggactcac tggataaagc aacg 24
<210> 35
   <211> 26
   <212> DNA
   <213> artificial
<220>
   <223> primer
<400> 35
   acgacaaatc ctggtgtatc cgcttg 26
<210> 36
   <211> 22
   <212> DNA
   <213> artificial
<220>
   <223> primer
<400> 36
   atggccacct tgaagctgcc ac 22
<210> 37
   <211> 13
   <212> PRT
   <213> artificial
<220>
   <223> completely synthetic
<400> 37
   Tyr Glu Pro Phe Gln Ile Pro Ser Gly Ser Ser Asn Cys
   1               5                   10
<210> 38
   <211> 35
   <212> DNA
   <213> artificial
<220>
   <223> primer
<400> 38
   ccaccacaat tgccctgttc tggaaaaccg ggctg 35
<210> 39
   <211> 36
   <212> DNA
   <213> artificial
<220>
   <223> primer
<400> 39
   caccatcacc attgaattca gcttggctgt tttggc 36
<210> 40
   <211> 45
   <212> DNA
   <213> artificial
<220>
   <223> primer
<400> 40
   atggtgatgg tgttttttgt tattaatgaa gtttgatttg tgaac 45
<210> 41
   <211> 30
   <212> DNA
   <213> artificial
<220>
   <223> primer
<400> 41
   gatcggacta caaagatgac gatgacaaag 30
<210> 42
   <211> 30
   <212> DNA
   <213> artificial
<220>
   <223> primer
<400> 42
   gatcctttgt catcgtcatc tttgtagtcc 30
<210> 43
   <211> 10
   <212> PRT
   <213> artificial
<220>
   <223> completely synthetic
<400> 43
   Asp Tyr Lys Asp Asp Asp Asp Lys Gly Ser
   1 5 10
<210> 44
   <211> 24
   <212> DNA
   <213> artificial
<220>
   <223> primer
<400> 44
   gatcggagaa cctgtacttc cagg 24
<210> 45
   <211> 24
   <212> DNA
   <213> artificial
<220>
   <223> primer
<400> 45
   gatccctgga agtacaggtt ctcc 24
<210> 46
   <211> 30
   <212> DNA
   <213> artificial
<220>
   <223> oligonucleotide
<400> 46
   gatcggacta caaagatgac gatgacaaag 30
<210> 47
   <211> 30
   <212> DNA
   <213> artificial
<220>
   <223> oligonucleotide
<400> 47
   gatcctttgt catcgtcatc tttgtagtcc 30
<210> 48
   <211> 10
   <212> PRT
   <213> artificial
<220>
   <223> completely synthetic
<400> 48
   Tyr Glu Pro Phe Gln Ile Pro Ser Gly Ser
   1               5                   10
<210> 49
   <211> 19
   <212> PRT
   <213> artificial
<220>
   <223> completely synthetic
<400> 49
   Glu Pro Phe Gln Ile Pro Ser Gly Ser Asp Tyr Lys Asp Asp Asp Asp
   1               5                   10                  15
   Lys Gly Ser
<210> 50
   <211> 6
   <212> PRT
   <213> artificial
<220>
   <223> completely synthetic
<400> 50
   Ser Pro Ser Ala Gln Ala
   1               5
<210> 51
   <211> 9
   <212> PRT
   <213> artificial
<220>
   <223> completely synthetic
<400> 51
   Leu Ile Val Arg Ser Phe Ile Tyr Glu
   1               5
<210> 52
   <211> 11
   <212> PRT
   <213> artificial
<220>
   <223> completely synthetic
<400> 52
   Leu Ile Val Ser Pro Ser Ala Gln Ala Tyr Glu
   1 5 10
<210> 53
   <211> 35
   <212> DNA
   <213> artificial
<220>
   <223> primer
<400> 53
   gcacaggcgg catatgaacc gttccagatc ccgtc 35
<210> 54
   <211> 33
   <212> DNA
   <213> artificial
<220>
   <223> primer
<400> 54
   agacggggac acaatcaata cgatagccag tac 33
<210> 55
   <211> 37
   <212> DNA
   <213> artificial
<220>
   <223> primer
<400> 55
   ccaggatccg aaagttattt gacaaaacaa gccgtac 37
<210> 56
   <211> 41
   <212> DNA
   <213> artificial
<220>
   <223> primer
<400> 56
   caagaattca caataaatct tcaacttgct tttttatgta g 41
<210> 57
   <211> 38
   <212> DNA
   <213> artificial
<220>
   <223> primer
<400> 57
   caacaacggc cgaaagttat ttgacaaaac aagccgta 38
<210> 58
   <211> 41
   <212> DNA
   <213> artificial
<220>
   <223> primer
<400> 58
   caagaattca caataaatct tcaacttgct tttttatgta g 41
<210> 59
   <211> 36
   <212> DNA
   <213> artificial
<220>
   <223> primer
<400> 59
   caacaagcgg ccgcctgtgc agacggggac acaatc 36
<210> 60
   <211> 26
   <212> DNA
   <213> artificial
<220>
   <223> primer
<400> 60
   cgggaattca gcttggctgt tttggc 26
<210> 61
   <211> 27
   <212> DNA
   <213> artificial
<220>
   <223> primer
<400> 61
   atggaaagtt atttgacaaa acaagcc 27
<210> 62
   <211> 30
   <212> DNA
   <213> artificial
<220>
   <223> primer
<400> 62
   cgcctgtgca gacggggaca caatcaatac 30
<210> 63
   <211> 30
   <212> DNA
   <213> artificial
<220>
   <223> primer
<220>
   <221> misc_feature
   <222> (8)..(8)
   <223> Deoxyuridine
<400> 63
   aggtaccuga attcagcttg gctgttttgg 30
<210> 64
   <211> 30
   <212> DNA
   <213> artificial
<220>
   <223> primer
<220>
   <221> misc_feature
   <222> (10)..(10)
   <223> Deoxyuridine
<400> 64
   agcctgtgcu gacggggaca caatcaatac 30
<210> 65
   <211> 34
   <212> DNA
   <213> artificial
<220>
   <223> primer
<220>
   <221> misc_feature
   <222> (10)..(10)
   <223> Deoxyuridine
<400> 65
   agcacaggcu gtttttgaaa ctgaagaggc actt 34
<210> 66
   <211> 34
   <212> DNA
   <213> artificial
<220>
   <223> primer
<220>
   <221> misc_feature
   <222> (8)..(8)
   <223> Deoxyuridine
<400> 66
   aggtaccuta tttgacttcc tttactattt ctgc 34
<210> 67
   <211> 8
   <212> PRT
   <213> Tobacco etch virus
<400> 67
   Glu Asn Leu Tyr Phe Gln Gly Ser
   1               5

## Description

### BACKGROUND OF THE INVENTION

Attempts have been made to express recombinant eukaryotic membrane proteins in bacteria but these methods are problematic for reasons that include toxicity and insolubility, and the undesirable formation of inclusion bodies (Grisshammer et al. Biochem. J. 295:571-576 (1993); Tucker and Grisshammer Biochem. J. 317:891-899 (1996); Weiss and Grisshammer Eur. J. Biochem. 269:82-92 (2002); Yeliseev et al. Protein Sci. 14:2638-53 (2005); Krepkiy et al. Protein Expr Purif. 49:60-70 (2006); Yeliseev et al. Protein Expr Purif. 53:153-163 (2007)). In addition there is a lack of reliable methods for overexpression of recombinant membrane proteins in *E. coli.*

Eukaryotic membrane proteins are targeted to various compartments within a eukaryotic cell by means of an address that is encoded in an N-terminal signal sequence or C-terminal targeting sequence (Emanuelsson et al. Nature Protocols 2:953-971 (2007)). The signal sequence is typically removed during biogenesis.

In bacteria that are not membrane-compartmentalized, most newly translated membrane proteins, even while associated with the ribosome, are recognized by signal recognition particles (SRP) and the SRP receptor protein for targeting to the inner membrane surrounding the bacterial cell. Most inner membrane proteins studied in *E. coli* use the SRP pathway and the Sec translocase for targeting to the membrane and membrane assembly. In addition, a subset of membrane proteins has an absolute requirement for YidC, a polytopic inner membrane protein of *E. coli* that aids the membrane insertion process. YidC is essential for *E. coli* cell viability (Samuelson et al. Nature 406:637-641 (2000); Urbanus et al. EMBO Rep. 2:524-529 (2001); van Bloois et al. J. Biol. Chem. 280:12996-13003 (2005)).

A method of choice for stable expression of proteins that rely on disulfide bonds for their native structure is to export the newly synthesized protein into the *E. coli* periplasm which contains disulfide bond catalyzing enzymes (SRP-dependent DsbA, DsbB, DsbC and DsbD) (Bardwell, et al. Cell 67:581 (1991); Kamitani et al. EMBO J. 11:57 (1992); Novagen (now EMD Chemicals, Inc., Gibbstown, NJ) pET system manual (11^{th} ed. Jan. 2007); Missiakas et al. EMBO J. 13:2013-2020 (1994); and Zapun et al. Biochemistry 34:5075-5089 (1995)).

Candidate proteins for export to the periplasm can be identified by an N-terminal signal peptide using signal peptide search algorithms such as Phobius (http://phobius.sbc.su.se/) (Käll et al., Nucleic Acids Res. 35:W429-32 (2007)) and Signal P http://www.cbs.dtu.dk/services/SignaIP/) (Emanuelsson et al. Nat Protoc. 2(4):953-71 (2007)).

Export to the periplasm is however limited by the throughput capacity of the Sec protein export machinery (Pérez-Pérez, et al. Biotechnology 12(2):178-80 (1994)) in which the Sec translocase may become saturated and result in the cytoplasmic accumulation of native secreted proteins as well as the overexpressed protein of interest (Wagner et al. Mol Cell Proteomics 6(9):1527-50 (2007)). This is more commonly observed when the protein of interest is non-native to *E. coli* and/or is not normally secreted. Alternatively, a non-native protein may become incorrectly folded during synthesis and hence become unsuited for Sec-mediated export across the inner membrane.

### SUMMARY

In one embodiment, the present invention provides a composition characterized in that it comprises: a recombinant DNA encoding an N-terminal protein vehicle for transporting a protein of interest fused to the vehicle from the cytoplasmic compartment to the envelope of a prokaryotic cell;
the encoded protein vehicle comprising: a membrane-targeting peptide characterized by a Goldman-Engelman-Steitz hydrophobicity score of at least 1.52 for an amino acid window size of 21, a cytoplasmic affinity-binding domain and a trans-membrane segment:
and
the DNA encoding the protein vehicle being fused to a DNA encoding a protein of interest.

More particularly, the present invention relates to such a composition: wherein the encoded membrane-targeting peptide has an amino acid window of 21 amino acids such that within the 21 amino acid window there is a hydrophobic core sequence of at least 9 amino acids lacking Asp, Glu, Arg and Lys; or wherein the encoded membrane-targeting peptide is YidC-dependent, preferably wherein the encoded membrane-targeting peptide is selected from pVIII, Pf3 coat and subunit C variant L31F; or wherein the encoded cytoplasmic affinity-binding domain is a carbohydrate-binding domain, preferably wherein the encoded cytoplasmic affinity-binding domain is a chitin-binding domain; or wherein the encoded cytoplasmic affinity-binding domain is selected from a His tag and a strep tag; or wherein the encoded trans-membrane segment has an N-terminal end and a C-terminal end, the segment having a 21 amino acid sequence window such that within the 21 amino acid window there is a core sequence of at least 9 amino acids lacking Asp, Glu, Arg and Lys and the 21 amino acid window is flanked on the N-terminal end by an amino acid sequence that comprises at least 9 amino acids having an overall charge of at least +1, preferably wherein the 21 amino acid window is flanked on the C-terminal end by an amino acid linker sequence, more preferably wherein the linker sequence contains a signal peptidase cleavage site, or wherein the linker sequence contains a heterologous protease cleavage site, preferably wherein the encoded trans-membrane segment is TM2 of signal peptidase.

In another embodiment, the present invention provides a fusion protein characterized in that it comprises: a membrane-targeting peptide characterized by a Goldman-Engelman-Steitz hydrophobicity score of at least 1.52 for an amino acid window size of 21, a cytoplasmic affinity-binding domain, a trans-membrane segment and a protein of interest.

More particularly, the present invention relates to such a fusion protein: wherein the protein of interest is a toxic protein, preferably wherein the toxic protein of interest is a restriction endonuclease, more preferably wherein the restriction endonuclease is Sau3AI or isoschizomer thereof.

In a further embodiment, the present invention provides a method of producing a recombinant protein in a gram-negative bacterial host cell characterized in that comprises:
(a) obtaining a recombinant DNA characterized as above fused to a DNA encoding a protein of interest;
   and
(b) transforming the host cell.

More particularly, the present invention relates to such a method: wherein the recombinant protein is purified from the host cell by binding the cytoplasmic affinity-binding domain to an affinity substrate; or wherein it further comprises: assaying the transformed host cell for detection of the protein of interest by binding to an affinity substrate via the cytoplasmic affinity-binding domain; or wherein the host cell has a DsbA⁻ phenotype, preferably wherein it further comprises: assaying the transformed host cell for determining at least one of enzymatic and binding activity of the protein of interest; or wherein the protein of interest is Sau3AI; or wherein the protein of interest is BfuCI.

In another embodiment, the present invention provides a DNA characterized in that it comprises: nucleotide sequence 631-1986 in accompanying SEQ ID NO: 23.

In a further embodiment, the present invention provides a method characterized in that it comprises: producing a restriction endonuclease in the absence of cognate methylation according to the above method.

More particularly, the present invention relates to such a method wherein the restriction endonuclease is Sau3AI or isoschizomer thereof.

In another embodiment, the present invention provides a vector characterized in that it comprises a DNA characterized as above.

In a further embodiment, the present invention provides a host cell characterized in that it comprises such a vector.

Having indicated the scope of the present invention, it will now be further described and illustrated in context in more general terms.

As mentioned above, an embodiment of the invention provides a recombinant DNA for introduction into a prokaryotic host cell having a cytoplasmic compartment and an envelope. The recombinant DNA encodes an N-terminal protein vehicle for transporting a protein of interest fused to the vehicle from the cytoplasmic compartment to the envelope where the encoded protein vehicle includes a membrane targeting peptide, a cytoplasmic affinity-binding domain, and a trans-membrane segment. The DNA encoding the protein vehicle is preferably fused to DNA encoding a protein of interest.

In one embodiment, the encoded membrane-targeting peptide is characterized by a Goldman-Engelman-Steitz (GES) hydrophobicity score of at least 1.52 for a window size of 21. In an additional embodiment, the encoded membrane-targeting peptide and the encoded trans-membrane segment which may be different from each other, nonetheless have an amino acid window of 21 amino acids such that within the 21 amino acid window there is a hydrophobic core sequence of at least 9 amino acids lacking Asp, Glu, Arg, and Lys. In an additional embodiment, the membrane-targeting peptide is YidC-dependent. Examples of YidC-dependent peptides are PVIII, Pf3 coat, and subunit C variant L31F. In an additional embodiment, the 21 amino acid window of the trans-membrane segment is flanked on the N-terminal end by an amino acid sequence that comprises at least 9 amino acids having an overall charge of at least +1. Additionally, the trans-membrane segment may be flanked on the C-terminal end by an amino acid linker sequence which may contain a signal peptidase cleavage site. The amino acid linker sequence may contain a heterologous protease cleavage site. The encoded trans-membrane segment may be TM2 of signal peptidase.

In an embodiment of the invention, the cytoplasmic affinity-binding domain is a carbohydrate-binding domain, for example a chitin-binding domain or a His tag and a strep tag.

In an embodiment of the invention, a fusion protein is provided that includes a membrane-targeting peptide, a cytoplasmic affinity-binding domain, and a trans-membrane segment. The fusion protein may additionally include a protein of interest. For example, the protein of interest may be membrane protein or a toxic protein such as a recombinant restriction endonuclease such as Sau3AI or an isoschizomer thereof.

In an embodiment of the invention, a method is provided for producing a recombinant protein in a gram-negative bacterial host cell that includes obtaining a recombinant DNA encoding a protein vehicle as characterized above fused to a DNA encoding a protein of interest and transforming the host cell with this DNA. The recombinant protein may be purified from the host cell by binding the cytoplasmic affinity-binding domain to an affinity substrate. The affinity substrate may be used to detect, purify and/or assay the amount of the expressed protein of interest. The enzymatic activity of the expressed protein of interest may also be determined. Examples of a protein of interest include Sau3AI and BfuCI. Where the protein of interest contains cysteines but does not require disulfide bridges, then the host cell preferably has a DsbA⁻ phenotype due to a mutation in the *dsbA* gene.

In an embodiment of the invention, a DNA comprising nucleotide sequence 631-1986 SEQ ID NO:23 is provided.

In an embodiment of the invention, a method is provided for producing a restriction endonuclease in the absence of cognate methylation as described above for Sau3AI or isoschizomer thereof. Additionally a vector is provided that includes the recombinant DNA described above. A host cell is also provided for transforming with the vector.

### BRIEF DESCRIPTION OF THE DRAWINGS

Figure 1 shows the membrane topology of pVIII-PhoA1 fusion protein with PhoA1 located in the cytoplasm.
Figure 2 shows the membrane topology of pVIII-TM2 in which the protein of interest is exported to the periplasm. ("TM2" is an abbreviation for any trans-membrane segment.)
Figure 3 shows the membrane topology of pVIII-8His in which the protein of interest is located between the pVIII and the fusion junction.

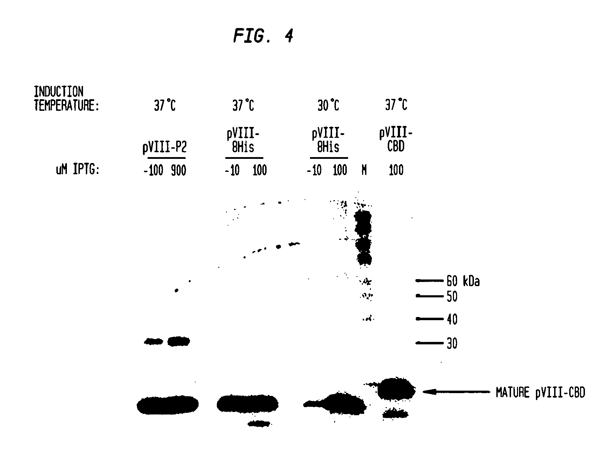

Figure 4 shows an anti-pVIII immunoblot demonstrating expression of various membrane-targeting peptides. pVIII-P2 is a control fusion protein expressed from the parent expression clone pVIII-P2. ("P2" is the abbreviation for the protein of interest.) pVIII-8His contains a cytoplasmic eight-histidine affinity tag. pVIII-CBD contains a cytoplasmic chitin-binding domain (CBD) affinity tag. Protein is not detected without IPTG addition (-) confirming IPTG-inducible expression control with all pMS119-derived plasmids. Lane (M) contains a biotinylated protein ladder (Cell Signaling Technology #7727S, Beverly, MA) detected by anti-biotin, HRP-linked antibody.
Figure 5 shows the membrane topology of pVIII-CBD.
Figure 6 shows the membrane topology of pVIII-CBD-PhoA9 fusion protein.
Figure 7 shows the membrane topology of pVIII-CBD-Oxa1p fusion protein, where Oxa1p is a eukaryotic membrane protein.
Figure 8 shows the membrane topology of pVIII-CBD-Oxa8His fusion protein.
Figure 9 shows the membrane topology of pVIII-CBD-Ek-Oxa8His fusion protein.
Figure 10 shows the membrane topology of pVIII-CBD-Tev-Oxa8His fusion protein. ("His" is an abbreviation for a histidine peptide for affinity binding. "Tev" is an abbreviation for Tobacco Etch Virus.)
Figure 11A shows an immunoblot where anti-pVIII monoclonal antibody was used to identify purified pVIII-CBD-Ek-Oxa1p-8His fusion protein. All samples were prepared for electrophoresis by addition of SDS sample buffer (New England Biolabs, Inc. (NEB) #B7703S, Ipswich MA) followed by heating at 37°C for 5 min.
   Lane (M) contains a biotinylated protein ladder (Cell Signaling Technology #7727S, Beverly, MA) detected by anti-biotin, HRP-linked antibody.
   Lane (m) was loaded with a sample of the membrane preparation before complete solubilization with n-dodecyl-beta-D-maltopyranoside (DDM).
   Lane (L) shows the composition of the load sample after DDM solubilization and removal of insoluble material by centrifugation.
   Lane (ft) shows the composition of the Nickel-NTA resin supernatant (flow-through) after incubation with load sample.
   Lane (e1) shows the eluted protein after addition of 80 µL elution buffer containing 400 mM imidazole.
   Lane (e2) shows the composition of a second 80 µL elution fraction. The arrow indicates the purified pVIII-CBD-Ek-Oxa1p-8His protein with calculated molecular weight of 62247 daltons.

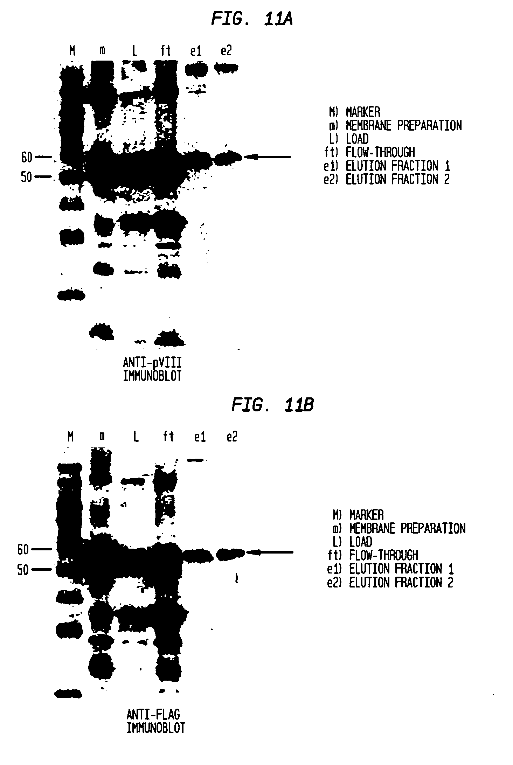

Figure 11B shows an immunoblot where anti-FLAG monoclonal antibody was used to confirm purification of pVIII-CBD-Ek-Oxa1p-8His fusion protein. The lane descriptions from Figure 11A apply to Figure 11B. The arrow indicates the purified pVIII-CBD-Ek-Oxa1p-8His protein with calculated molecular weight of 62247 daltons.

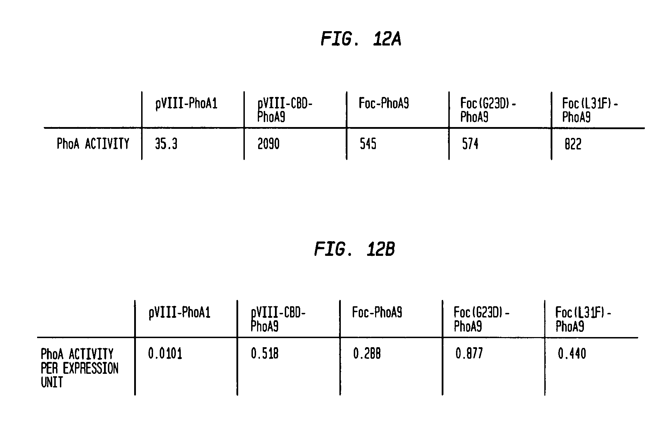

Figures 12A and 12B are tables showing PhoA activity for different constructs (Figure 12A) and PhoA activity per expression unit (Figure 12B). DHB4 cells were grown at 30°C and induced for 1 hr with 1 mM IPTG to express the exemplified fusion proteins.

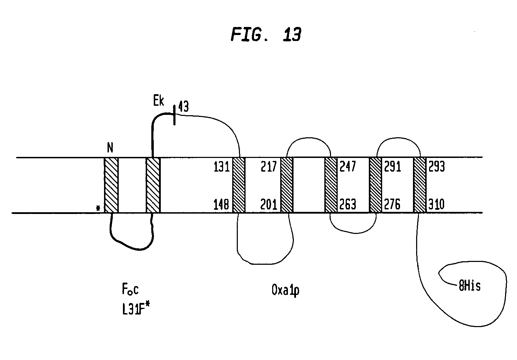

Figure 13 shows the membrane topology of a mutant subunit C Fₒc(L31F) fused to a eukaryotic membrane protein (Oxa1p).

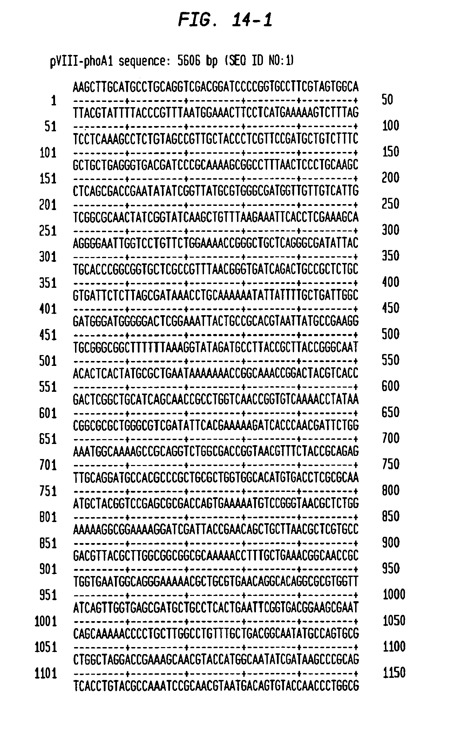

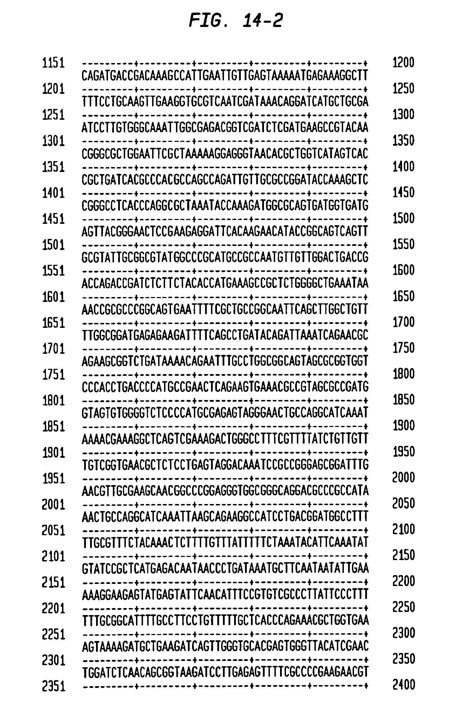

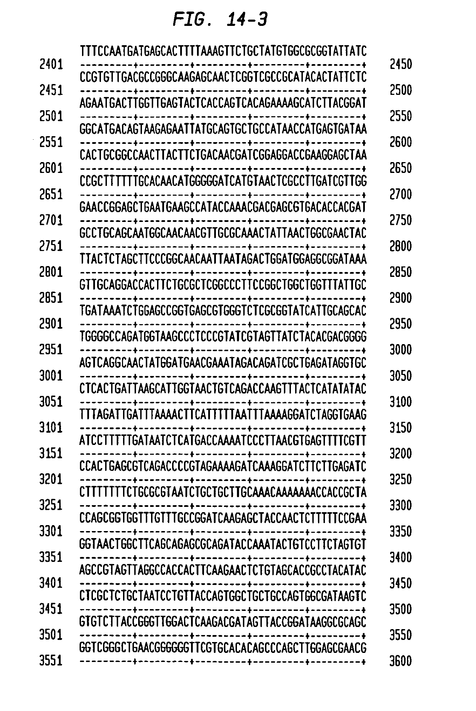

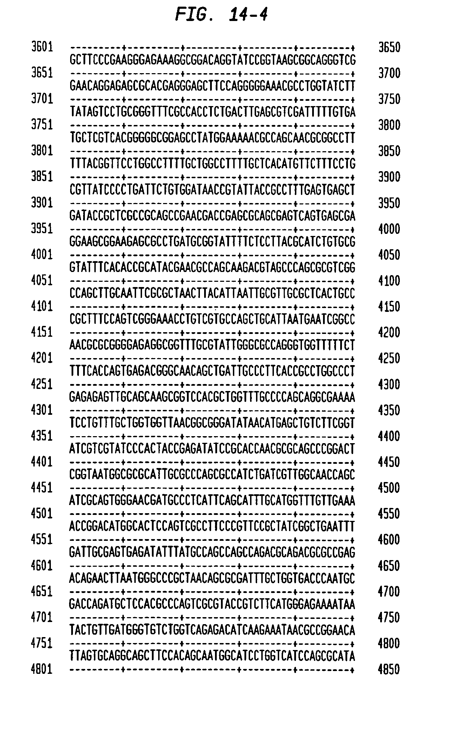

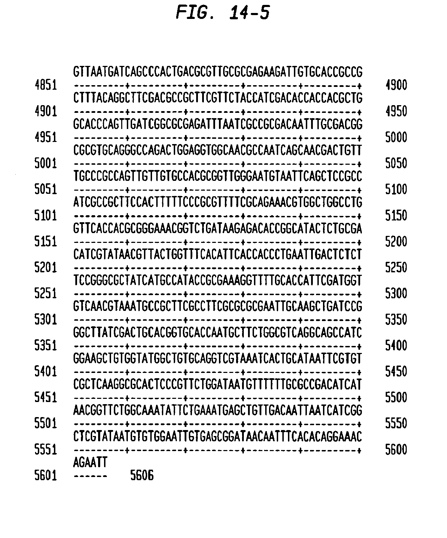

Figure 14 shows the pVIII-PhoA1 sequence: 5606 bp (SEQ ID NO:1).

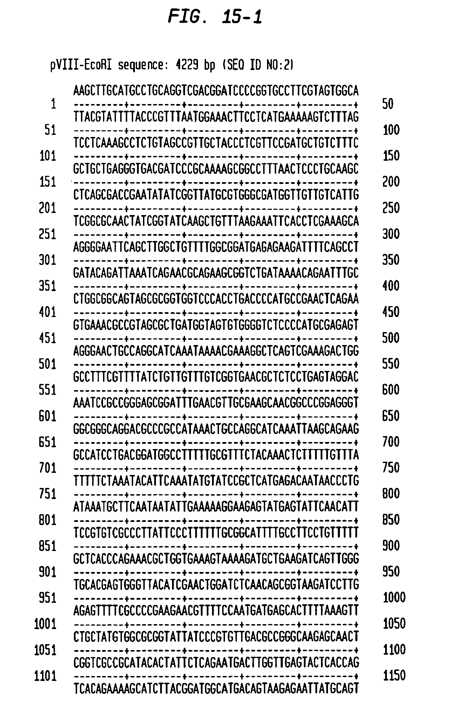

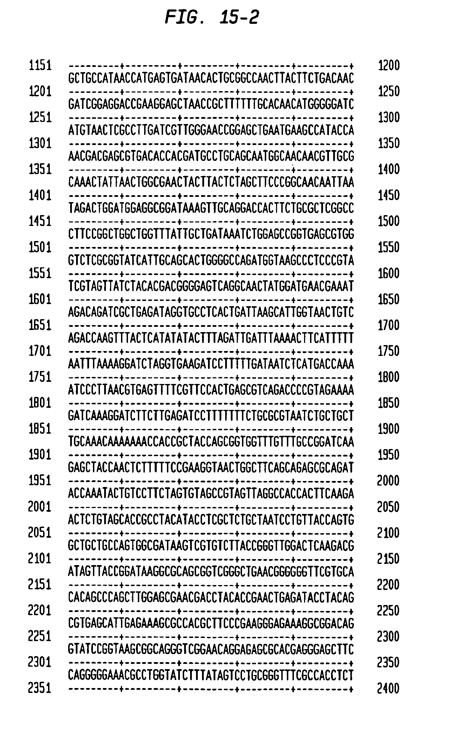

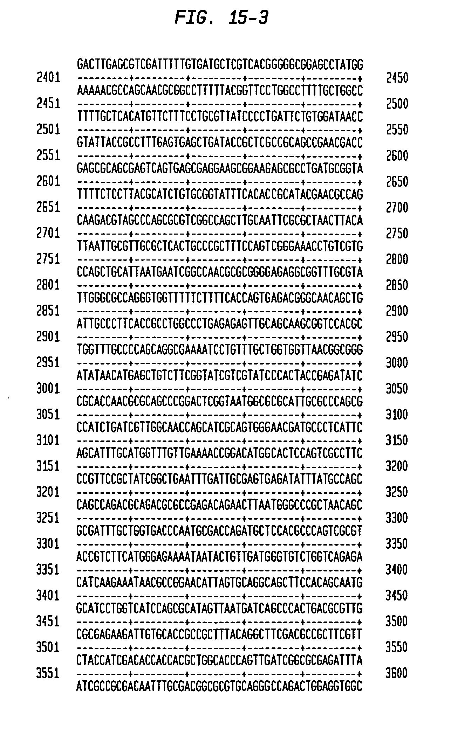

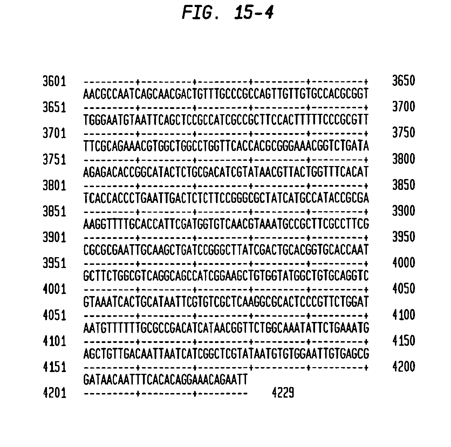

Figure 15 shows the pVIII-EcoRI sequence: 4229 bp (SEQ ID NO:2).

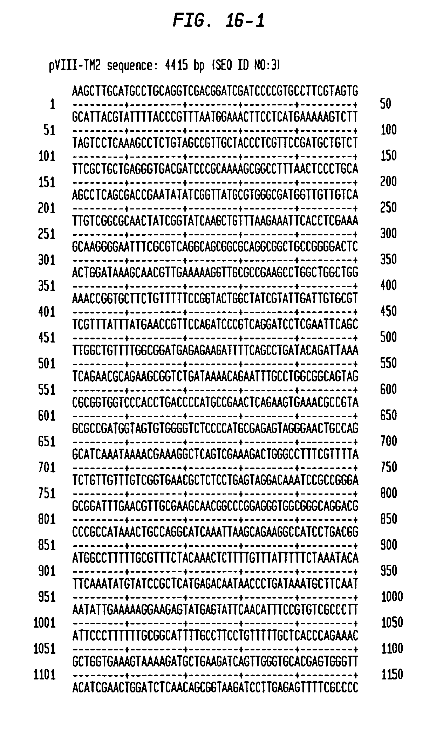

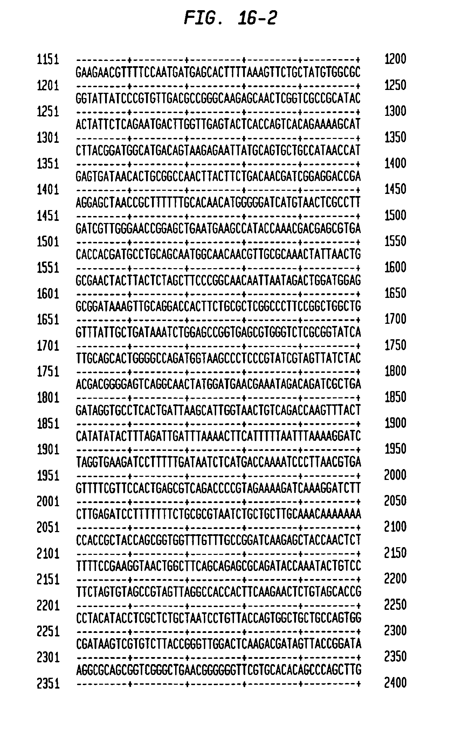

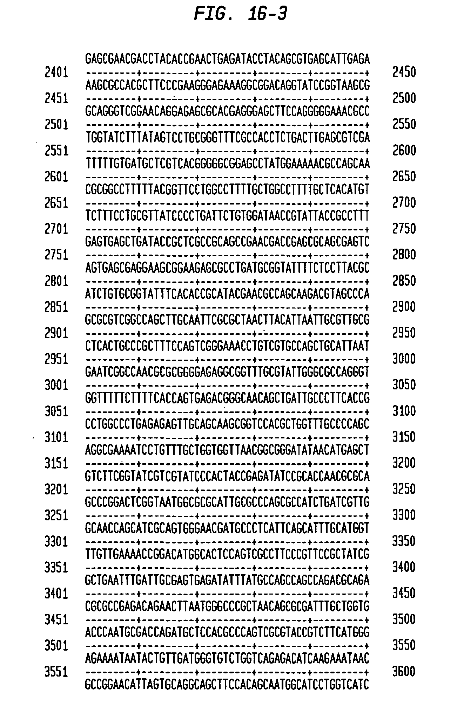

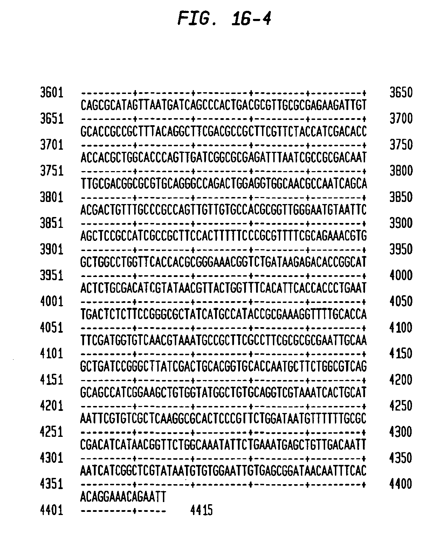

Figure 16 shows the pVIII-TM2 sequence: 4415 bp (SEQ ID NO:3).

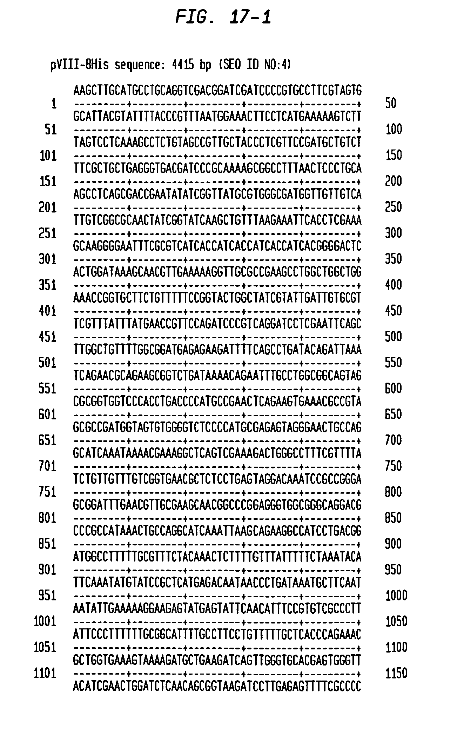

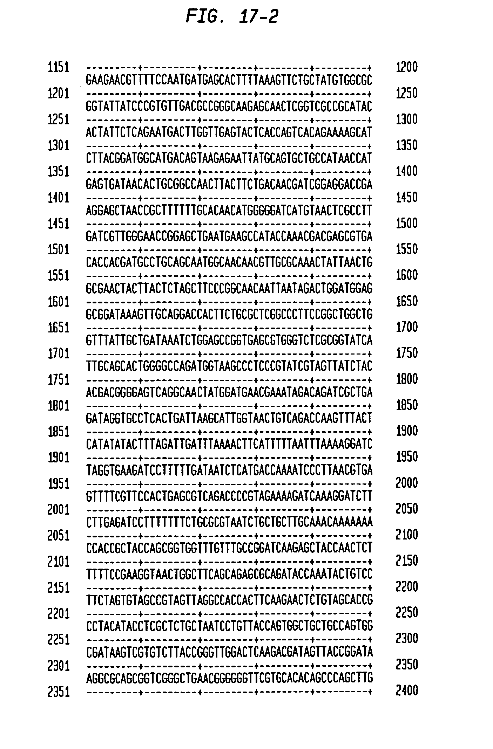

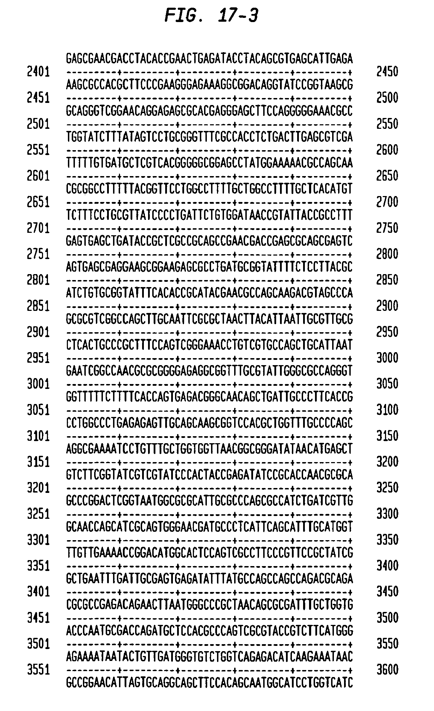

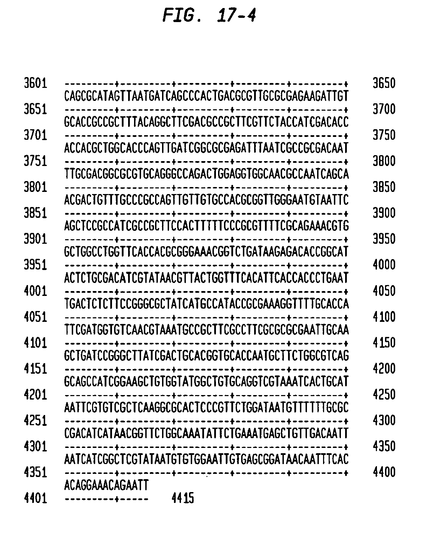

Figure 17 shows the pVIII-8His sequence: 4415 bp SEQ ID NO:4).

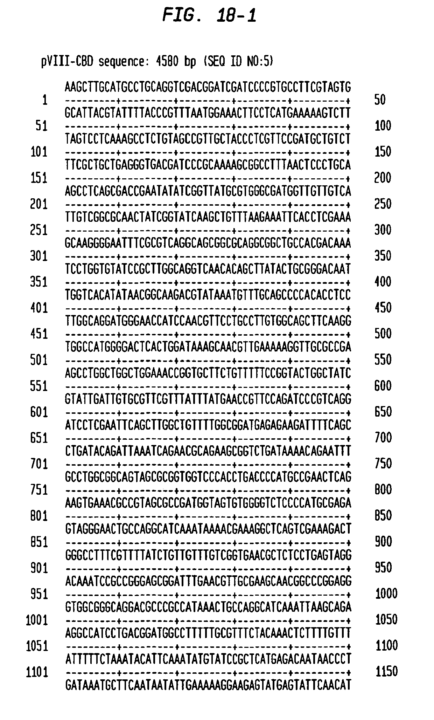

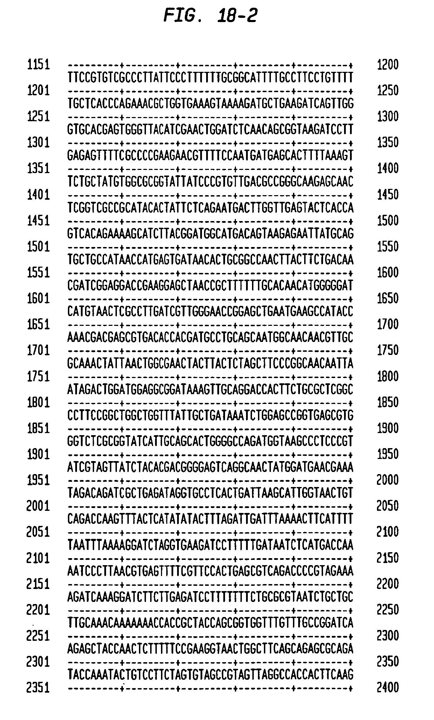

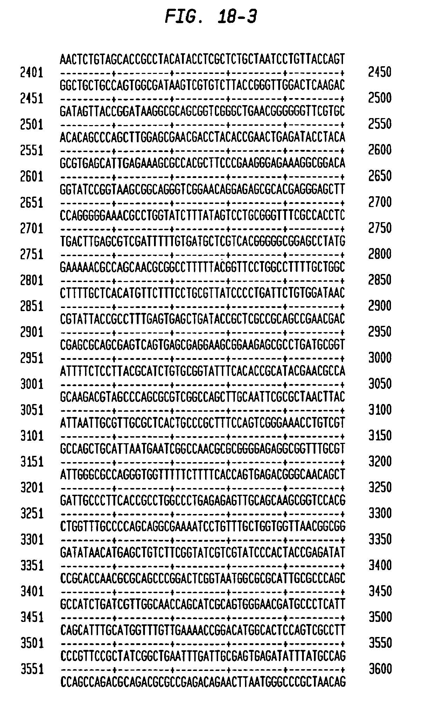

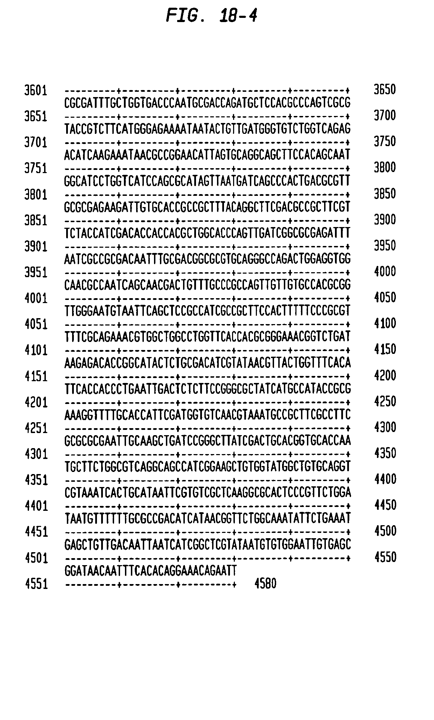

Figure 18 shows the pVIII-CBD sequence: 4580 bp (SEQ ID NO:5).

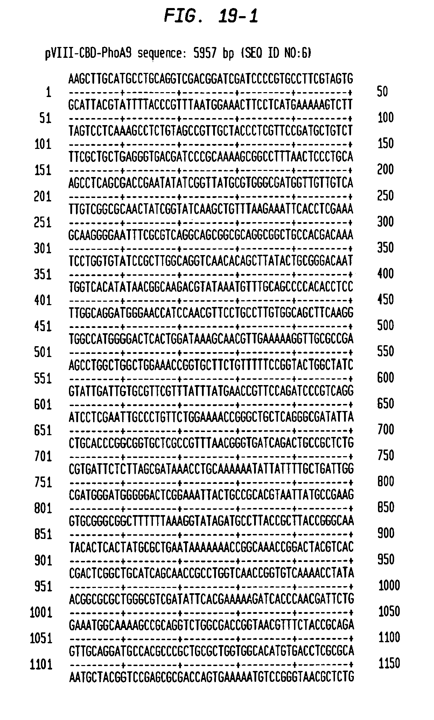

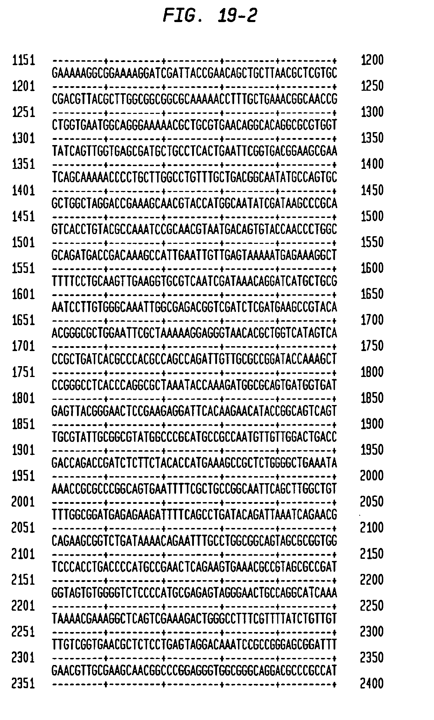

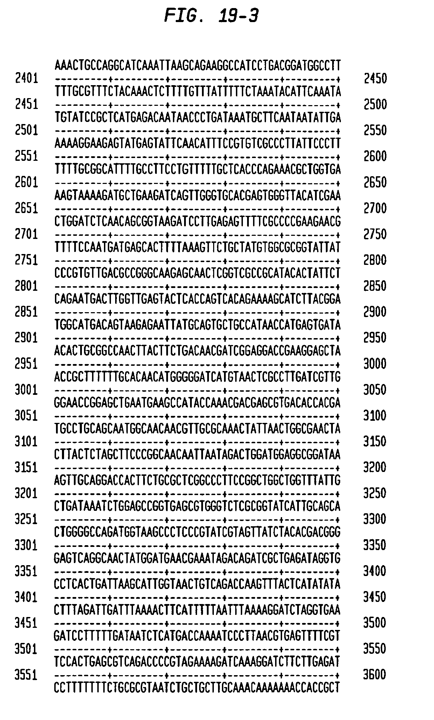

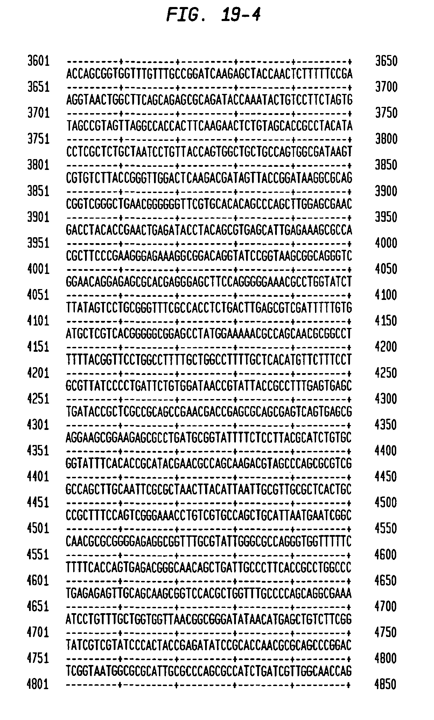

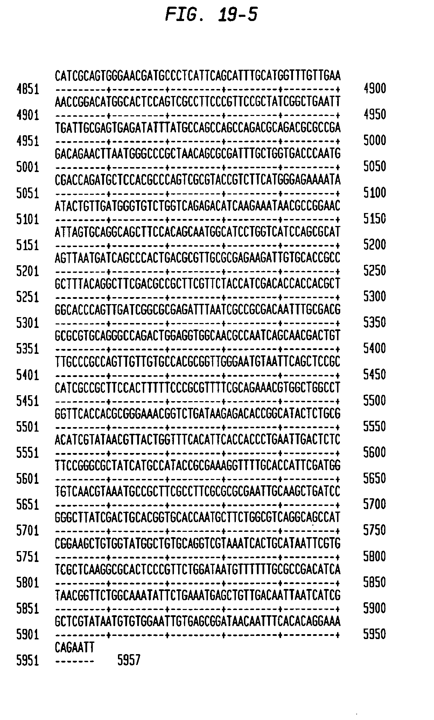

Figure 19 shows the pVIII-CBD-PhoA9 sequence: 5957 bp (SEQ ID NO:6).

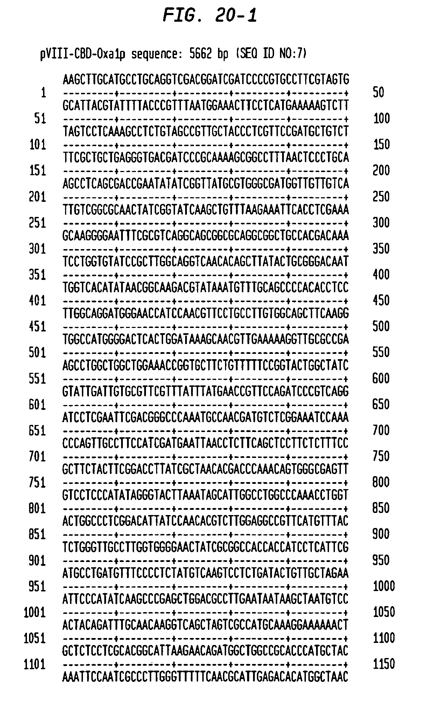

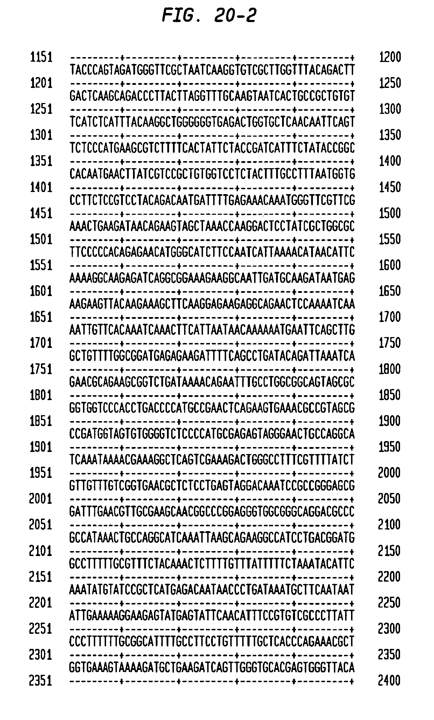

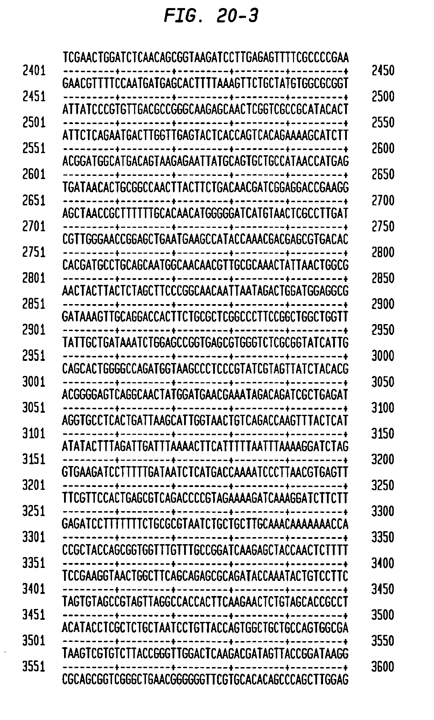

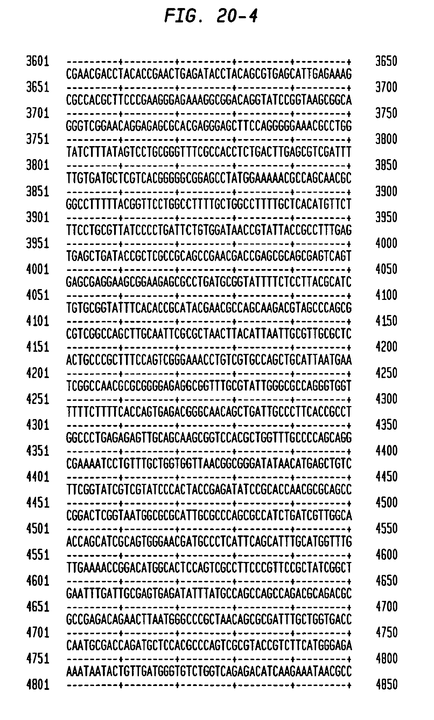

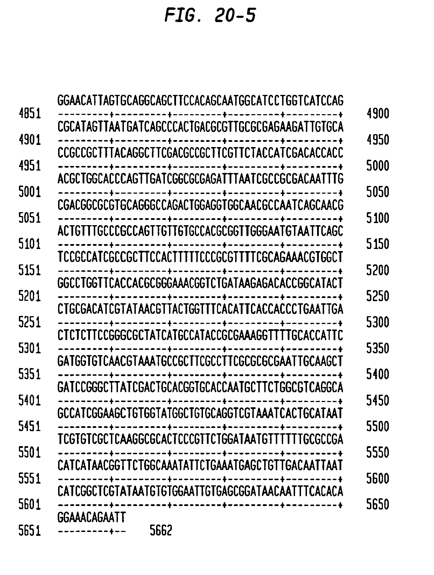

Figure 20 shows the pVIII-CBD-Oxa1p sequence: 5662 bp (SEQ ID NO:7).

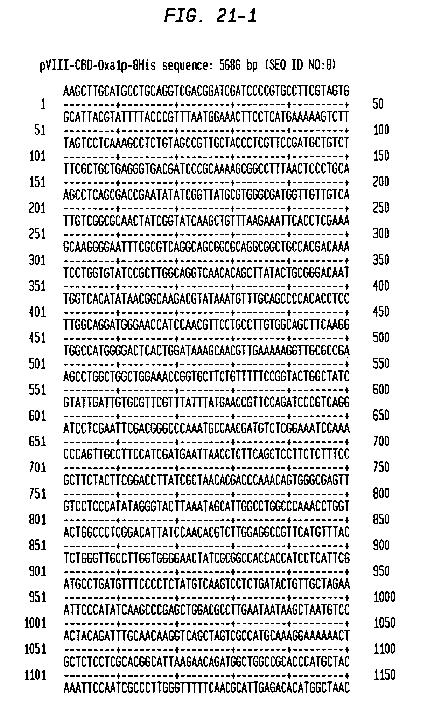

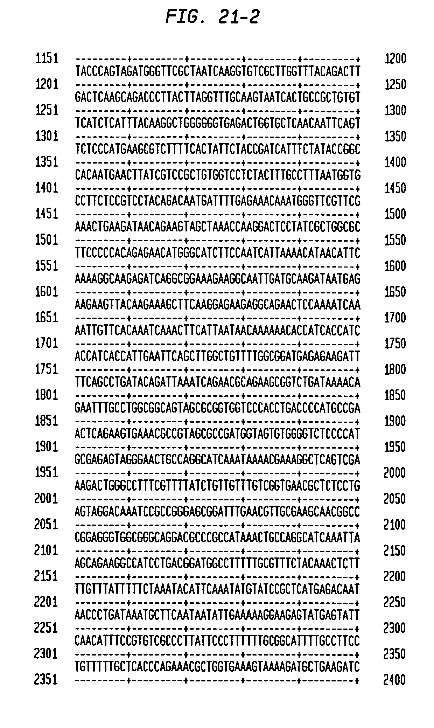

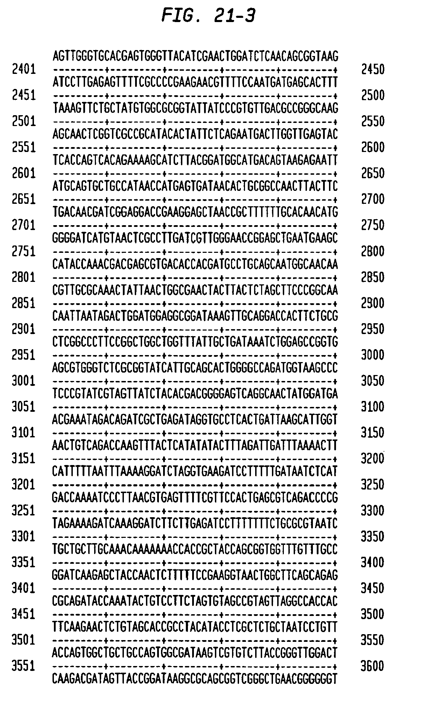

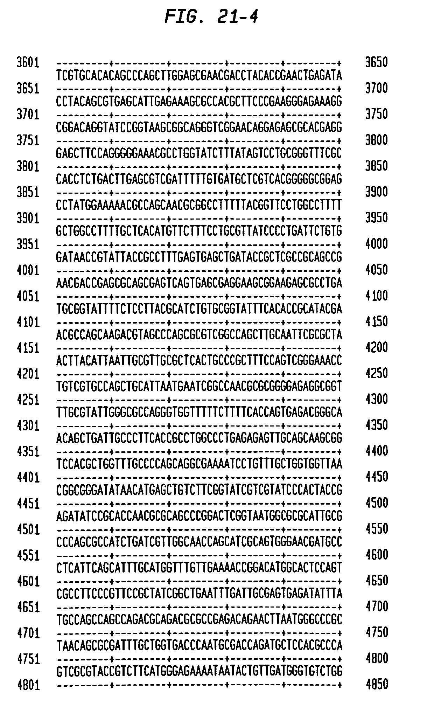

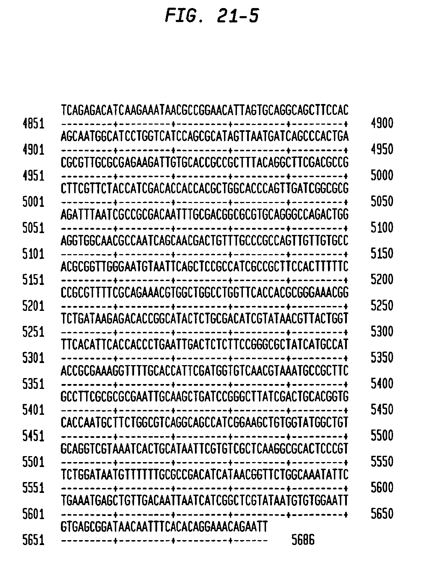

Figure 21 shows the pVIII-CBD-Oxa1p-8His sequence: 5686 bp (SEQ ID NO:8).

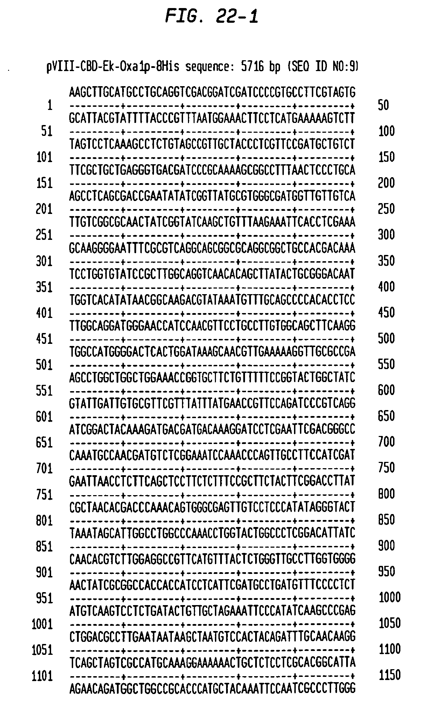

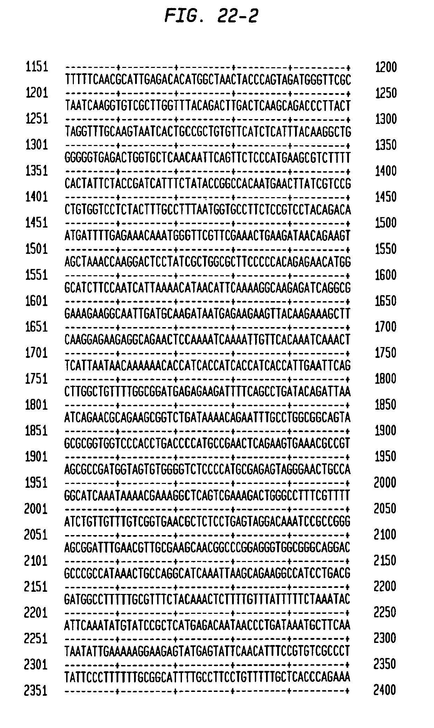

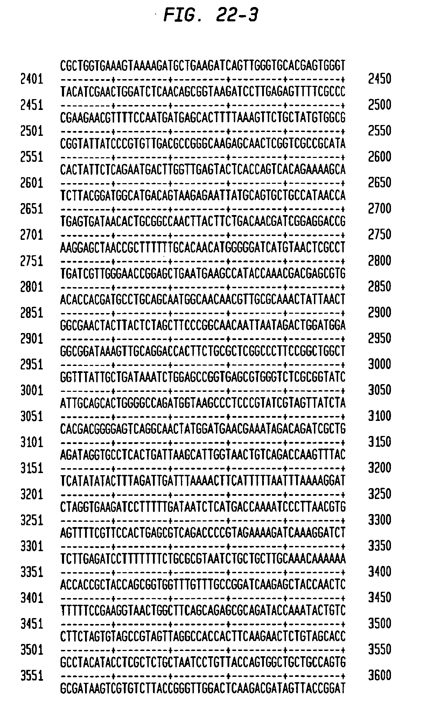

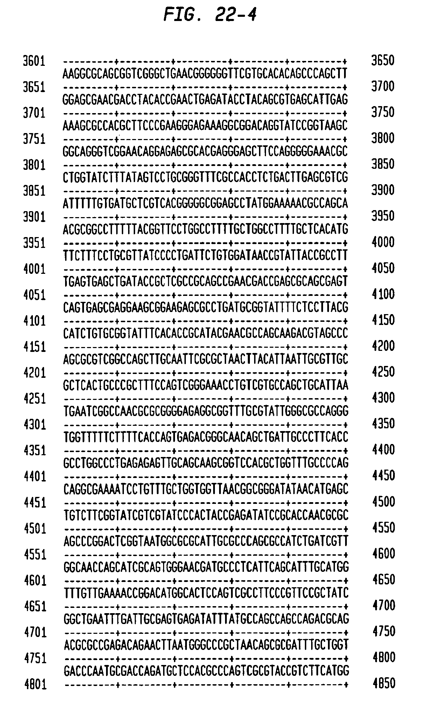

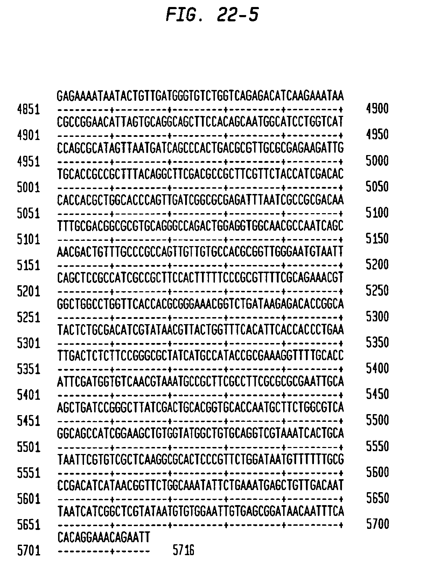

Figure 22 shows the pVIII-CBD-Ek-Oxa1p-8His sequence: 5716 bp (SEQ ID NO:9).

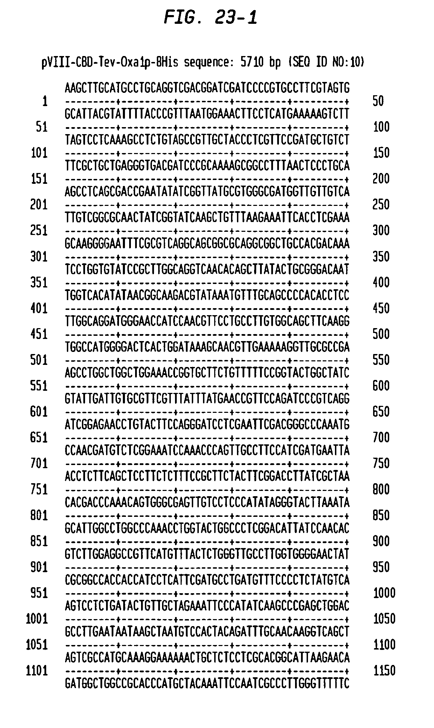

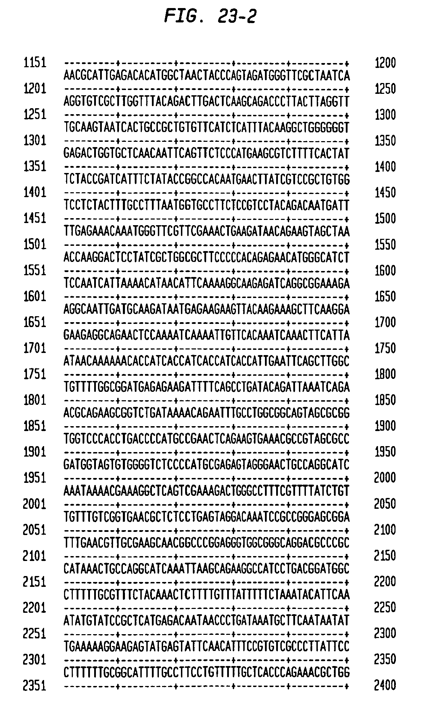

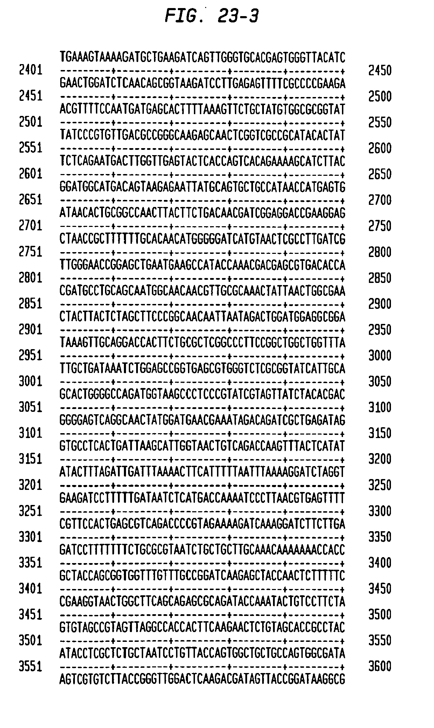

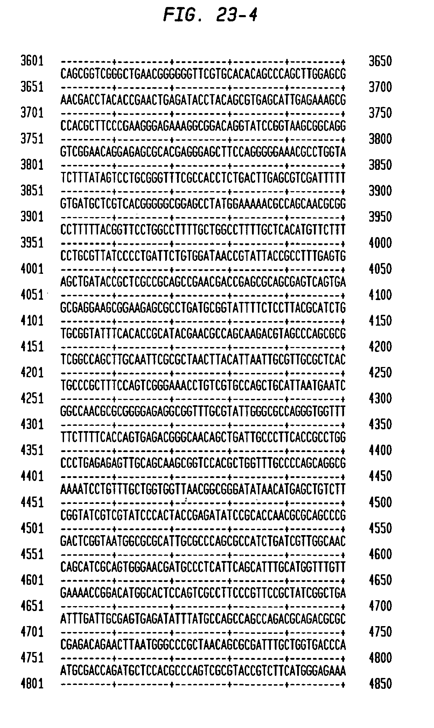

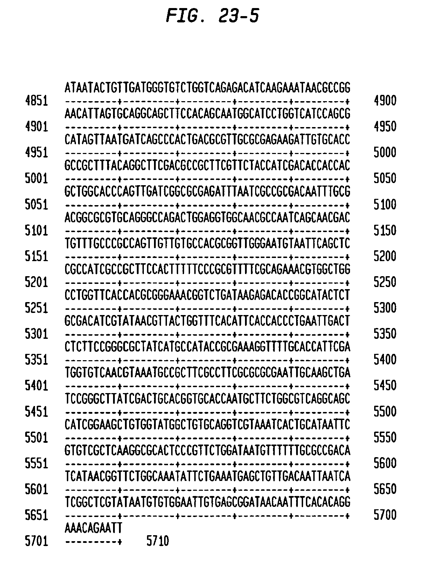

Figure 23 shows the pVIII-CBD-Tev-Oxa1p-8His sequence: 5710 bp (SEQ ID NO:10).

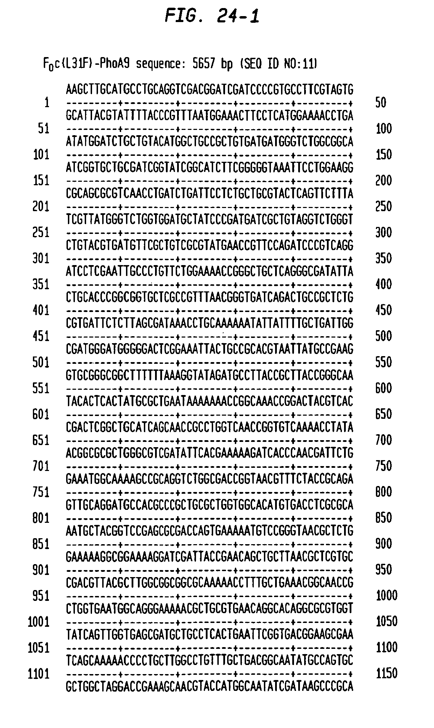

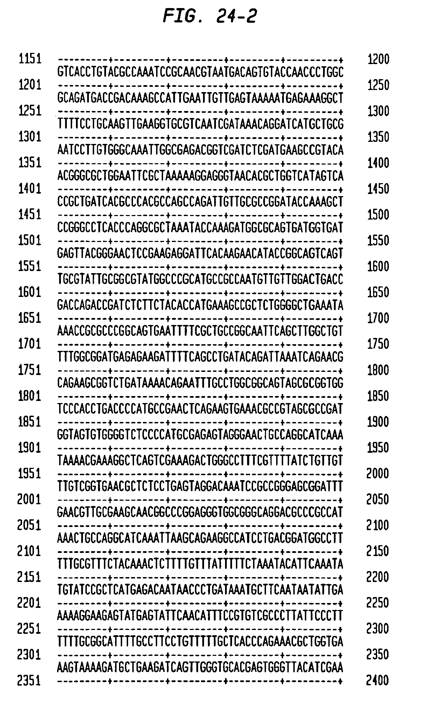

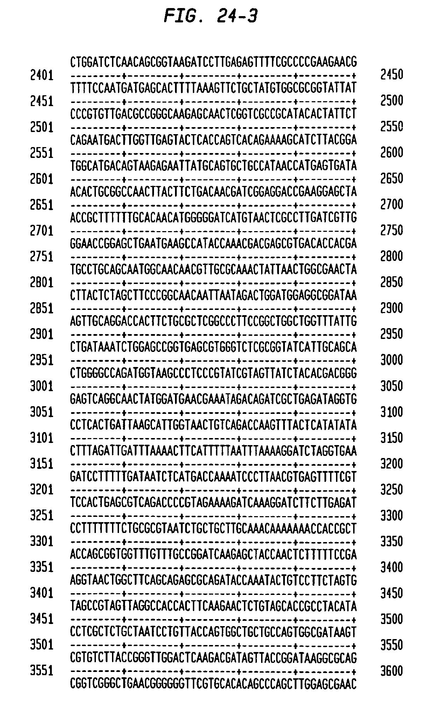

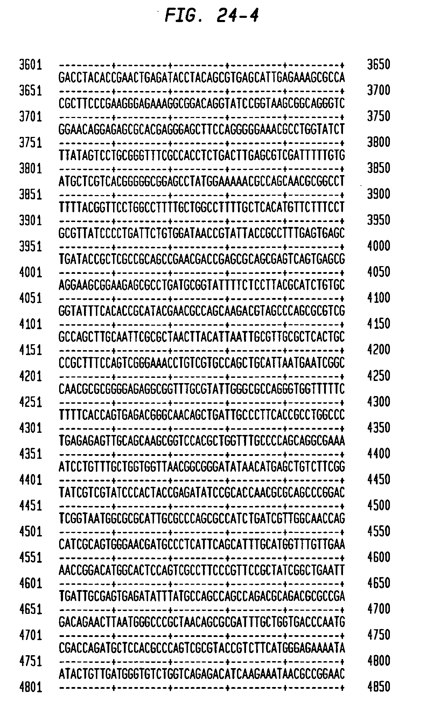

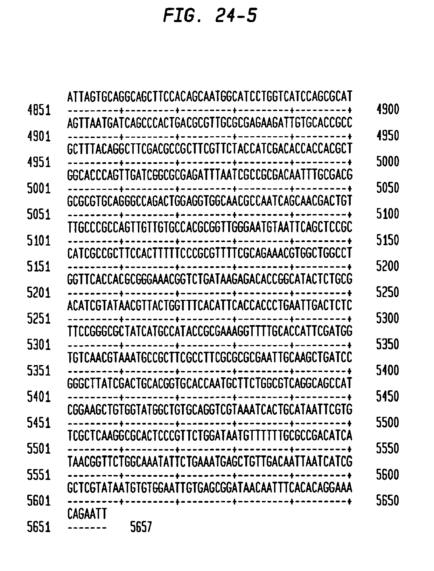

Figure 24 shows the Fₒc(L31F)-PhoA9 sequence: 5657 bp (SEQ ID NO:11).

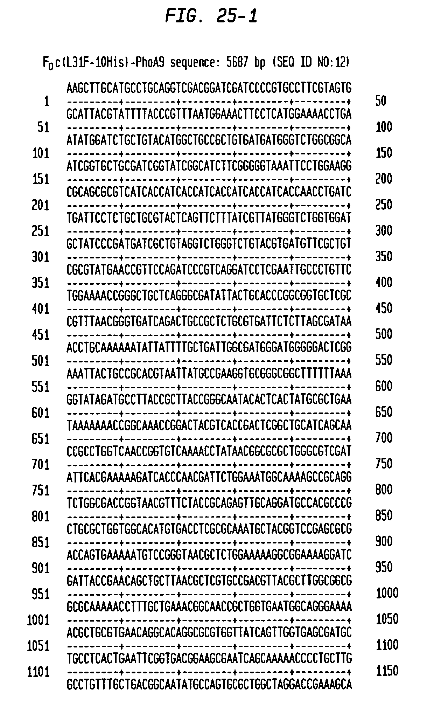

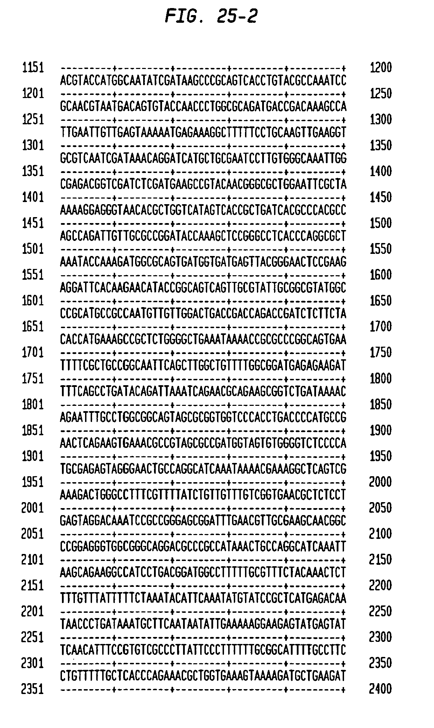

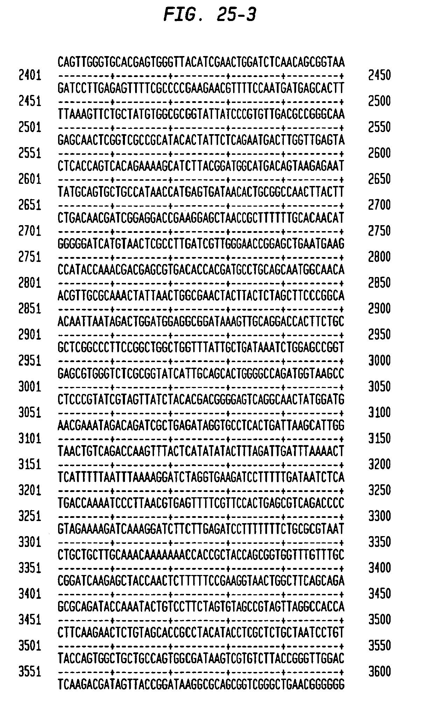

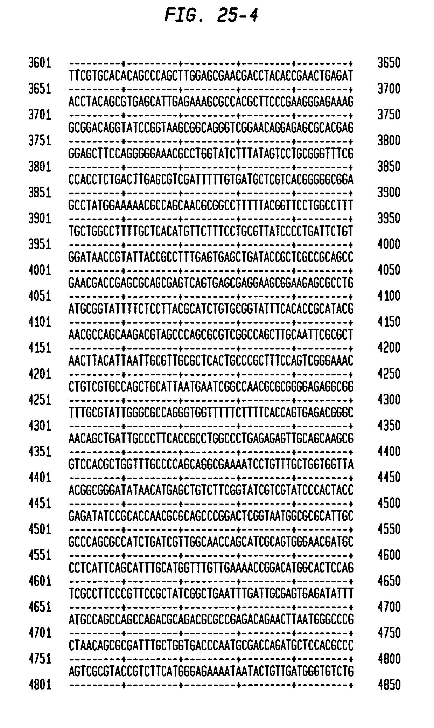

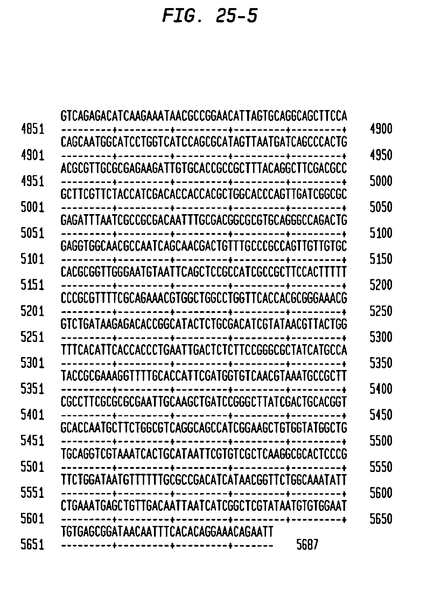

Figure 25 shows the Fₒc(L31F-10His)-PhoA9 sequence: 5687 bp (SEQ ID NO:12).

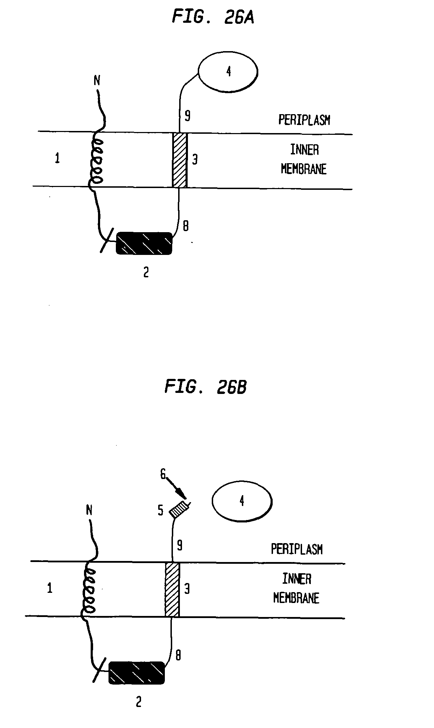

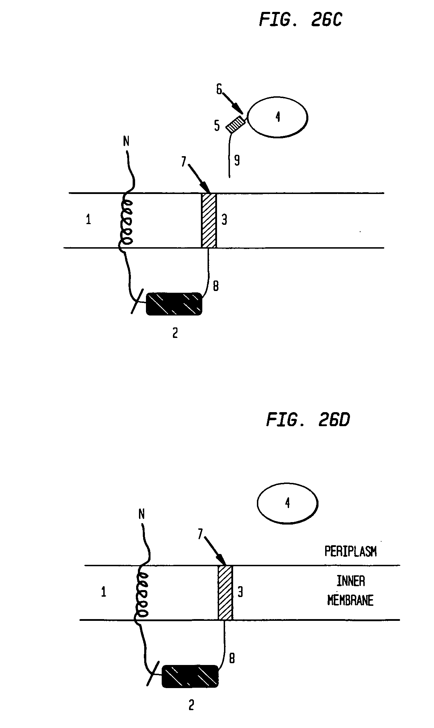

Figure 26 shows cartoons of different types of N-terminal protein vehicles for envelope-targeting. (1) corresponds to a membrane- targeting peptide that extends through the membrane into the cytoplasm and optionally into the extracellular space. (2) corresponds to a cytoplasmic protein domain, where the protein domain is capable of binding to an affinity substrate. (3) corresponds to a trans-membrane segment (TM2), the TM2 being linked to the affinity-binding domain via a protease-resistant cytoplasmic peptide (8). The protein of interest is (4). The extracellular epitope is (5). (9) is the linker sequence optionally containing a cleavage site for signal peptidase (SPase) (7) or a cleavage site for a heterologous protease (6).
Figure 26A shows that (4) is tethered in the extra-cytoplasmic space.
Figure 26B shows that (4) can ultimately be cleaved *in vivo* from (5) at a cleavage site (6) located between (5) and (4). This requires a strain expressing a heterologous protease for secretion into the periplasm in a regulated or constitutive manner. In vivo cleavage by a heterologous protease targeted to the periplasm may involve for example, Enterokinase (Ek) or Tobacco Etch Virus (Tev).
Figure 26C shows that (4) attached to (5) can ultimately be cleaved at an SPase recognition sequence (7) between (3) and (5), leaving the extracellular epitope linked to the protein of interest (4). The host cell inherently contains SPase hence *in vivo* cleavage is constitutive.
Figure 26D shows a protein fusion in which the SPase cleaves directly between (3) and (4) at (7) in the absence of (5).

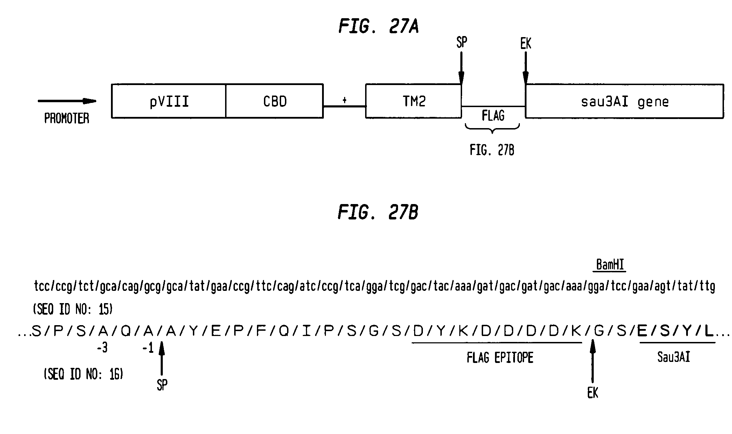

Figures 27A and 27B show a gene fusion combining desirable elements for protein trans-membrane export in which the pVIII-CBD vector may be employed for export of a protein of interest and released into the periplasm by constitutive cleavage by *E. coli* SPase.
Figure 27A shows a vector map of a pVIII construct. This includes a promoter of choice (Ptac), a DNA coding for a membrane-targeting peptide (pVIII), a DNA coding for an affinity-binding protein (CBD), a DNA coding for a TM2, an optional SPase site, an optional DNA encoding an epitope (FLAG), an optional DNA encoding a protease cleavage site (Ek) and a DNA encoding a protein of interest.
Figure 27B shows a linear diagram of a cloning strategy where the exported protein may be expressed with an N-terminal FLAG epitope after *in vivo* cleavage by *E. coli* SPase. This modification of the pVIII-CBD vector is designated as pVIIICBDspEk. The abbreviation "sp" indicates an SPase site. The abbreviation "Ek" indicates the inclusion of the FLAG epitope (DYKDDDDK) (SEQ ID NO:13) and corresponding Enterokinase protease cleavage site (DDDDK) (SEQ ID NO:14).

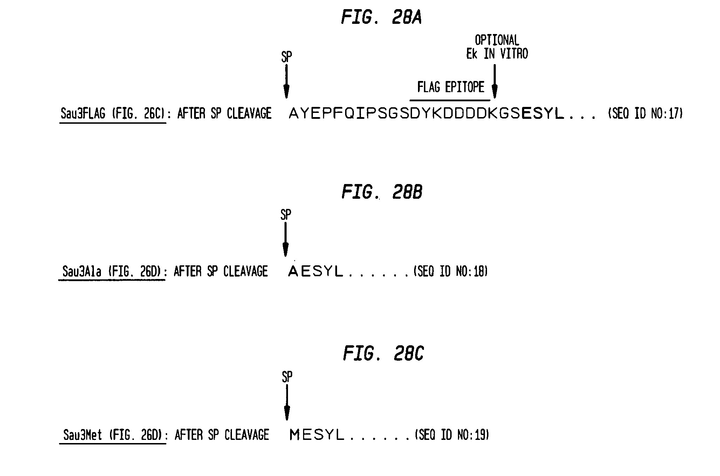

Figures 28A-C show cloning and export strategies for Sau3AI in *E. coli.*
Figure 28A: Sau3FLAG after SPase cleavage having a configuration shown in Figure 26C (SEQ ID NO:17).
Figure 28B: Sau3Ala after SPase cleavage having a configuration as shown in Figure 26D (SEQ ID NO:18).
Figure 28C: Sau3Met after SPase cleavage having a configuration as shown in Figure 26D (SEQ ID NO:19).

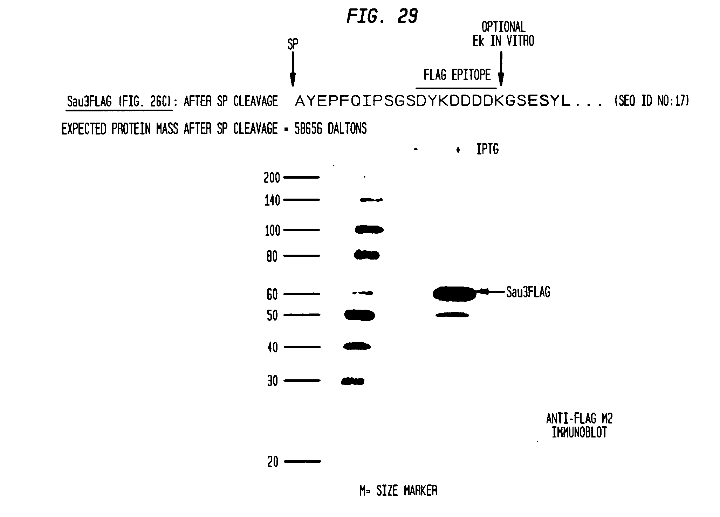

Figure 29 shows Sau3AI export. The expected protein mass after SPase cleavage was 58656 daltons. After SPase cleavage, Sau3AI protein fused to FLAG was obtained as detected by immunoblot of the FLAG epitope using anti-FLAG M2 where the N-terminal amino acids are AYEPFQIPSGSDYKDDDDKGS**ESYL**.... (SEQ ID NO:17).

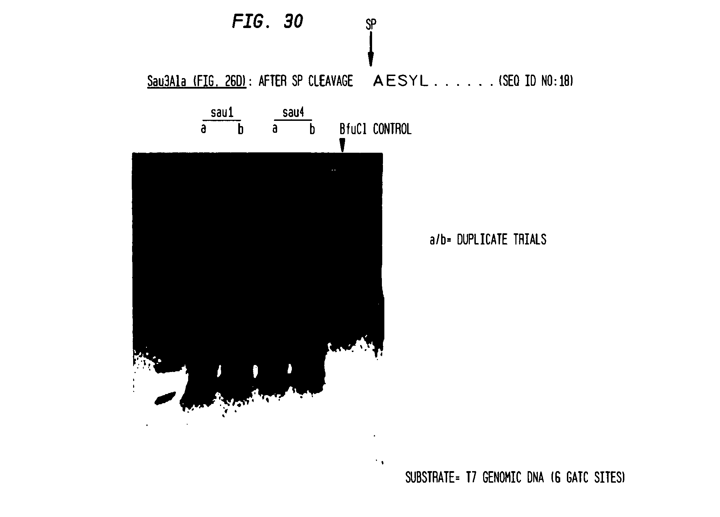

Figure 30 shows expression of Sau3AI in a non-methylase protected *E. coli dsbA⁻* host MB68. The DNA substrate is T7 genomic DNA (6 GATC sites). After SPase cleavage, the N-terminal end of the Sau3AI protein was AESYL... (SEQ ID NO:18). The results of duplicate trials are shown in lanes a and b as labeled. The left-hand lane is a size marker while the right-hand lane is the product of digestion by BfuCI which serves as a control.

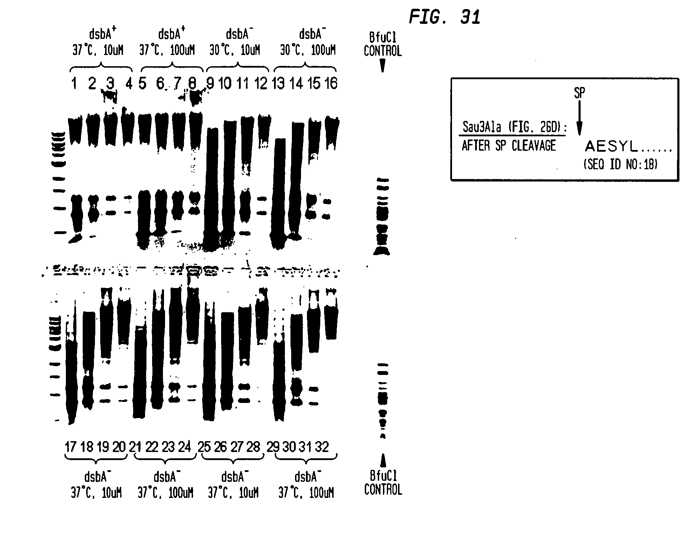

Figure 31 shows expression of Sau3AI in *E. coli dsbA⁻* mutant. After SPase cleavage, the protein has an N-terminal end: **A**ESYL...... (SEQ ID NO:18). The lanes are labeled and are replicated four times for each sample. From left to right, the results of substrate cleavage are shown at 37°C using 6 units purified enzyme and a BfuCI enzyme serves as a control.
   The *dsbA⁻* heading indicates protein expression was carried out in strain MB68 while the heading *dsbA⁺* indicates protein expression was carried out in the wild type (wt) *dsbA* strain DHB4 (isogenic parent strain of MB68). Each lane shows the results of digesting 1 µg phage lambda genomic DNA with either 2 µl of cell lysate or 2 µl of diluted cell lysate. Thus each set of 4 lanes represents a 2-fold serial dilution series. The temperature within the heading indicates the culture outgrowth temperature while the concentration indicates the final IPTG concentration used to induce Sau3AI expression. All cultures were shifted to 20°C during the Sau3AI induction period. The right-hand lane is the product of digestion by BfuCI which serves as a control.

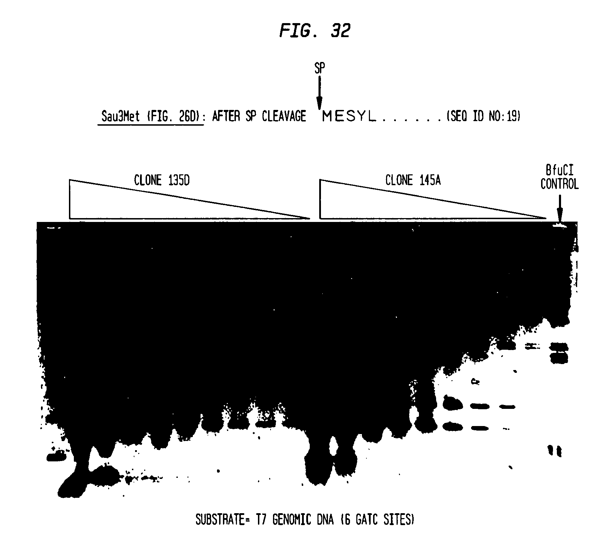

Figure 32 shows the export of wt Sau3AI using pVIII-CBDsp in the *dsbA⁻ E. coli* strain MB68. Sau3AI was expressed from either clone 135D or 145A. The substrate is 1 µg of T7 genomic DNA (6 GATC sites). After SPase cleavage, the Sau3AI has an N-terminal sequence of MESYL.......(SEQ ID NO:19). The results with 2 different clones are shown. The left-most lane is a 1 kb marker (NEB, Ipswich, MA). The right-most lane is the BfuCI digest control. A two-fold serial dilution is shown of the lysate of each clone. Each series of lanes represent reactions resulting from a 2-fold series dilution of cell lysate (beginning with 2 µl of undiluted lysate).

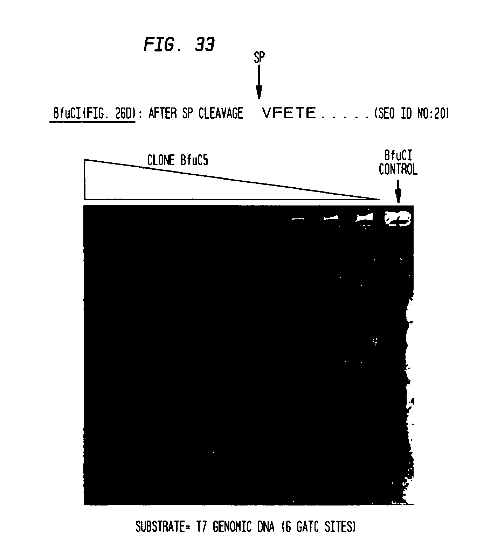

Figure 33 shows export of BfuCI using pVIII-CBDsp in the *dsbA⁻ E. coli* strain MB68. The substrate is T7 genomic DNA (6 GATC sites). After SPase cleavage, the N-terminal end of the BfuCI is VFETE....... (SEQ ID NO:20). The right-most lane is a BfuCI control digest.

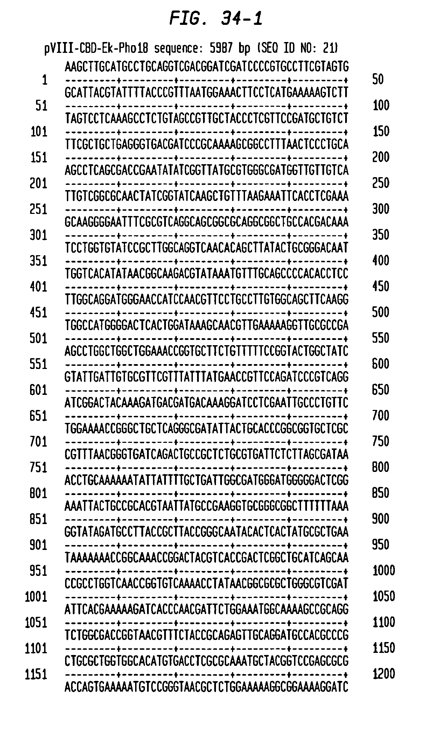

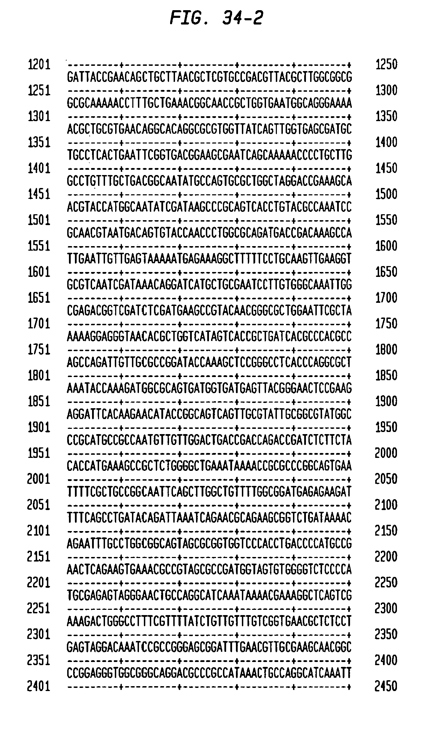

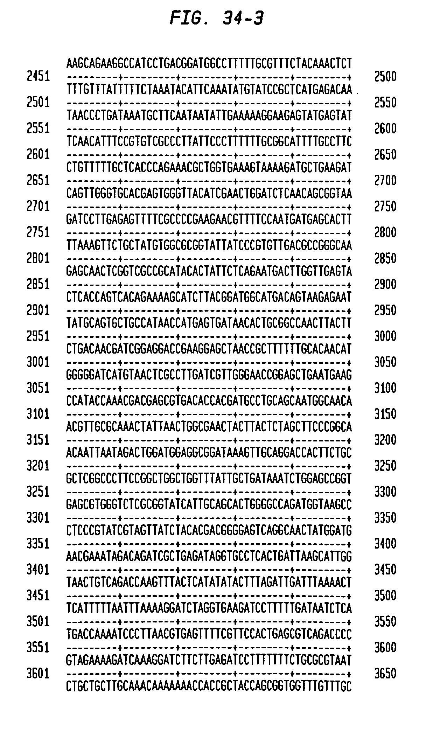

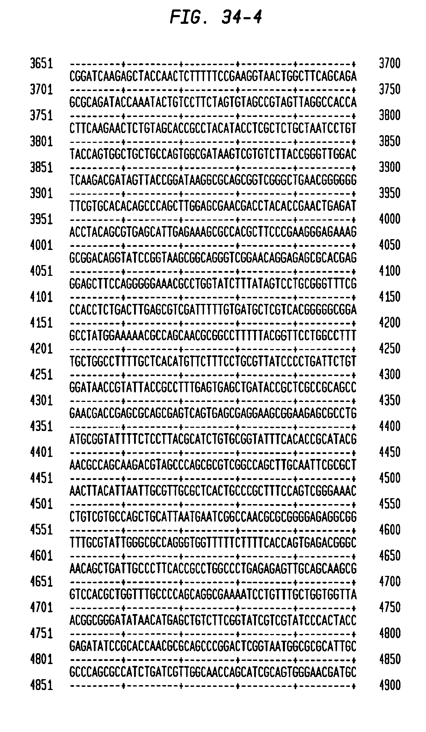

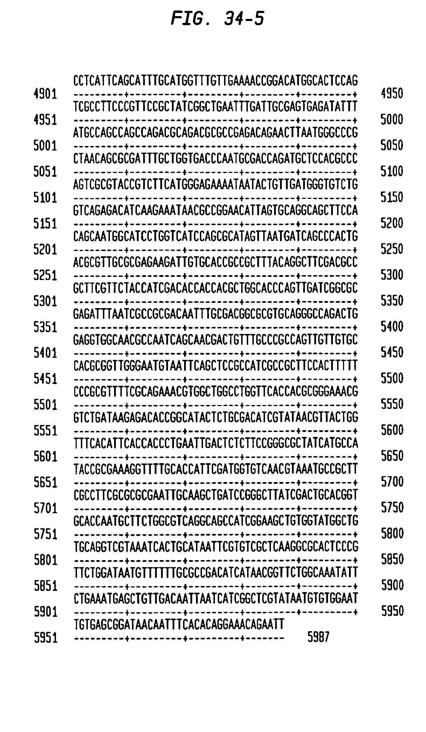

Figure 34 shows the pVIII-CBD-Ek-Pho18 DNA sequence (SEQ ID NO:21).

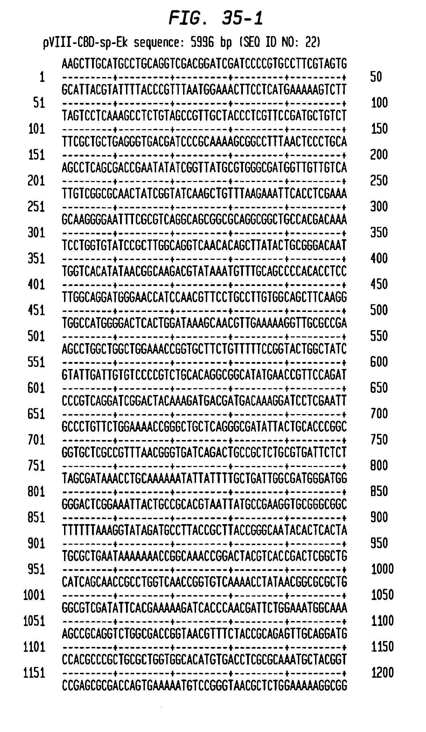

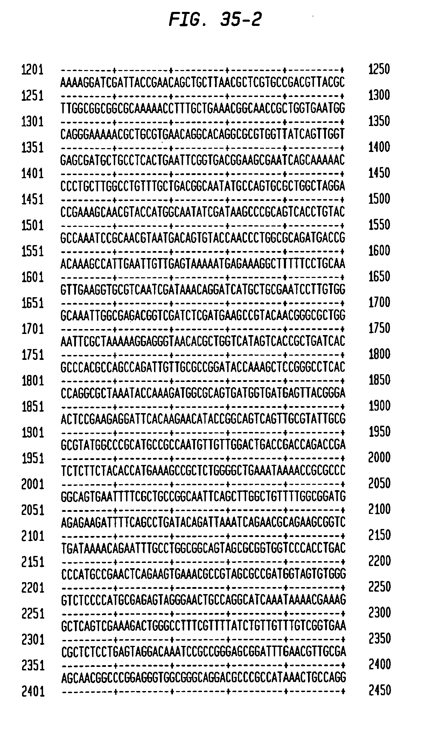

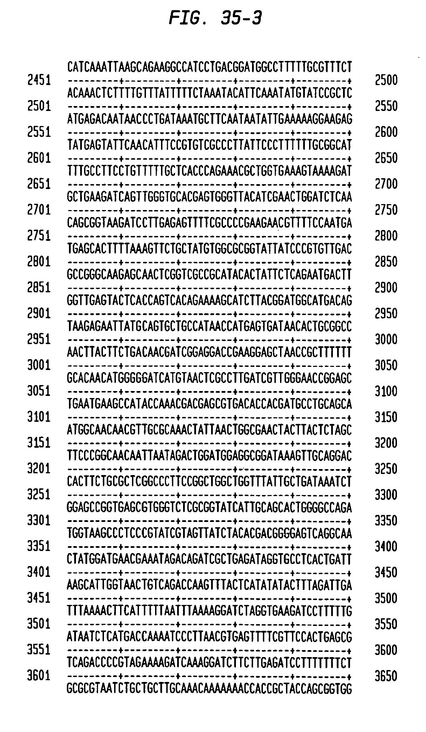

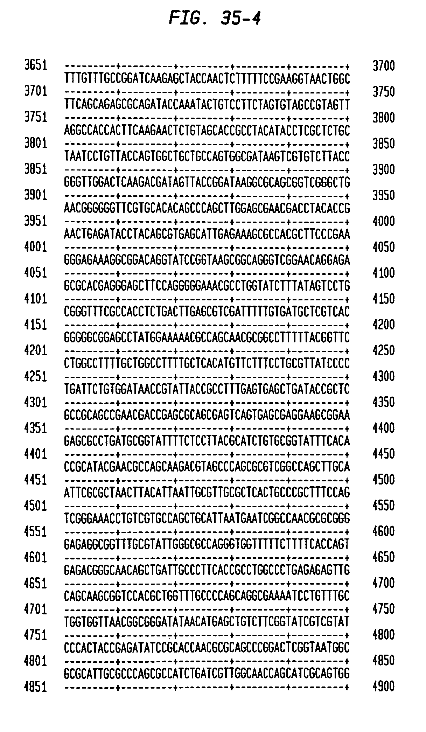

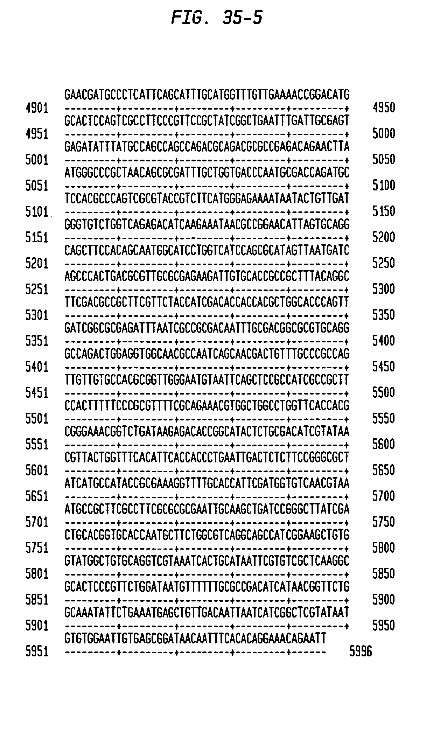

Figure 35 shows the pVIII-CBD-sp-Ek DNA sequence (SEQ ID NO:22).

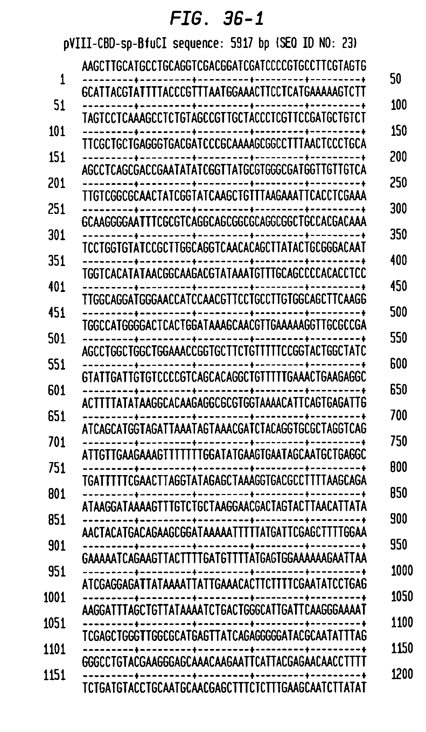

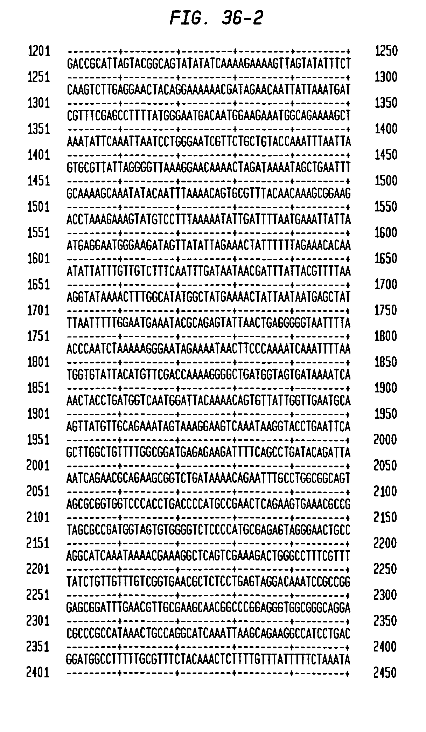

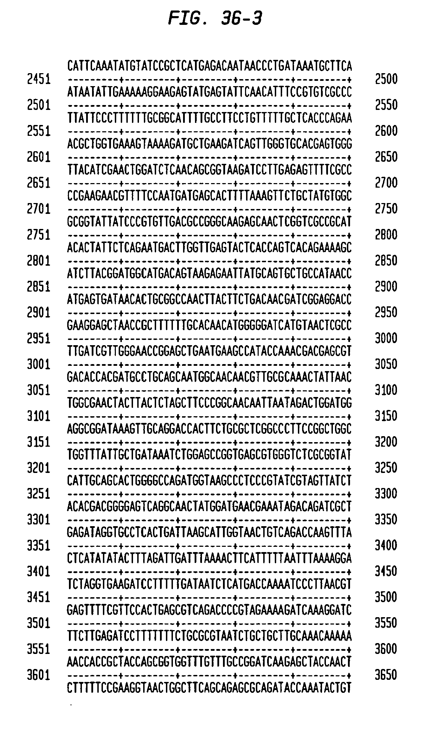

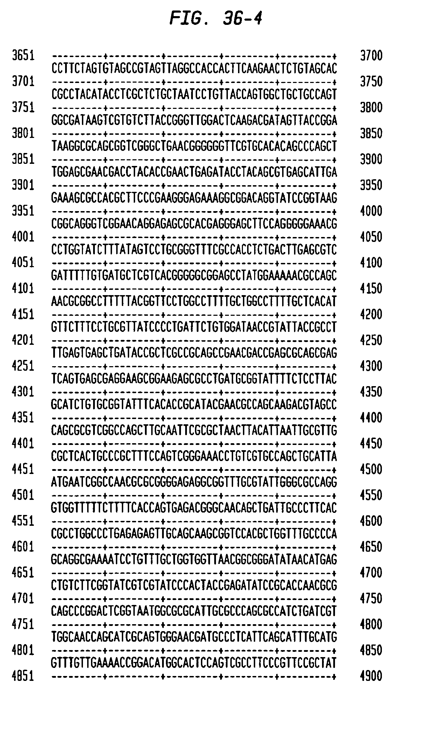

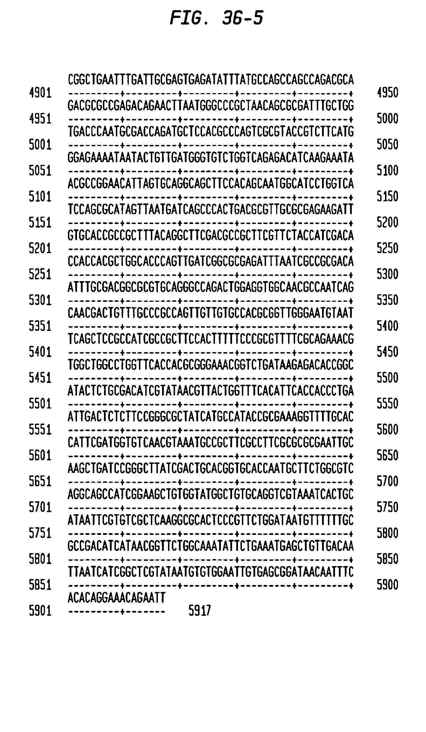

Figure 36 shows the pVIII-CBD-sp-BfuCI DNA sequence (SEQ ID NO:23).

### DETAILED DESCRIPTION OF THE EMBODIMENTS

Present embodiments of the invention provide methods and compositions for expression and transport of heterologous membrane proteins (proteins of interest) to the inner membrane, and membrane translocation of heterologous soluble proteins including toxic proteins to the periplasm of host cells. The methods include expression of DNA encoding N-terminal protein vehicles fused to DNA encoding proteins of interest. The expressed fusion proteins are expressed in the cytoplasm and targeted to the inner membrane in a YidC-dependent and/or SRP-dependent manner.

The compositions include recombinant DNA which encodes an N-terminal protein vehicle and the protein of interest. The N-terminal protein vehicle preferably includes a membrane-targeting peptide, a cytoplasmic affinity-binding domain, and a trans-membrane segment. In addition, the N-terminal protein vehicle may contain an SPase site such that the *in vivo* cleavage by an SPase releases the soluble protein of interest or releases the fused membrane protein of interest. Additionally or alternatively, the N-terminal protein vehicle may include a heterologous protease site such as a Tev protease cleavage site or an Enterokinase cleavage site for *in vivo* or *in vitro* cleavage and release of the protein of interest.

The expression of the genetically engineered DNA occurs in a host cell that is suited for expression of the protein of interest. For example, toxic soluble proteins that contain cysteines and do not require disulfide bonds for folding and activity are preferably expressed in *dsbA⁻* host cells. Using embodiments of the method described above, toxic restriction enzymes were successfully expressed and exported to the periplasm in active form (without modification of host genomic DNA with a protective group such as cytosine or adenine methylation) without toxic effects to the host cell.

### The protein of interest

The "protein of interest" refers to a desired recombinant protein for integration within the host bacterial cell inner membrane or for translocation across the inner membrane into the periplasmic space.

Examples of proteins of interest include: DNA metabolizing enzymes that negatively affect host cell viability (e.g. restriction enzymes) and other cytotoxic proteins; proteases that affect host cell physiology (e.g., ATP-dependent proteases); proteins that are normally secreted in native host, but where expression and export are unknown in *E. coli* (e.g. alpha-1,3-galactosidase from *Xanthomonas manihotis);* and proteins that are prone to aggregation/ inclusion body formation when expressed in the cytoplasm (e.g., peripheral membrane proteins and proteins normally residing in multi-protein complexes). Such proteins often possess a hydrophobic protein-interaction surface, which causes aggregation when the protein is expressed alone without its partner. These proteins may be more stably expressed when targeted to the surface of the inner membrane. Anchoring such a protein to the inner membrane may aid in stable expression of the fused protein.

A protein of interest is here exemplified by P2, the polytopic integral membrane protein Oxa1p, or the secreted protein PhoA and also by toxic restriction endonucleases Sau3AI and BfuCI.

### Host cells

Host cells for expressing fusion proteins are preferably gram negative bacteria such as *E. coli* which have an envelope. (An "envelope" refers to the bacterial cell structure that forms a barrier between the cytoplasmic space and the extracellular environment. This generally includes the inner membrane and periplasm of a bacterium.) The host cell preferably is a DsbA⁻ phenotype if the proteins have cysteines but do not require disulfide bonds for folding and activity. For example, Sau3AI contains 3 cysteines and is inactive when expressed into the periplasm in a wt *dsbA* strain but is active when expressed into the periplasm in a strain lacking periplasmic DsbA (DsbA⁻). Restriction enzyme BfuCI (2 cysteines) is active when expressed to the periplasm in a DsbA⁻ host cell.

While not wishing to be limited by theory, we propose that pVIIICBD-PhoA membrane-targeting peptide depends on signal recognition particles in *E. coli* host cells. This was demonstrated using cells containing wt signal recognition particle component Ffh (fifty-four homologue encoded by the *ffh* gene) or mutated Ffh (protein variant Ala37Pro expressed from the *ffh77* allele) cells (mutation reported by Tian et al. (J Bacteriol. 184(1):111-8 (2002)) and Huber et al. (J Bacteriol. 187(9):2983-91 (2005)). We constructed an MC1061 derivative with the *ffh77* mutation in order to examine the SRP-dependence of the pVIII-CBD N-terminal protein vehicle. MC1061 (*ffh77*) was derived from MC1061 using the allele exchange method described by Hamilton et al. (J Bacteriol. 171(9):4617-22 (1989)). When pVIII-C4BD-Ek-PhoA18 was expressed in MC1061 (*wt ffh*) and MC1061 (*ffh77*) cells using the same conditions, PhoA activity in the *ffh77* cells (1120 units) was found to be only 39% of the level expressed in wt *ffh* cells (2860 units) indicating that pVIII-CBD-mediated PhoA export utilizes the co-translational SRP pathway.

### The membrane-targeting peptide

The "membrane-targeting peptide" as used herein refers to a protein, polypeptide, peptide, segment or domain that facilitates the targeting of a recombinant protein to the inner membrane for integration or membrane translocation.

A membrane-targeting peptide may be described according to its topology in the membrane. For example, the term "N-out" referring to a topology of either a prokaryotic or eukaryotic protein means that the N-terminus of the protein is external to or externally protrudes from the membrane containing the protein. (N-out location in bacteria is the periplasmic space.)

The membrane-targeting peptides are recognized by signal recognition particles in a co-translational process. This avoids the release of aggregation-prone hydrophobic segments into the cytoplasm. The membrane-targeting peptide utilized here was selected from naturally or synthetic proteins according to its hydrophobicity as defined below. The hydrophobicity is here calculated by the GES model (Engelman et al. Annu. Rev. Biophys. Chem. 15:321-353 (1986)). This calculation may be performed using the TopPred algorithm (http://mobyle.pasteur.fr/cgibin/MobylePortal/portal.py?form=top pred) (Claros et al. CABIOS 10:685-686 (1994)). It was found that a membrane-targeting peptide could be characterized by a 21 amino acid window in which there is a hydrophobic core sequence of at least 9 amino acids lacking Asp, Glu, Arg, and Lys. A GES hydrophobicity score was assigned to the membrane-targeting peptide using this window of 21 amino acids. The GES score is preferably greater than 1.521 (with a full window size of 21). The term "window" as used herein refers to a continuous stretch of amino acids that can be identified by a computer algorithm programmed to search for a sequence of a specified length.

Examples of such membrane-targeting peptides are pVIII, or mutants of subunit C of F1Fo ATP synthase (Fₒc) which are defective in oligomerization (Kol et al. J. Biol. Chem. 281:29762-29768 (2006)) or Pf3 coat protein (Thiaudière et al. Biochemistry 32(45):12186-96 (1993)). These proteins are dependent on YidC for membrane assembly and form trans-membrane segments (Chen et al. J Biol Chem 277(10):7670-5 (2002); Samuelson et al. J Biol Chem. 276(37):34847-52 (2001)).

pVIII protein is the major coat protein of M13 bacteriophage expressed by gene VIII (gVIII). pVIII has evolved to insert efficiently into the inner membrane of *E. coli* as part of the M13 phage biogenesis process. The precursor form of pVIII (Procoat) (Thiaudière et al. Biochemistry 32(45):12186-96 (1993)) is 73 amino acids while the mature coat protein is 50 amino acids. The pVIII precursor form is cleaved by bacterial SPase upon insertion into the inner membrane.

The precursor pVIII protein preferentially inserts in the membrane with an N-in, C-in topology. ("In" refers to a cytoplasmic location while "out" for this protein in a gram-negative cell refers to a periplasm location. "Topology" refers to the orientation within a lipid bilayer that constitutes a natural or synthetic membrane.)

The pVIII membrane-targeting peptide or variants or derivatives thereof preferably contain a hydrophobic region (residues 44-64 of the precursor form of pVIII as provided in Thiaudière et al. Biochemistry 32(45):12186-96 (1993)) which has been assigned a GES hydrophobicity score of 1.801, (significantly above the 1.52 threshold value for the SRP-dependent DsbA signal sequence). Hence, the pVIII membrane-targeting peptide is recognized by the *E. coli* signal recognition particle facilitating the membrane integration of a fused membrane protein of interest or to aid the membrane translocation of soluble proteins.

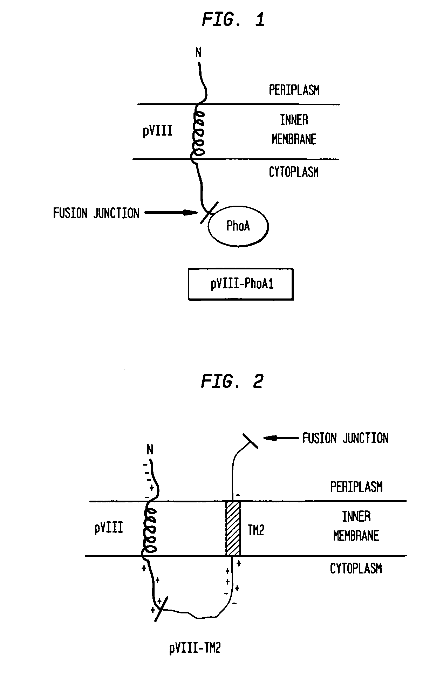

Embodiments of the invention preferably use heterologous membrane proteins of interest with an N-out topology. In such cases, the C-terminus of the N-terminal protein vehicle is preferably extra-cytoplasmic. Accordingly, a series of membrane-targeting constructs were designed and created using standard techniques where pVIII is extended using a segment of bacterial SPase protein (see Examples 2-4, Figures 2, 3, 5). This segment preferably includes part of the SPase cytoplasmic loop and a second TM2 plus a plurality of amino acids that extend into the periplasm. Demonstration of SPase TM2 as an efficient export signal was confirmed in Example 5 upon expression of the pVIII-CBD-PhoA9 fusion protein where the PhoA domain was active after translocation across the inner membrane was mediated by SPase TM2.

The C-out modified pVIII membrane-targeting peptide was named pVIII-TM2 (Figure 2). After SPase cleavage, the mature form of pVIII maintained its position in the membrane with an N-out, C-in topology.

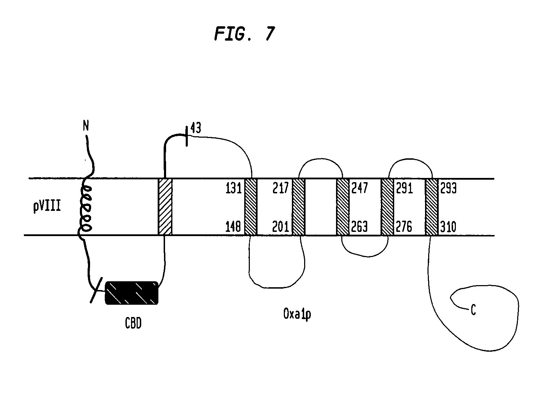

When the membrane protein of interest was fused to the C-terminal end of the trans-membrane segment projecting into the periplasm, the membrane protein of interest was referred to as an N-out membrane protein. "N-out" proteins of interest may be derived from "N-in" proteins of interest by mutagenesis of the open-reading frame (ORF) to create a mutant recombinant gene for fusion to the ORF of the N-terminal protein vehicle for the purpose of expressing a recombinant fusion protein. For example, the recombinant fusion protein ORF may be constructed so that the native signal peptide of the protein of interest is replaced by an ORF encoding one of the membrane-targeting peptides described herein. In embodiments of the invention, methods are provided for producing N-out eukaryotic membrane proteins with correct topology by expressing such proteins as fusions where the fusion junction is located in the *E. coli* periplasm (see for example, Examples 6-9, Figures 7-10).

This is in contrast with proteins or domains fused directly to the C-terminus of a membrane-targeting peptide such as pVIII (Figure 1). In these circumstances, the fusion junction is in the cytoplasm upon membrane assembly of the fusion protein. For example, pVIII-PhoA1 (pVIII bacterial alkaline phosphatase 1) was constructed by ligation of a PhoA reporter domain gene sequence into the EcoRI site downstream of the pVIII ORF. Expression of fusion protein pVIII-PhoA1 (Figure 1) is robust in bacterial host cells and can be detected by using PhoA monoclonal antibody (Sigma, St. Louis, MO). It was found that PhoA activity in these cells is negligible demonstrating that the PhoA domain is cytoplasmic as expected. PhoA is functionally active only when the necessary disulfide bonds are formed by the Dsb system upon export to the periplasm.

An N-terminal protein vehicle for delivering a recombinant eukaryotic membrane protein of interest to a host bacterial inner membrane may include a YidC-dependent ATP synthase subunit C mutant (Fₒc L31F) which has two trans-membrane segments where the C-terminal end projects into the periplasm. The C-terminal end can be fused to a target membrane protein of interest. This is optionally fused to a tag at the C-terminal end. Affinity tags may be inserted into the cytoplasmic region between the two trans-membrane segments of the subunit C mutants (Figure 13).

### The cytoplasmic affinity-binding domain

Suitable cytoplasmic protein-binding domains within the N-terminal fusion partner include: carbohydrate-binding domains such as chitin-binding domain, cellulose-binding domain and maltose-binding domain; poly-histidine tags, Strep tags and any other protein tags amenable to cytoplasmic expression.

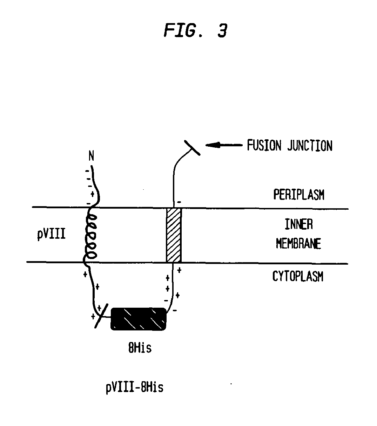

We have discovered that adding an affinity tag to the cytoplasmic loop of the N-terminal fusion partner does not interfere with membrane assembly (Figures 3-6, Examples 5-9).

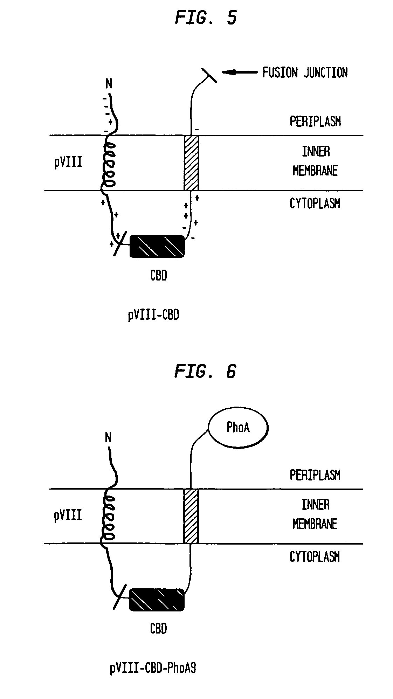

For example, part of the SPase-cytoplasmic loop within pVIII-TM2 was replaced by any affinity tag or detection domain such as a CBD (Figure 5) or His tag (Figure 3). In Figure 3, pVIII-TM2 was mutated to encode a stretch of eight histidines in the cytoplasmic loop at positions 35-42 of SPase. Efficient membrane assembly of pVIII-8His and pVIII-CBD was confirmed by an immunoblot procedure using monoclonal antibody, which preferentially recognizes the mature N-terminus of pVIII after membrane insertion and processing by SPase *in vivo* (Figure 4).

In an additional example, pVIII-CBD was shown to mediate the translocation of the PhoA9 protein into the periplasm of *E. coli.* Expression of fusion protein pVIII-PhoA9 (Figure 6) is robust in bacterial host cells and can be detected by using PhoA monoclonal antibody or measured by performing phosphatase assays (Figure 12) as the periplasmic PhoA domain is activated by disulfide bond formation.

### The trans-membrane domain

"TM2" is a generic term for any trans-membrane segment of defined topology. The segment preferably contains for an amino acid sequence window of 21 amino acids a core sequence of at least about 9 amino acids lacking Asp, Glu, Arg, and Lys, and the 21 amino acid window is flanked on the N-terminal end by an amino acid sequence that comprises at least 9 about amino acids having an overall charge of at least +1. The N-terminal end of the trans-membrane domain is preferably located in the cytoplasm and linked to the cytoplasmic affinity-binding domain. The C-terminal end of the trans-membrane domain may under certain circumstances have a net negative charge. The C-terminal end of the trans-membrane region may be linked via an amino acid linker sequence to the protein of interest. The linking amino acid sequence may contain cleavage sites for SPases and/or a heterologous protease. The organization of the linker and cleavage sites are shown in Figures 26 and 27. In one example, *E. coli* SPase recognition sequences were used. *E. coli* SPase recognition is dependent on TM segment positioning within the inner membrane and also dependent on a relaxed amino acid consensus sequence at -3 and -1 with respect to the point of cleavage (Nielsen et al. Protein Eng. 10(1):1-6 (1997)). Alanine is found most often at -3 and -1 but could be substituted by other small neutral side chains. When proline is present at +1, SPase activity may be inhibited (Barkocy-Gallagher et al. J Biol Chem. 267(2):1231-8 (1992)). However, any amino acid at +1 would be acceptable (except proline) according to *in vivo* studies on SPase function (Dalbey et al. Protein Sci. 6(6):1129-38 (1997)).

If an intervening TM2 sequence is incorporated between pVIII and the protein of interest, then the N-terminal region of the protein of interest will be translocated into the periplasmic space (Figure 2).

### Cleavage sites

In an embodiment of the invention, a cleavage site or sites have been engineered into the fusion protein. Engineered protease cleavage sites are here shown not to interfere with membrane assembly. Examples demonstrating this include: a fusion protein with a cleavage site engineered at the cytoplasmic C-terminal end of pVIII; and a site (Ek, Tev and/or SPase) at the C-terminal end of a second TM2 projecting into the periplasm. Hence, this enables the release of the N-terminus of the membrane protein of interest (the targeted membrane protein) from the membrane-targeting peptide. Protease digestion of the fusion protein may be accomplished *in vivo* or *in vitro.*

### Epitopes

Examples of possible epitopes that may be incorporated for antibody detection of an expressed protein of interest are numerous. For example, anti-CBD monoclonal antibody and anti-CBD serum (NEB, Ipswich MA) may be used to detect the incorporated CBD. Antibody products are commercially available for poly-His detection. Recognition of the extreme N-terminus of the mature pVIII fusion protein is possible with pVIII monoclonal antibody B62-FE2 (Progen Biotechnik GmbH, Heidelberg Germany) to assess expression of full-length protein (Kneissel et al. J. Mol. Biol. 288: 21-28 (1999)). A suitable extracellular epitope is FLAG which may be recognized by anti-FLAG antibody (Sigma, St. Louis, MO).

### The N-terminal protein vehicle

The effectiveness of the pVIII-CBD fusion partner for expression of membrane proteins can be established by an assay in a bacterial host in which endogenous YidC expression is repressed and the viability is compromised in the absence of a complementing protein (van Bloois et al. J. Biol Chem 280: 12996-13003 (2005)). In the assay, the complementing protein was here provided by Oxa1p, which is expressed as a fusion to the pVIII-CBD N-terminal protein vehicle. Oxa1p is a eukaryotic membrane protein, which is the functional homolog of YidC and is found within the mitochondrial inner membrane of eukaryotic cells. Only if the Oxa1p fusion is expressed and inserted correctly in the bacterial inner membrane will the bacteria be viable. Figures 7-10 show diagrams of fusion proteins that were expressed and inserted into the *E. coli* inner membrane in functional form. These results exemplify the utility of the pVIII-CBD fusion partner for heterologous membrane protein expression.

### Fusion protein purification

Methods of fusion protein purification using affinity tags to bind to immobilized substrates displayed on resins, beads (including magnetic beads) or matrices known in the art may be employed to purify the expressed membrane proteins.

Isolating the membrane protein of interest may also require removal of pVIII or subunit C mutant membrane-targeting peptide by protease digestion (Enterokinase, for example). Following *in vitro* digestion of the (pVIII)-(affinity tag)-(protease substrate sequence)-(membrane protein) coupled to a second affinity tag different from the first, the digested fusion protein may be isolated by passing the mixture through a column or using magnetic beads.

Affinity tags in the cytoplasmic loop may be used for fusion protein purification (in constructs lacking a SPase site). In fusion proteins expressed with an SPase site after TM2, the affinity tag in the cytoplasmic loop between the pVIII sequence and TM2 may be used to remove non-processed fusion proteins. For example, in applications where the protein of interest is exported and released by SPase, it is expected that some non-processed full-length fusion protein may be retained within the cytoplasm upon saturation of the export pathway and the SecYEG protein translocase. In this situation, the affinity tag within the N-terminal fusion partner (e.g. pVIII-CBD) may be employed for removal of the non-desirable fusion protein from the soluble protein of interest from within a complex cell lysate.

Export of pVIII-CBD-sp fusions is easily monitored by SDS-PAGE analysis as in vivo cleavage by SPase removes the 19 kDa pVIII-CBD N-terminal partner. In contrast, monitoring typical signal peptide cleavage by SDS-PAGE is often problematic due to the small size (e.g.18-27 amino acids) of native signal peptides. Using *E. coli* SPase to cleave overexpressed fusion proteins *in vivo* is novel and eliminates the need for expensive and often problematic *in vitro* protease processing steps.

### Expression of proteins

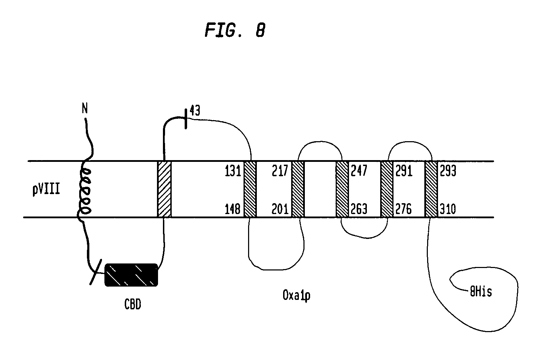

DNA encoding N-terminal protein vehicles such as pVIII-CBD, pVIII-8His, and FₒC variant L31F fused to DNA encoding a protein of interest may be used to express a wide range of eukaryotic and prokaryotic proteins using the system described herein. The Figures illustrate various aspects of embodiments of the invention. Figures 1-10 describe the following:
a membrane-targeting peptide that is incorporated into the inner membrane of a bacterial host where the fusion junction between the membrane-targeting peptide and the target membrane protein is located in the cytoplasm (Figure 1);
a second trans-membrane segment (TM2) from a second protein is fused at its C-terminal end to a membrane protein of interest or a soluble protein of interest where the fusion junction between the C-terminal end of the membrane-targeting peptide and N-terminal end of the protein of interest is located in the periplasm (Figure 2). An affinity tag exemplified by a CBD or His tag may be incorporated between the first and second TM segments (Figures 3, 5, 6, 7, 8, 9 and 10). An additional affinity tag may be fused to the target membrane protein at its C-terminal end (Figures 8, 9, 10).

Examples of engineered fusion proteins expressed with membrane-targeting peptides include: pVIII-PhoA1 (cytoplasmic fusion); pVIII-TM2 (TM2 from SPase added to extend C-terminus to periplasm); pVIII-CBD (cytoplasmic CBD); pVIII-CBD-PhoA9 (cytoplasmic CBD, periplasmic PhoA); pVIII-8His (cytoplasmic 8His); pVIII-CBD-Oxa1p; pVIII-CBD-Oxa1p-8His; pVIII-CBD-Ek-Oxa1p-8His; pVIII-CBD-Tev-Oxa1p-8His; wt C-PhoA9; G23D subunit C-PhoA9; L31F subunit C-PhoA9; and L31F subunit C(10His)-PhoA9. These were derived from the expression clone pVIII-P2 (referred to as Procoat-Lep in Samuelson et al. J. Biol. Chem. 276: 34847-34852 (2001)).

There are multiple different methods for making DNA fusions encoding protein expression constructs. A method described here is not intended to be limiting. This method involved a medium copy pMS119 plasmid, which contains an IPTG-inducible *Ptac* promoter and the *lacIq* gene (Furste et al. Gene 48: 119-131 (1986)). Any inducible protein expression vector may be used by incorporation of the nucleotide-coding sequence for any of the N-terminal protein vehicles described herein. For example, the method may contain a T7 promoter and the *lacI* gene for use in one of many host strains expressing the T7 RNA polymerase.
These expression constructs encode the M13 gene VIII 5' untranslated region (position 1250-1300 of the M13 phage genome) to provide an efficient Shine-Delgarno sequence for protein expression In the bacterial host cell.

The gene VIII Shine-Delgarno sequence is underlined. It should be noted that any 5' untranslated region with an *E. coli* Shine-Delgarno sequence may be employed to initiate pVIII fusion or subunit C fusion protein expression in a bacterial host cell.

### EXAMPLES

The following bacterial strains are used in the present examples. Cloning and propagation of plasmid DNA occurred in NEB 10-beta-competent *E. coli* (NEB #C3019H, Ipswich MA). The genotype of NEB 10-beta strain is *fhuA mcrA f80dlacZDM15 DlacX74 endA1 recA1araD139 D(ara,leu)7697 galU galk rpsL nupG D(mrr-hsdRMS-mcrBC).*

DHB4 was used for alkaline phosphatase (PhoA) assays (Jander et al. J. Bacteriol. 178: 3049-3058 (1996)). MB68 is DHB4 with a *dsbA* gene deletion.

JS7131 was used for YidC-complementation assays (Samuelson et al. Nature 406:637-641 (2000)).

MC1061 was used for protein expression (Casabadan and Cohen J. Mol. Biol. 138:179-207 (1980)).

MG1655 was used as a source of genomic DNA for *E. coli* gene cloning (Blattner et al. Science 277:1453-74 (1997)).

Abbreviations: P2 refers to the soluble P2 domain of *E. coli* SPase. Signal peptidase is abbreviated as either Lep or SPase.

### Example 1: Construction of plasmid pVIII-PhoA1

Plasmid pVIII-PhoA1 (Figure 14) was constructed from plasmid pVIII-P2. pVIII-P2 was digested with EcoRI to excise the P2 ORF. The remaining vector fragment pVIII-EcoRI (Figure 15) was treated with Antarctic Phosphatase (NEB, Ipswich MA) to prepare for ligation of the PhoA gene fragment. Primers 344-112 and 344-113 were used to amplify the PhoA reporter gene from MG1655 genomic DNA. MfeI cloning sites are underlined:

PCR amplification of the PhoA gene was accomplished with Phusion High Fidelity DNA polymerase (NEB #F-540S, Ipswich MA) according to recommended thermocycling conditions. The PhoA gene fragment was digested with MfeI (NEB #R0589S, Ipswich MA) and ligated into the EcoRI sites of the pVIII vector. Ligation clones were sequenced to confirm the entire pVIII -PhoA1 ORF. In the pVIII-P2 and the pVIII-PhoA1 protein fusions, pVIII amino acid 73 was mutated from Ser to Arg. In the pVIII-P2 fusion protein the linker region was Arg73-Gly-Ile-Arg (SEQ ID NO:27), which conforms to the Furin protease recognition sequence: Arg-X-X-Arg (SEQ ID NO:28) (NEB #P8077S, Ipswich MA). In pVIII-PhoA1 the amino acid linker sequence is Arg73-Gly-Ile-Gly (SEQ ID NO:27) immediately preceding Pro6 of PhoA. This linker may be altered to include a protease recognition site. For example, the linker sequence may include the Arg-X-X-Arg (SEQ ID NO:28) recognition sequence for Furin to enable *in vitro* removal of the pVIII-targeting peptide from the protein of interest. In principle, any protease recognition sequence may be inserted into the cytoplasmic linker region at a position C-terminal to pVIII to aid in isolating the protein of interest.

*E. coli* expression of fusion protein pVIII-PhoA1 (Figure 1) was robust in strain DHB4 (*ΔphoA*) upon addition of IPTG. Alkaline phosphatase activity was measured using the standard method by analyzing p-nitrophenyl phosphate (PNPP) hydrolysis (Michaelis et al. J. Bacteriol. 154: 366-375 (1983)). The activity reported in these cells was negligible (Figure 12) indicating that the PhoA domain was expressed within the cytoplasm as expected.

### Example 2: Construction of expression vector pVIII-TM2

Expression vector pVIII-TM2 (Figure 16) was constructed from plasmid pVIII-P2 as follows: A segment of the *E. coli lepB* gene (encoding SPase) was amplified from MG1655 genomic DNA using Taq DNA polymerase and primers 342-239 and 342-240. ApoI restriction sites are underlined: The *lepB* gene fragment was digested with ApoI and ligated into the compatible EcoRI sites of the pVIII-EcoRI vector fragment described in Example 1. Ligation clones were analyzed for the proper orientation of the *lepB* gene fragment by sequencing the plasmid clones with a primer annealing at the Ptac promoter (NEB #s1260s, Ipswich MA). Clone c contained the desired ORF where pVIII is extended by a segment of the *E. coli* SPase protein. This segment (SPase amino acids 34-91) included part of the SPase cytoplasmic loop and the entire second trans-membrane segment (TM2) plus ten amino acids predicted to extend into the periplasm. This amino acid segment was chosen since the membrane topology of SPase TM2 is well documented and SPase TM2 is known to be an efficient export signal for the periplasmic catalytic domain of SPase (von Heijne et al. Proc. Natl. Acad. Sci. USA 85:3363-3366 (1988)). The end of the coding sequence for the *lepB* gene fragment was modified by designing primer 342-240 to encode a BamHI site and an EcoRI site for creating in-frame genetic fusions to the pVIII-TM2 ORF. The genetic fusion sequence was modeled after the polylinker sequence of pUC19 where the BamHI/EcoRI sites code for the amino acids SGSSNS (SEQ ID NO:31), an ideal flexible amino acid linker sequence. The final step of constructing expression vector pVIII-TM2 was to eliminate the BamHI recognition sequence immediately following the Ptac promoter: Clone c was partially digested with BamHI, filled-in with Klenow fragment and ligated to reclose the vector. The final desired expression vector with a single BamHI site (at the fusion junction) was named pVIII-TM2 (Figure 16).

### Example 3: Construction of expression vector pVIII-8His

Expression vector pVIII-8HIS (Figure 17) was constructed from plasmid pVIII-TM2. pVIII-8HIS contains a poly-His sequence within the cytoplasmic loop to enable protein purification or immunodetection. pVIII-TM2 was mutated to encode a stretch of eight histidines in the cytoplasmic loop (replacing amino acids QAAAQAAA (SEQ ID NO:32) equivalent to positions 35-42 of SPase). This expressed membrane-targeting peptide was named pVIII-8His (Figure 3) and efficient membrane assembly was confirmed by an immunoblot procedure using pVIII monoclonal antibody, which preferentially recognizes the mature N-terminus of pVIII after membrane insertion and processing by SPase *in vivo* (Figure 4).

### Example 4: Construction of expression vector pVIII-CBD

An affinity domain was demonstrated to be inserted into the cytoplasmic loop of pVIII-TM2 without interfering with membrane assembly as follows.

pVIII-CBD (Figure 5) is a membrane-targeting peptide encoded by expression clone pVIII-CBD (Figure 18) in which CBD is obtained from *Bacillus circulans* and was inserted within the cytoplasmic loop of pVIII-TM2. Expression vector pVIII-TM2 was amplified by inverse PCR mutagenesis using Phusion™ DNA polymerase (NEB, Ipswich, MA) to isolate the vector fragment for ligation to the CBD gene fragment. Primers 342-890 and 342-891 were used to amplify the pVIII-TM2 vector fragment. The CBD ORF was amplified from NEB (Ipswich MA) vector pTYB11 (position 5798-5950) using primers 342-888 and 342-889. Blunt ligation of the two fragments in the proper orientation resulted in creating a 51 amino acid insertion of the CBD ORF following the cytoplasmic QAAAQAAA (SEQ ID NO:32) sequence of pVIII-TM2. Experimental results showed that the pVIII-CBD targeting protein was highly over-expressed upon IPTG addition and the majority of the protein was localized to the *E. coli* membrane fraction. Binding of pVIII-CBD targeting protein to chitin resin (NEB #S6651S, Ipswich MA) was confirmed in the presence of 0.1% triton X-100, a typical concentration of detergent used during the purification of membrane proteins.
Primer 342-890 5'-GGCAGCCGCCTGCGCCGCTG-3'
   (SEQ ID NO:33).
Primer 342-891 5'-GGGGACTCACTGGATAAAGCAACG-3'
   (SEQ ID NO:34).
Primer 342-888 5'-ACGACAAATCCTGGTGTATCCGCTTG-3'
   (SEQ ID NO:35).
Primer 342-889 5'-ATGGCCACCTTGAAGCTGCCAC-3'
   (SEQ ID NO:36).

### Example 5: Construction of pVIII-CBD-PhoA9

Expression clone pVIII-CBD-PhoA9 (Figure 19) was constructed from expression vector pVIII-CBD to evaluate the membrane topology of membrane-targeting peptide, pVIII-CBD. The pVIII-CBD-PhoA9 fusion protein was designed to contain the amino acid sequence YEPFQIPSGSSNC (SEQ ID NO:37) as the periplasmic linker between SPase TM2 and Pro6 of the PhoA reporter domain. The phoA gene fragment was amplified from MG1655 genomic DNA using Phusion™ DNA polymerase (NEB, Ipswich, MA) and forward primer 344-112 and reverse primer 344-114 described in Example 1.

The PCR amplified phoA gene fragment was digested with MfeI and ligated into vector pVIII-CBD prepared by EcoRI digestion. A clone encoding the PhoA gene in the proper orientation was designated pVIII-CBD-PhoA9. Fusion protein expression from this clone was robust upon IPTG addition. Alkaline phosphatase activity was measured in DHB4 (Δ*phoA*) cells after induction of pVIII-CBD-PhoA9 with 1mM IPTG for 60 minutes at 30°C. In this experiment, the activity was recorded as 2090 units, a 59-fold higher level than for pVIII-PhoA1 expression (Figure 12). This showed that the pVIII-CBD-PhoA9 fusion protein was integrated into the inner membrane with the correct C-out topology (as displayed in Figure 6) since the PhoA domain was active.

### Example 6: Construction of pVIII-CBD-Oxa1p and demonstration of Oxa1p membrane protein function in vivo

Expression clone pVIII-CBD-Oxa1p (Figure 20) was constructed from pVIII-CBD to demonstrate expression of a functional eukaryotic membrane protein in *E. coli.* Oxa1p is an integral membrane protein, which naturally resides within the mitochondrial inner membrane. Oxa1p functions as a membrane protein insertase and is required for the assembly of integral membrane respiratory complexes (Luirink et al. FEBS Lett. 13:1-5 (2001). The YidC integral membrane protein from *E. coli* is a homologue of Oxa1p and has a similar function in bacterial membrane protein biogenesis (van der Laan et al. Proc. Natl. Acad. Sci. USA 100:5801-5806 (2003)). van Bloois et al. (J. Biol. Chem. 280:12996-13003 (2005)) demonstrated that a YidC-Oxa1p chimera is able to complement the loss of YidC in *E. coli.* We created a chimeric fusion between pVIII-CBD and mature Oxa1p (lacking the N-terminal matrix-targeting peptide) to test the membrane-targeting potential of pVIII-CBD. Strain JS7131 was dependent upon arabinose-induced expression of YidC for growth (Samuelson et al. Nature 406:637-641 (2000)). In contrast, providing glucose as the carbon source turned off YidC expression and resulted in loss of viability. Thus, YidC complementation studies were performed by simply plating transformants on LB agar containing glucose to turn off YidC expression and IPTG which induced expression of YidC-complementing proteins.

The *oxa1* gene was amplified from *S. cerevisiae* strain BY4734 (ATCC#200896) using Phusion™ DNA polymerase (NEB, Ipswich, MA) and forward primer 354-1110 and reverse primer 354-1098. Reverse primer 354-1098 contained a silent mutation to destroy a naturally-occurring EcoRI site near the end the *oxa1* ORF. The pVIII-CBD vector was prepared by inverse PCR amplification using Phusion™ DNA polymerase (NEB, Ipswich, MA) and primer 354-1097 and primer 342-240 described in Example 2. Both amplified fragments were digested with EcoRI and ligated to create expression clone pVIII-CBD-Oxa1. This expression clone expressed Oxa1p (beginning at Asn43) as a fusion to targeting protein pVIII-CBD where the fusion junction projected into the periplasm (Figure 7).

Strain JS7131 was transformed with expression clone pVIII-CBD-Oxa1p and plated on LB agar containing 0.1% glucose. Transformants were obtained only when 10 µM IPTG was included in the LB-glucose plates. Confirmation of complementation was done by streaking transformants from the 10 µM IPTG plates to plates containing either zero or 10 µM IPTG. Again, cell growth was observed on the 10 µM IPTG plates only. This result showed that JS7131 growth required expression of pVIII-CBD-Oxa1p and that Oxa1p functioned as an integral membrane protein when expressed as a fusion to pVIII-CBD.

### Example 7: Construction of pVIII-CBD-Oxa1p-8His and demonstration of Oxa1p membrane protein function in vivo

The Oxa1p ORF present in expression clone pVIII-CBD-Oxa1 was modified to encode a C-terminal eight-histidine sequence to aid in protein purification. This was accomplished using inverse PCR mutagenesis with Phusion™ DNA polymerase (NEB, Ipswich, MA)and primers 358-145 and 358-154.

The sequence verified expression clone was named pVIII-CBD-Oxa1p-8His (Figure 21). The expressed protein was tested for *in vivo* function using the YidC-complementation assay described in Example 6. It was concluded that pVIII-CBD-Oxa1p-8His functioned *in vivo* by adopting the topology displayed in Figure 8.

### Example 8: Construction of pVIII-CBD-Ek-Oxa1p-8His and demonstration of Oxa1p membrane Protein function in vivo

The fusion protein ORF in expression clone pVIII-CBD-Oxa1p-8His was modified to encode the Ek protease (NEB #P80705, Ipswich MA) recognition sequence immediately preceding the fusion junction to Oxa1p. This was accomplished by digesting the plasmid expression clone with BamHI and ligating a double-stranded insert comprised of the complementary pair of oligonucleotides: 358-155 and 358-204.
Primer 358-155 5'-GATCGGACTACAAAGATGACGATGACAAAG-3'
   (SEQ ID NO:41).
Primer 355-204 5'-GATCCTTTGTCATCGTCATCTTTGTAGTCC-3'
   (SEQ ID NO:42).

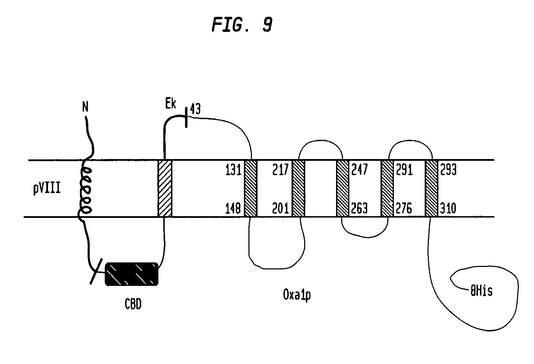

Correct ligation of this oligonucleotide pair resulted in expression clone pVIII-CBD-Ek-Oxa1p-8His (Figure 22). A unique BamHI restriction site was maintained at the DNA fusion junction to enable cloning of membrane protein genes in place of the *oxa1p* gene. The "Ek" amino acid sequence inserted at the fusion junction was DYKDDDDKGS (SEQ ID NO:43). The expressed protein was tested for *in vivo* function using the YidC-complementation assay described in Example 6. pVIII-CBD-Ek-Oxa1p-8His (Figure 9) was shown to function *in vivo.* Note that Ek cleaves after the amino acid sequence DDDDK (SEQ ID NO:14). Also, FLAG M2 monoclonal antibody (Sigma F1804, St. Louis, MO) specifically recognizes the amino acid sequence DYKDDDDK (SEQ ID NO:13). Thus the inserted "Ek" sequence serves to enable specific immuno-detection of fusion protein and to enable *in vivo* or *in vitro* digestion of fusion protein with Enterokinase protease. *In vivo* digestion of fusion protein requires expressing Enterokinase protease into the *E. coli* periplasm.

### Example 9: Construction of pVIII-CBD-Tev-Oxa1p-8His and demonstration of Oxa1p membrane protein function in vivo

The fusion protein ORF present in expression clone pVIII-CBD-Oxa1p-8His was modified to encode the Tev protease recognition sequence immediately preceding the fusion junction to Oxa1p. The inserted "Tev" sequence ENLYFQGS (SEQ ID NO:67) enabled *in vivo* or *in vitro* digestion of fusion protein with Tev protease. *In vivo* cleavage of fusion protein required expressing Tev protease into the *E. coli* periplasm. The pVIII-CBD-Oxa1p-8His expression clone was constructed by digesting clone pVIII-CBD-Oxa1p-8His with BamHI and ligating a double-stranded insert comprised of the complementary pair of oligonucleotides: 358-307 and 358-308.
Primer 358-307 5'- GATCGGAGAACCTGTACTTCCAGG-3'
   (SEQ ID NO:44).
Primer 355-308 5'- GATCCCTGGAAGTACAGGTTCTCC-3'
   (SEQ ID NO:45).

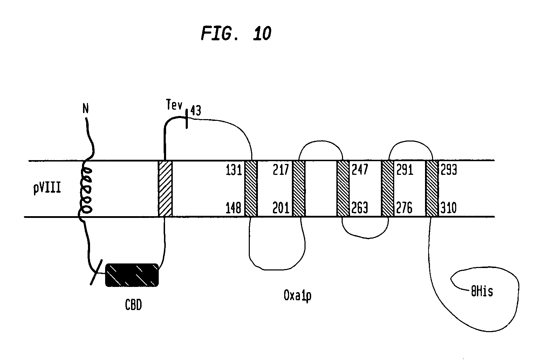

Correct ligation of this oligonucleotide pair resulted in expression clone pVIII-CBD-Tev-Oxa1p-8His (Figure 23). A unique BamHI restriction site was maintained at the DNA fusion junction to enable cloning of membrane protein genes in place of the *oxa1p* gene. The expressed protein was tested for *in vivo* function using the YidC-complementation assay described in Example 6. The results showed that pVIII-CBD-Tev-Oxa1p-8His fusion protein functioned *in vivo* by adopting the topology displayed in Figure 10.

### Example 10: Expression and in vitro purification of pVIII-CBD-Ek-Oxa1p-8His fusion protein

Expression clone pVIII-CBD-Ek-Oxa1p-8His was transformed into *E. coli* strain MC1061. A single transformant was cultured in Terrific Broth (Tartof and Hobbs, Bethesda Res. Lab. Focus 9:12 (1987)) plus 100 µg/mL ampicillin at 30°C until reaching OD600 = 0.4. The temperature was reduced to 20°C and protein expression was induced with 100 µM IPTG for 4 hours. Cells were harvested and frozen. The cell membrane fraction was prepared as follows: Cell pellet (1.2 g) from 250 mL culture was resuspended in 5 mL spheroplast buffer [33 mM Tris-HCl (pH 8.0), 20% sucrose]. Lysozyme was added to a final concentration of 0.01 mg/mL and EDTA was added at 1 mM to create spheroplasts by slow stirring at 4°C for 30 min. Spheroplasts were isolated by centrifugation at 12,000 g for 20 min at 4°C. Spheroplasts were resuspended with 5 mL spheroplast buffer. DNaseI (NEB #M0303S, Ipswich MA) was added at 20 units per mL, MgCl₂ was added at 2 mM and protease inhibitor cocktail (Sigma P8849, St. Louis, MO) was added before disrupting the spheroplasts with a French press (2 passes at 8,000 psi). This mixture was incubated on ice briefly to allow DNase I to reduce viscosity. Intact cells and any insoluble protein were removed by centrifuging at 24,000 g for 15 min at 4°C. This supernatant was subjected to ultra-centrifugation to pellet the membrane fraction. Ultra-centrifugation was carried out using a Beckman Ti70 rotor at 40,000 rpm (147,000 g) (Beckman-Coulter, Fullerton, CA). The membrane pellet was resuspended in 50 mM HEPES-KOH (pH 8.0), 50 mM KCI, 10% glycerol, 0.05% DDM. The membrane fraction was aliquoted into 100 µL units and quick-frozen at -80°C.

Protein purification was carried out as follows: 1% DDM was added to a 100 µL aliquot of membrane fraction. The vial was mixed gently for 1 hour at 4°C to solubilize integral membrane protein. Buffer A [10mM Tris-HCl (pH 8.0), 100 mM KCI, 10% glycerol, 0.1% DDM, 10 mM imidazole] was added to create the Load sample for nickel chelate chromatography. Nickel-NTA agarose resin was pre-washed 3X with buffer A. The load sample (400 µL) was added to 100 µL pre-washed Nickel-NTA resin in an Eppendorf tube and mixed gently for 1 hour at 4°C. The resin void volume was removed and the resin was washed 3X with 200 µL wash buffer [10 mM Tris-HCl (pH 8.0), 100 mM KCI, 10% glycerol, 0.1 % DDM, 40 mM imidazole]. Histidine-tagged protein was eluted with two 80 µL volumes of elution buffer [10 mM Tris-HCl (pH 7.0), 100 KCI, 10% glycerol, 0.1% DDM, 400 mM imidazole]. The elution fractions (e1 and e2) as well as other samples taken throughout the purification were analyzed for the presence of pVIII-CBD-Ek-Oxa1p-8His by immunoblot procedures employing anti-pVIII and anti-FLAG monoclonal antibodies (Figure 11). Figure 11 shows that pVIII-CBD-Ek-Oxa1p-8His fusion protein may be purified from the membrane fraction of an *E. coli* transformant using standard procedures. Furthermore, the identical elution profile shown in Figures 11A and 11B indicated that the pVIII-CBD membrane-targeting peptide was not subject to *in vivo* or *in vitro* proteolytic degradation. This conclusion was made as the FLAG antibody detected the same singular protein species as the pVIII antibody, which specifically recognizes the N-terminus of pVIII (Kneissel et al. J. Mol. Biol. 288: 21-28 (1999)).

### Example 11: Targeting of PhoA to the periplasm using ATP synthase subunit C mutant L31F

Three Fₒc proteins (wt, G23D and L31F) were tested for membrane-targeting and translocation of the PhoA domain. The tabulated results are shown in Figure 12. The L31F mutant Fₒc - PhoA9 fusion expression level was the same as the wt Fₒc -PhoA9 fusion protein expression level. However, the Fₒc(L31F)-PhoA9 fusion reported 52% greater alkaline phosphatase activity. This result is explained by the following: 1) the L31F mutant possesses superior characteristics for membrane-targeting and membrane translocation of C-terminally fused proteins and/or; 2) the lack of subunit C-mediated oligomerization allows C-terminally fused proteins to assemble more readily into functional form. Integral membrane proteins may be also be fused to the C-terminus of oligomerization-defective Fₒc mutants and targeted into the bacterial membrane in functional form (see hypothetical Example 12). Furthermore, affinity and/or detection epitopes may be incorporated into the cytoplasmic loop of Fₒc mutant membrane-targeting peptides. For example, a 10-histidine tag was incorporated into the cytoplasmic loop of Fₒc(L31F)-PhoA9 (Figure 24) to create Fₒc(L31F-10His)-PhoA9 (Figure 25). The 10-histidine tag of Fₒc(L31F-10His)-PhoA9 model protein was placed between R41 and Q42 of the Fₒc amino acid sequence. The PhoA activity of the Fₒc(L31F-10His)-PhoA9 fusion was found to be 80% of the PhoA activity of Fₒc(L31F)-PhoA9 (non-His-tagged protein). This showed that affinity tag incorporation did not seriously affect membrane assembly. It was concluded that other affinity tags or detection domains known in the art could be inserted into the cytoplasmic loop of subunit C without affecting membrane assembly of fused proteins.

### Example 12: Constructs for expressing a target membrane protein fused to a mutant Fₒc membrane-targeting peptide

Membrane protein expression clones may be constructed as gene fusions to a mutant Fₒc gene, which is known to express an Fₒc mutant protein defective in oligomerization. The clones can be constructed according to Examples 2 through 9. The gene encoding the mutant Fₒc may replace the gene encoding pVIII-TM2. Consequently, upon expression of the fusion protein, the fusion junction would be present in the periplasm. Figure 13 shows such an example where Fₒc(L31F) is fused to Oxa1p. Preferably, a protease cleavage site would be encoded at the fusion junction as described in Examples 8, 9 and 10. In addition, one or more affinity domains would be incorporated to enable eventual purification of the membrane-targeting peptide after cleavage from the Fₒc mutant-targeting protein. The protease cleavage of such fusions may occur *in vivo* or *in vitro.*

### Example 13: Construction of pVIII-CBD-Ek-PhoA18 and pVIII-CBD-sp-Ek-PhoA vectors

The pVIII-CBD-Ek-PhoA18 expression construct was derived from the pVIII-CBD-PhoA9 construct (Figure 19) to enable *in vivo* or *in vitro* fusion protein cleavage by Enterokinase. The abbreviation "Ek" indicates the inclusion of the FLAG epitope (DYKDDDDK) (SEQ ID NO:13) and corresponding Enterokinase (Ek) protease cleavage site (DDDDK) (SEQ ID NO:14). The PhoA gene insert in each of these constructs may be replaced with a gene of interest encoding either a membrane protein or a soluble protein destined for export to the periplasm. The DNA coding sequence for the Ek/FLAG amino acid sequence was inserted into the unique BamHI site of plasmid pVIII-CBD-PhoA9. Complementary oligonucleotides 358-155 and 358-204 were annealed at a concentration of 1 micromolar in NEB T4 ligase buffer (NEB, Ipswich, MA). where P = phosphorylated nucleotide.

This short oligonucleotide duplex was ligated to plasmid pVIII-CBD-PhoA9 prepared by BamHI digestion and Antarctic phosphatase treatment (NEB #M0289S). Clone pVIII-CBD-Ek-PhoA18 contained the desired DNA insertion that resulted in the following change to the linker region of the expressed fusion protei n :
pVIII-CBD-PhoA9 linker: Y₍₁₈₀₎EPFQIPSGS (SEQ ID NO:48) where the "GS" coding region is the cloning site (BamHI) for the gene of interest.
pVIII-CBD-Ek-PhoA18 linker: Y₍₁₈₀₎EPFQIPSGSDYKDDDDKGS (SEQ ID NO:49) where the final "GS" coding region is the cloning site (BamHI) for the gene of interest. The FLAG epitope and Enterokinase recognition site are underlined.

The pVIII-CBD-sp-Ek expression vector was constructed from the pVIII-CBD-Ek vector to enable *in vivo* fusion protein cleavage by *E.coli* SPase (Figure 26C). Accordingly, the designation "sp" indicates that an SPase cleavage site was included at the end of TM2 of fusion constructs to cause constitutive *in vivo* fusion protein cleavage. pVIII-CBD-sp fusion proteins can be designed to possess any amino acid at +1 except praline following the SPase recognition sequence. The pVIII-CBD-sp-Ek vector was engineered to encode the amino acids SPSAQA (SEQ ID NO:50) at the end of TM2 at positions -6 to -1 relative to the SPase cleavage site. The SPSAQA (SEQ ID NO:50) sequence is a consensus SPase recognition site (Meindl-Beinker et al. EMBO Rep. 7(11):1111-6 (2006)) and Nilsson et al. J Cell Biol. 126(5):1127-32 (1994)); however, other sequences could be processed efficiently. Accordingly, the pVIII-CBD-Ek-TM2 coding sequence for L₍₁₇₃₎IVRSFIYE₍₁₈₁₎ (SEQ ID NO:51) was altered to result in the amino acid sequence L₍₁₇₃₎IVSPSAQA9^YE₍₁₈₃₎ (SEQ ID NO:52) and to create the pVIII-CBD-sp-Ek fusion vector where ^ indicates the SPase cleavage site. The coding sequence was mutated by inverse PCR mutagenesis using the inverse PCR primers 366-361 and 366-362 to amplify the vector with Phusion DNA polymerase (NEB).

After amplification with primers 366-361 and 366-362, the mutated vector was recircularized by ligation to obtain the pVIII-CBD-sp-Ek vector of interest. (See Figure 27 for a diagram of the spEk linker region.) Fusion protein expression from the pVIIICBDspEk vector resulted in production of the protein of interest with an N-terminal FLAG tag after *in vivo* processing by *E. coli* SPase (Example 14, Figures 28-29).

Gene fusions (lacking the Ek/FLAG sequence) could be created by amplifying the pVIII-CBD-sp-Ek vector to create a unique EagI restriction enzyme cloning site (Example 15) or a USER^{™} (NEB, Ipswich, MA) compatible single-stranded overhang as described in Examples 17 and 18 below. Such fusion constructs were referred to as pVIII-CBD-sp fusions. EagI gene fusion cloning would result in an AE, AD or AG amino acid pair at +1 and +2 of the protein of interest after SPase cleavage. When the USER^{™} method (NEB, Ipswich, MA) of cloning was applied, seamless pVIII-CBD-sp fusion proteins were produced having any desired sequence at +1 and +2 of the protein of interest (Example 17, Figure 33).

### Example 14. Export of Sau3AI restriction enzyme to the periplasm of a bacterium with an N-terminal FLAG tag

Routine expression of the Sau3AI restriction-modification system in the *E. coli* cytoplasm was problematic due to the toxicity of the Sau3AI endonuclease. The protective Sau3AI methylase modifies the cytosine residues of 5'-GATC-3' sequences (Seeber et al. Gene 94(1):37-43 (1990)). However, complete Sau3AI protective methylation of *E*. *coli* genomic DNA could not be achieved during exponential growth.

To overcome these problems, Sau3AI was exported to the periplasm of an *E. coli* strain using the methods described herein, but without modification of the genomic DNA by the Sau3AI methylase.

The first attempt at Sau3AI expression employed the pVIII-CBD-sp-Ek vector so that expression and export was monitored by anti-FLAG immunoblotting. Figure 27 shows the linker region of clone sau4BE. Clone sau4BE expresses a fusion protein designated as pVIII-CBD0sp-Sau3FLAG. After export and SPase cleavage, the N-terminus of this Sau3AI variant was predicted to display the FLAG epitope and the processed protein was predicted to be 58656 daltons. The non-exported and non-processed pVIII-CBD-sp-Sau3FLAG fusion protein was predicted to be 77512 daltons. The *sauBE* gene (BE = BamHI and EcoRI cloning sites) was amplified from *Staphylococcus aureas* genomic DNA using primers 370-097 and 368-219 (see below) and ligated into vector pVIII-CBD-sp-Ek digested with BamHI and EcoRI. Note that the BamHI cloning site (GGATCC) corresponds to the Gly and Ser codons following the Ek site (see Figure 27).
Primer 370-097:
   5'-CCAGGATCCGAAAGTTATTTGACAAAACAAGCCGTAC-3' (SEQ ID NO:55)
The BamHI restriction site is underlined. The EcoRI restriction site is underlined.

The sauBE recombinant clones were transformed into C2523 (NEB Express) cells (NEB, Ipswich, MA). Nine individual clones including sau4BE were grown in LB plus 100 µg/mL ampicillin at 37°C to OD600 = 0.4, then induced with 0.1mM IPTG at 20°C for 3 hours. After harvest, cells were lysed by 0.1 mg/mL lysozyme treatment and sonicated in sonication buffer A [10 mM Tris-HCl (pH 7.5), 50 mM KCl, 0.1 mM EDTA, 5 mM beta-mercaptoethanol]. Cell lysates were tested for restriction activity on lambda DNA. However, all clones including sau4BE were negative for restriction activity. The same induced cells were analyzed for sauBE4 clone (Sau3FLAG protein) expression by anti-FLAG immunoblot analysis. Figure 29 shows that a protein of the correct mass (58656 daltons) was expressed upon IPTG induction.

It was concluded that the lack of restriction activity was due to disulfide bonding of the 3 cysteines within Sau3AI upon export to the periplasm by the native Dsb oxidation system which was primarily mediated by the DsbA oxidoreductase enzyme. It is unusual for bacterial cytoplasmic enzymes to possess disulfide bonds so it was reasonable to assume that native Sau3AI did not require disulfide bond(s) for activity. In fact, it was determined that DsbA-mediated disulfide bond formation could result in mis-folded inactive restriction enzyme.

### Example 15. Expression of Sau3AI restriction enzyme to the periplasm of a dsbA⁻ strain

Strain MB68 is an *E. coli* strain lacking the *dsbA* gene and lacking the periplasmic DsbA oxidoreductase enzyme. pVIII-CBD-sp-Sau3AI clones were constructed without an Ek/FLAG linker for expression in MB68. The *sau3AI* gene was amplified from *Staphylococcus aureus* genomic DNA using primers 368-218 and 368-219 and Phusion™ DNA polymerase (NEB, Ipswich, MA). The pVIII-CBD-sp-Ek vector was amplified with primers 368-220 and 365-074 with Phusion™ DNA polymerase (NEB, Ipswich, MA) to create a unique EagI restriction site at the gene fusion junction.
Sau3AI forward primer 368-218:
   5'-CAACAACGGCCGAAAGTTATTTGACAAAACAAGCCGTAC-3'
   (SEQ ID NO:57). The EagI site is underlined.
Sau3AI reverse primer 368-219:
   5'-CAAGAATTCACAATAAATCTTCAACTTGCTTTTTTATGTAG-3'
   (SEQ ID NO:58). The EcoRI site is underlined.
spEk vector EagI primer 368-220:
   5'-CAACAAGCGGCCGCCTGTGCAGACGGGGACACAATC-3'
   (SEQ ID NO:59). The EagI site is underlined.
spEk vector EcoRI primer 365-074:
   5'-CGGGAATTCAGCTTGGCTGTTTTGGC-3' (SEQ ID NO:60)
   The EcoRI site is underlined.

After vector/insert ligation, the expressed protein of interest (Sau3Ala) was expected to contain an Alanine in place of the initial Met found in native Sau3AI. As described in Example 13 and shown in Figure 28, EagI gene fusion cloning resulted in alanine (A) and glutamate (E) amino acids at +1 and +2 of Sau3AI upon cleavage by SPase. DNA sequencing confirmed that clones "*sau1*" and *"sau4"* encoded the desired EagI gene fusion. Identical clones containing *sau1* and *sau4* genes were transformed into MB68 and grown, induced for protein expression and cell lysates were prepared as described in Example 14. Two microliters of cell lysate was incubated with 1 microgram phage T7 genomic DNA for 60 minutes (37°C) in the presence of 1X NEB buffer 4 (NEB, Ipswich, MA).

Figure 30 shows that the [MB68]sau1 and [MB68]sau4 lysates contain active Sau3AI restriction enzyme when compared to the control digest using 6 units purified BfuCI (NEB #R0636S, NEB, Ipswich, MA) which has the same GATC specificity. Figure 31 demonstrates that the *dsbA⁻* strain mutation was preferably required for production of active Sau3AI in the *E*. *coli* periplasm as there was no measurable fragmentation of the lambda DNA substrate in lanes 1-8 corresponding to wt DsbA host cells.

### Example 16. Expression of periplasmic Sau3AI with an N-terminal amino acid sequence identical to native Sau3AI

Clone sau1 (Sau3Ala protein) was mutated to create an ATG codon corresponding to the +1 amino acid relative to the SPase cleavage site. The objective of this Example was to produce a Sau3Met protein with same N-terminus as native Sau3AI according the gene sequence reported in Seeber et al. (Gene 94(1): 37-43 (1990)). Clone sau1 was amplified in an inverse PCR reaction with Phusion™ DNA polymerase (NEB, Ipswich, MA) using primers 372-913 and 372-915 to create the Ala+1Met mutation:

Clones 135D and 145A were sequenced to confirm desired Ala+1Met codon mutation. Then clones 135D and 145A were transformed into MB68 to confirm Sau3AI restriction enzyme expression and activity. The MB68 cultures were grown in LB plus 100 µg/mL ampicillin at 37°C until OD600=0.4, then shifted to 20°C for induction with 0.1 mM IPTG for 3 hours. Cell lysates were prepared by 0.1 mg/mL lysozyme treatment followed by sonication in sonication buffer A. Figure 32 shows that Sau3AI restriction activity was obtained with a Sau3Met (+1) protein expressed in a *dsbA⁻* strain.

### Example 17. Production of active BfuCI restriction enzyme using a pVIII-CBD-Sp vector compatible with USER^{™} cloning

The BfuCI restriction enzyme from *Bacillus fusiformis* 1226 had not been over-expressed in *E. coli.* BfuCI has the same GATC specificity as Sau3AI and was expected to be toxic when expressed in the *E. coli* for the same reasons as described in Example 14. In this example, BfuCI was expressed from a pVIII-CBD-sp USER^{™} vector for export to the periplasm. The pVIII-CBD-sp-Ek vector was amplified (without the Ek linker coding region) to create USER^{™} enzyme generated single-stranded cohesive ends. pVIII-CBD-sp-Ek was amplified with PfuCx polymerase (Stratagene, LaJolla, CA) and primers 372-072 and 372-073 and then incubated with the USER^{™} enzyme (NEB, Ipswich, MA) for 30 minutes. The *bfucI* gene was amplified from *Bacillus fusiformis* 1226 genomic DNA using PfuCx polymerase and primers 372-074 and 372-075 and then incubated with the USER^{™} enzyme for 30 minutes.
Primer 372-072: 5'-AGGTACC(dU)GAATTCAGCTTGGCTGTTTTGG-3'
   (SEQ ID NO:63)
Primer 372-073: 5'-AGCCTGTGC(dU)GACGGGGACACAATCAATAC-3' (SEQ ID NO:64)

The vector and insert solutions were mixed to enable fragment assembly via 8 and 10 base compatible ends and then directly transformed into C2523 competent *E. coli* cells. DNA sequencing confirmed that clone BfuC5 encoded the desired BfuCI amino acid sequence (VFETE...) (SEQ ID NO:20) following the SPase cleavage site (Figure 33). Clone BfuC5 was designed for expression into the periplasm beginning with the +2 position of the native protein. This course of action was taken since the methionine of native BfuCI was expected to be removed by the host cytoplasmic enzyme methionine aminopeptidase when Valine is encoded at position +2 (Meinnel et al. Biochimie. 75(12):1061-75 (1993)). Figure 33 shows that active BfuCI was expressed from clone BfuC5 in strain MB68. Culture growth, protein expression, lysate preparation, reaction conditions, and presentation of results were the same as described in Example 16.

## Claims

1. A composition **characterized in that** it comprises: a recombinant DNA encoding an N-terminal protein vehicle for transporting a protein of interest fused to the vehicle from the cytoplasmic compartment to the envelope of a prokaryotic cell;
the encoded protein vehicle comprising: a membrane-targeting peptide **characterized by** a Goldman-Engelman-Steitz hydrophobicity score of at least 1.52 for an amino acid window size of 21, a cytoplasmic affinity-binding domain and a trans-membrane segment:
and
the DNA encoding the protein vehicle being fused to a DNA encoding a protein of interest.

2. A composition according to claim 1 wherein the encoded membrane-targeting peptide has an amino acid window of 21 amino acids such that within the 21 amino acid window there is a hydrophobic core sequence of at least 9 amino acids lacking Asp, Glu, Arg and Lys.

3. A composition according to claim 1 wherein the encoded membrane-targeting peptide is YidC-dependent.

4. A composition according to claim 3 wherein the encoded membrane-targeting peptide is selected from pVIII, Pf3 coat and sunbunit C variant L31F.

5. A composition according to claim 1 wherein the encoded cytoplasmic affinity-binding domain is a carbohydrate-binding domain.

6. A composition according to claim 5 wherein the encoded cytoplasmic affinity-binding domain is a chitin-binding domain.

7. A composition according to claim 1 wherein the encoded cytoplasmic affinity-binding domain is selected from a His tag and a strep tag.

8. A composition according to claim 1 wherein the encoded trans-membrane segment has an N-terminal end and a C-terminal end, the segment having a 21 amino acid sequence window such that within the 21 amino acid window there is a core sequence of at least 9 amino acids lacking Asp, Glu, Arg and Lys and the 21 amino acid window is flanked on the N-terminal end by an amino acid sequence that comprises at least 9 amino acids having an overall charge of at least +1.

9. A composition according to claim 8 wherein the 21 amino acid window is flanked on the C-terminal end by an amino acid linker sequence.

10. A composition according to claim 9 wherein the linker sequence contains a signal peptidase cleavage site.

11. A composition according to claim 9 wherein the linker sequence contains a heterologous protease cleavage site.

12. A composition according to claim 8 wherein the encoded trans-membrane segment is TM2 of signal peptidase.

13. A fusion protein **characterized in that** it comprises: a membrane-targeting peptide **characterized by** a Goldman-Engelman-Steitz hydrophobicity score of at least 1.52 for an amino acid window size of 21, a cytoplasmic affinity-binding domain, a trans-membrane segment and a protein of interest.

14. A fusion protein according to claim 13 wherein the protein of interest is a toxic protein.

15. A fusion protein according to claim 14 wherein the toxic protein of interest is a restriction endonuclease.

16. A fusion protein according to claim 15 wherein the restriction endonuclease is Sau3AI or isoschizomer thereof.

17. A method of producing a recombinant protein in a gram-negative bacterial host cell **characterized in that** comprises:
(a) obtaining a recombinant DNA **characterized in** claim 1 fused to a DNA encoding a protein of interest;
and
(b) transforming the host cell.

18. A method according to claim 17 wherein the recombinant protein is purified from the host cell by binding the cytoplasmic affnity-binding domain to an affinity substrate.

19. A method according to claim 17 wherein it further comprises: assaying the transformed host cell for detection of the protein of interest by binding to an affinity substrate via the cytoplasmic affinity-binding domain.

20. A method according to claim 17 wherein the host cell has a DsbA⁻ phenotype.

21. A method according to claim 20 wherein it further comprises: assaying the transformed host cell for determining at least one of enzymatic and binding activity of the protein of interest.

22. A method according to claim 17 wherein the protein of interest is Sau3AI.

23. A method according to claim 17 wherein the protein of interest is BfuCI.

24. A DNA **characterized in that** it comprises: nucleotide sequence 631-1986 in accompanying SEQ ID NO: 23.

25. A method **characterized in that** it comprises: producing a restriction endonuclease in the absence of cognate methylation according to claim 17.

26. A method according to claim 25 wherein the restriction endonuclease is Sau3AI or isoschizomer thereof.

27. A vector **characterized in that** it comprises: a DNA **characterized in** claim 1.

28. A host cell **characterized in that** it comprises: a vector according to claim 27.

## Patentansprüche

1. Zusammensetzung, **dadurch gekennzeichnet, dass** sie umfasst: eine rekombinante DNA, die ein N-terminales Proteinvehikel zum Transport eines Proteins von Interesse, das an das Vehikel gebunden ist, vom zytoplasmatischen Kompartiment zur Hülle einer prokaryotischen Zelle kodiert;
wobei das kodierte Proteinvehikel umfasst: ein auf die Membran zielendes Peptid, **gekennzeichnet durch** ein Goldman-Engelman-Steitz Hydrophobizitätsergebnis von zumindest 1,52 für eine Aminosäurefenstergröße von 21; eine zytoplasmatische affinitätsbindende Domäne und ein Transmembransegment:
und die DNA, die das Proteinvehikel, das an eine DNA kodierend ein Protein von Interesse gebunden ist, kodiert.

2. Zusammensetzung gemäß Anspruch 1, wobei das kodierte auf die Membran zielende Peptid ein Aminosäurefenster von 21 Aminosäuren hat, sodass innerhalb des Fensters von 21 Aminosäuren eine hydrophobe Kernsequenz von zumindest 9 Aminosäuren ohne Asp, Glu, Arg und Lys vorhanden ist.

3. Zusammensetzung gemäß Anspruch 1, wobei das kodierte auf die Membran zielende Peptid YidC-abhängig ist.

4. Zusammensetzung gemäß Anspruch 3, wobei das kodierte auf die Membran zielende Peptid ausgewählt ist aus pVIII, Pf₃-Hüllprotein ("Pf₃ coat") und Untereinheit C Variante L31F.

5. Zusammensetzung gemäß Anspruch 1, wobei die kodierte zytoplasmatische affinitätsbindende Domäne eine Kohlenhydrat bindende Domäne ist.

6. Zusammensetzung gemäß Anspruch 5, wobei die kodierte zytoplasmatische affinitätsbindende Domäne eine Chitin bindende Domäne ist.

7. Zusammensetzung gemäß Anspruch 1, wobei die kodierte zytoplasmatische affinitätsbindende Domäne ausgewählt ist aus einem His-Tag und einem Strep-Tag.

8. Zusammensetzung gemäß Anspruch 1, wobei das kodierte Transmembransegment ein N-terminales Ende und ein C-terminales Ende hat, das Segment ein 21-Aminosäurensequenzfenster hat, sodass innerhalb des Fensters von 21-Aminosäuren eine Kernsequenz von zumindest 9 Aminosäuren ohne Asp, Glu, Arg und Lys vorhanden ist, und das Fenster von 21-Aminosäuren auf dem N-terminalen Ende durch eine Aminosäuresequenz flankiert ist, die zumindest 9 Aminosäuren mit einer Gesamtladung von zumindest +1 umfasst.

9. Zusammensetzung gemäß Anspruch 8, wobei das Fenster von 21-Aminosäuren am C-terminalen Ende durch eine Aminosäurelinkersequenz flankiert ist.

10. Zusammensetzung gemäß Anspruch 9, wobei die Linkersequenz eine Signalpeptidase-Spaltstelle enthält.

11. Zusammensetzung gemäß Anspruch 9, wobei die Linkersequenz eine heterologe Protease-Spaltstelle enthält.

12. Zusammensetzung gemäß Anspruch 8, wobie das kodierte Transmembransegment TM₂ von Signalpeptidase ist.

13. Fusionsprotein, **dadurch gekennzeichnet, dass** es umfasst: ein auf die Membran zielendes Peptid, **gekennzeichnet durch** ein Goldman-Engelman-Steitz Hydrophobizitätsergebnis von zumindest 1,52 für eine Aminosäurefenstergröße von 21, eine zytoplasmatische affinitätsbindende Domäne, ein Transmembransegment und ein Protein von Interesse.

14. Fusionsprotein gemäß Anspruch 13, wobei das Protein von Interesse ein toxisches Protein ist.

15. Fusionsprotein gemäß Anspruch 14, wobei das toxische Protein von Interesse eine Restriktions-Endonuklease ist.

16. Fusionsprotein gemäß Anspruch 15, wobei die Restriktions-Endonuklease Sau₃Al oder ein Isoschizomer davon ist.

17. Verfahren zur Herstellung eines rekombinanten Proteins in einer Gram-negativen bakteriellen Wirtszelle, **dadurch gekennzeichnet, dass** es umfasst:
(a) Erhalten einer in Anspruch 1 gekennzeichneten rekombinanten DNA, die an eine DNA gebunden ist, die ein Protein von Interesse kodiert;
und
(b) Transformieren der Wirtszelle.

18. Verfahren gemäß Anspruch 17, wobei das rekombinante Protein aus der Wirtszelle durch Binden der zytoplasmatischen affinitätsbindenden Domäne an ein Affinitätssubstrat gereinigt wird.

19. Verfahren gemäß Anspruch 17, wobei es weiter umfasst: Untersuchen der transformierten Wirtszelle zur Detektion des Proteins von Interesse durch Binden an ein Affinitätssubstrat durch die zytoplasmatische affinitätsbindende Domäne.

20. Verfahren gemäß Anspruch 17, wobei die Wirtszelle einen DsbA⁻-Phänotypen hat.

21. Verfahren gemäß Anspruch 20, wobei es weiter umfasst: Untersuchen der transformierten Wirtszelle zur Bestimmung von zumindest einem der enzymatischen Aktivität und der Bindeaktivität des Proteins von Interesse.

22. Verfahren gemäß Anspruch 17, wobei das Protein von Interesse Sau₃Al ist.

23. Verfahren gemäß Anspruch 17, wobei das Protein von Interesse BfuCl ist.

24. DNA, **dadurch gekennzeichnet, dass** sie umfasst: Nukleotidsequenz 631 bis 1986 in beigefügter SEQ ID NO: 23.

25. Verfahren, **dadurch gekennzeichnet, dass** es umfasst: Herstellen einer Restriktions-Endonuklease in Abwesenheit einer kognaten Methylierung gemäß Anspruch 17.

26. Verfahren gemäß Anspruch 25, wobei die Restriktions-Endonuklease Sau3Al oder ein Isoschizomer davon ist.

27. Vektor, **dadurch gekennzeichnet, dass** er umfasst: eine in Anspruch 1 gekennzeichnete DNA.

28. Wirtszelle, **dadurch gekennzeichnet, dass** sie umfasst: einen Vektor gemäß Anspruch 27.

## Revendications

1. Composition **caractérisée en ce qu'**elle comprend : un ADN recombinant codant pour une protéine véhicule N-terminale afin de transporter une protéine d'intérêt fusionnée au véhicule à partir du compartiment cytoplasmique vers l'enveloppe d'une cellule procaryote ;
la protéine véhicule codée comprenant : un peptide ciblant une membrane **caractérisé par** un score d'hydrophobicité de Goldman-Engelman-Steitz d'au moins 1,52 pour une taille de fenêtre d'acides aminés de 21, un domaine de liaison ayant une affinité cytoplasmique et un segment transmembranaire ;
et
l'ADN codant pour la protéine véhicule étant fusionné à un ADN codant pour une protéine d'intérêt.

2. Composition selon la revendication 1, dans laquelle le peptide codé ciblant une membrane possède une fenêtre d'acides aminés de 21 acides aminés de sorte qu'au sein de la fenêtre de 21 acides aminés, il existe une séquence centrale hydrophobe d'au moins 9 acides aminés exempte de Asp, Glu, Arg et Lys.

3. Composition selon la revendication 1, dans laquelle le peptide codé ciblant une membrane est YidC-dépendant.

4. Composition selon la revendication 3, dans laquelle le peptide codé ciblant une membrane est sélectionné parmi pVIII, l'enveloppe de Pf3 et le variant de sous-unité C L31F.

5. Composition selon la revendication 1, dans laquelle le domaine de liaison codé ayant une affinité cytoplasmique est un domaine de liaison aux glucides.

6. Composition selon la revendication 5, dans laquelle le domaine de liaison codé ayant une affinité cytoplasmique est un domaine de liaison à la chitine.

7. Composition selon la revendication 1, dans laquelle le domaine de liaison codé ayant une affinité cytoplasmique est sélectionné parmi un marqueur His et un marqueur strep.

8. Composition selon la revendication 1, dans laquelle le segment transmembranaire codé possède une extrémité N-terminale et une extrémité C-terminale, le segment ayant une fenêtre de séquence de 21 acides aminés de sorte qu'au sein de la fenêtre de 21 acides aminés, il existe une séquence centrale d'au moins 9 acides aminés exempte de Asp, Glu, Arg et Lys, et la fenêtre de 21 acides aminés est flanquée sur l'extrémité N-terminale par une séquence d'acides aminés qui comprend au moins 9 acides aminés ayant une charge globale d'au moins +1.

9. Composition selon la revendication 8, dans laquelle la fenêtre de 21 acides aminés est flanquée sur l'extrémité C-terminale par une séquence de liaison d'acides aminés.

10. Composition selon la revendication 9, dans laquelle la séquence de liaison contient un site de clivage de signal peptidase.

11. Composition selon la revendication 9, dans laquelle la séquence de liaison contient un site de clivage de protéase hétérologue.

12. Composition selon la revendication 8, dans laquelle le segment transmembranaire codé est TM2 d'une signal peptidase.

13. Protéine de fusion **caractérisée en ce qu'**elle comprend : un peptide ciblant une membrane **caractérisé par** un score d'hydrophobicité de Goldman-Engelman-Steitz d'au moins 1,52 pour une taille de fenêtre d'acides aminés de 21, un domaine de liaison ayant une affinité cytoplasmique, un segment transmembranaire et une protéine d'intérêt.

14. Protéine de fusion selon la revendication 13, où la protéine d'intérêt est une protéine toxique.

15. Protéine de fusion selon la revendication 14, où la protéine toxique d'intérêt est une endonucléase de restriction.

16. Protéine de fusion selon la revendication 15, où l'endonucléase de restriction est Sau3AI ou un isoschizomère de celle-ci.

17. Procédé pour produire une protéine recombinante dans une cellule hôte bactérienne Gram-négatif, **caractérisé en ce qu'**il consiste à :
(a) obtenir un ADN recombinant caractérisé dans la revendication 1, fusionné à un ADN codant pour une protéine d'intérêt ;
et
(b) transformer la cellule hôte.

18. Procédé selon la revendication 17, dans lequel la protéine recombinante est purifiée à partir de la cellule hôte par une liaison du domaine de liaison ayant une affinité cytoplasmique à un substrat d'affinité.

19. Procédé selon la revendication 17, qui consiste en outre à : analyser la cellule hôte transformée afin de détecter la protéine d'intérêt par une liaison à un substrat d'affinité via le domaine de liaison ayant une affinité cytoplasmique.

20. Procédé selon la revendication 17, dans lequel la cellule hôte possède un phénotype DsbA⁻.

21. Procédé selon la revendication 20, qui consiste en outre à : analyser la cellule hôte transformée afin de déterminer au moins une activité parmi une activité enzymatique et une activité de liaison de la protéine d'intérêt.

22. Procédé selon la revendication 17, dans lequel la protéine d'intérêt est Sau3AI.

23. Procédé selon la revendication 17, dans lequel la protéine d'intérêt est BfuCI.

24. ADN **caractérisé en ce qu'**il comprend : la séquence de nucléotides 631-1986 dans la SEQ ID NO:23 jointe.

25. Procédé **caractérisé en ce qu'**il consiste à : produire une endonucléase de restriction en l'absence de méthylation associée selon la revendication 17.

26. Procédé selon la revendication 25, dans lequel l'endonucléase de restriction est Sau3AI ou un isoschizomère de celle-ci.

27. Vecteur **caractérisé en ce qu'**il comprend : un ADN caractérisé dans la revendication 1.

28. Cellule hôte **caractérisée en ce qu'**elle comprend : un vecteur selon la revendication 27.
